(19) Europäisches Patentamt
European Patent Office
Office européen des brevets

(11) Publication number: **0 247 091 B1**

# (12) EUROPEAN PATENT SPECIFICATION

(45) Date of publication of patent specification: **29.09.93**

(51) Int. Cl.5: **C12N 15/13**, C12P 21/08, C12N 15/10

(21) Application number: **86906676.1**

(22) Date of filing: **27.10.86**

(86) International application number: **PCT/US86/02269**

(87) International publication number: **WO 87/02671 (07.05.87 87/10)**

Divisional application 92115754.1 filed on 27/10/86.

## (54) MODULAR ASSEMBLY OF ANTIBODY GENES, ANTIBODIES PREPARED THEREBY AND USE.

(30) Priority: **01.11.85 US 793980**

(43) Date of publication of application: **02.12.87 Bulletin 87/49**

(45) Publication of the grant of the patent: **29.09.93 Bulletin 93/39**

(84) Designated Contracting States: **AT BE CH DE FR GB IT LI LU NL SE**

(56) References cited:
**EP-A- 125 023**
**EP-A- 171 496**
**EP-A- 173 494**
**EP-A- 0 184 187**
**WO-A-86/01533**
**WO-A-86/05513**
**GB-A- 2 137 631**

(73) Proprietor: **Xoma Corporation**
**2910 7th Street**
**Berkeley California 94718(US)**

(72) Inventor: **ROBINSON, Randy, R.**
**5606 W. 79th Street**
**Los Angeles, CA 90045(US)**
Inventor: **LIU, Alvin, Y.**
**807 - 8th Street Apt. 6**
**Santa Monica, CA 90403(US)**
Inventor: **HORWITZ, Arnold, H.**
**7529 Midfield Avenue**
**Los Angeles, CA 90045(US)**
Inventor: **WALL, Randolph**
**5106 Van Noord**
**Sherman Oaks, CA 91423(US)**

(74) Representative: **Patentanwälte Grünecker, Kinkeldey, Stockmair & Partner**
**Maximilianstrasse 58**
**D-80538 München (DE)**

EP 0 247 091 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

PROCEEDINGS OF THE NATIONAL ACADEMY OF SCIENCES, US, vol. 80, October 1983, pages 6351-6355, US; A. OCHI et al.: "Functional immunoglobulin M production after transfection of cloned immunoglobulin heavy and light chain genes into lymphoid cells"

PROCEEDINGS OF THE NATIONAL ACADEMY OF SCIENCES, (USA) (Washington, D.C) Vol. 77, Issued October 1980 (DOLBY ET AL) "Cloning and Partial Nucleotide Sequence of Human Immunoglobulin ,u chain cDNA from B cells and Mouse-Human Hybridomas". S. pp. 6027-6031

NATURE (London) Vol. 314 Issued 21 March 1985 (NEUBERGER ET AL) "A Hapten-Specific Chimaeric IgE Antibody with Human Physiological Effector Function" s. pp. 268-270.

NATURE (London) Vol. 309, Issued 24 May 1984 (SHARON ET AL) "Expression of a VHCk Chimaeric Protein in Mouse Myeloma Cells", s. pp. 364-367.

PROCESSINGS OF THE NATIONAL ACADEMY OF SCIENCES, (USA), (Washington, D.C.), vol. 81, Issued November 1984 (MORRISON ET AL) "Chimeric Human Antibody Molecules: Mouse Antigen Binding Domains with Human Constant Region Domains", s. pp. 6851-6855

NATURE (London) Vol. 314 Issued 4 April 1985 (TAKEDA ET AL) "Construction of Chimaeric Processed Immunoglobulin Genes Containing Mouse Variable and Human Constant Region Sequences", s. pp. 452-454

NATURE (London), Vol. 312, Issued 13 December 1984, (BOULIANNE ET AL) "Production of Functional Chimaeric Mouse/Human Antibody", s. pp. 643-646

## Description

### BACKGROUND OF THE INVENTION

This application is a continuation in part of Application Serial No. 793,980, filed November 1, 1985, the contents of which are herein fully incorporated by reference.

### Field of the Invention

This invention relates to recombinant DNA methods of preparing immunoglobulins, genetic sequences coding therefor, as well as methods of obtaining such sequences.

### Background Art

The application of cell-to-cell fusion for the production of monoclonal antibodies by Köhler and Milstein (Nature (London), 256: 495, 1975) has spawned a revolution in biology equal in impact to the invention of recombinant DNA cloning. Hybridoma-produced monoclonal antibodies are already widely used in clinical diagnoses and basic scientific studies. Applications of human B cell hybridoma-produced monoclonal antibodies hold great promise for the clinical treatment of cancer, viral and microbial infections, B cell immunodeficiencies with diminished antibody production, and other diseases and disorders of the immune system.

Unfortunately, yields of monoclonal antibodies from human hybridoma cell lines are relatively low (1 ug/ml in human x human compared to 100 ug/ml in mouse hybridomas), and production costs are high for antibodies made in large scale human tissue culture. Mouse x mouse hybridomas, on the other hand, are useful because they produce abundant amounts of protein, and these cell lines are more stable than the human lines. However, repeated injections of "foreign" antibodies, such as a mouse antibody, in humans, can lead to harmful hypersensitivity reactions.

There has therefore been recent exploration of the possibility of producing antibodies having the advantages of monoclonals from mouse-mouse hybridomas, yet the species specific properties of human monoclonal antibodies.

Another problem faced by immunologists is that most human monoclonal antibodies (i.e., antibodies having human recognition properties) obtained in cell culture are of the IgM type. When it is desirable to obtain human monoclonals of the IgG type, however, it has been necessary to use such techniques as cell sorting, to separate the few cells which have switched to producing antibodies of the IgG or other type from the majority producing antibodies of the IgM type. A need therefore exists for a more ready method of switching antibody classes, for any given antibody of a predetermined or desired antigenic specificity.

The present invention bridges both the hybridoma and monoclonal antibody technologies and provides a quick and efficient method, as well as products derived therefrom, for the improved production of chimeric human/non-human antibodies, or of "class switched" antibodies.

Approaches to the problem of producing chimeric antibodies have been published by various authors.

For example, Cabilly et al. disclose in EP-A-125023, the construction of an expression vector for a chimeric immunoglobulin chain which comprises repeated steps of digestion with restriction endonuclease, purification of fragments or filling-in with Klenow-polymerase and dNTP's and ligation. The resulting plasmid carries a human/mouse hybrid gene, comprising a mouse variable region and a human constant region.

GB-A-2137631 discloses cDNA clones encoding immunoglobulin lambda light chains and immunoglobulin $\mu$ heavy chains.

Sharon et al., Nature 309: 364 to 367 (May 24, 1984) disclose the expression of chimeric antibodies comprising the V region of a mouse heavy chain and the C region of a mouse $\kappa$ light chain. Construction of the gene was performed using genomic DNA fragments.

Kudo et al. disclose in EP-A-184187 (prior art under Article 54 (3) EPC) the construction of mouse-human chimeric immunoglobulin heavy chain DNA, which DNA is isolated from genomic DNAs of mouse and human, respectively.

Weissman et al. disclose in WO86/05513 (document under Article 54 (3) EPC) a plasmid vector for use in preparing mouse/human $\kappa$-chain genes.

Morrison, S. L. et al., Proc. Natl. Acad. Sci., USA, 81: 6851-6855 (November 1984), describe the production of a mouse-human antibody molecule of defined antigen binding specificity, produced by joining the variable region genes of a mouse antibody-producing myeloma cell line with known antigen binding specificity to human immunoglobulin constant region genes using recombinant DNA techniques. Chimeric genes were constructed, wherein the heavy chain variable region exon from the myeloma cell line S107 well joined to human IgG1 or IgG2 heavy chain constant region exons, and the light chain variable region exon from the same myeloma to the human kappa light chain exon. These genes were transfected into mouse myeloma cell lines and. Transformed cells producing chimeric mouse-human antiphosphocholine antibodies were thus developed.

Morrison, S. L. et al., European Patent Publication No. 173494 (published March 5, 1986), disclose chimeric "receptors" (e.g. antibodies) having variable regions derived from one species and constant regions derived from another. Mention is made of utilizing cDNA cloning to construct the genes, although no details of cDNA cloning or priming are shown. (see pp 5, 7 and 8).

Boulianne, G. L. et al., Nature, 312: 643 (December 13, 1984), also produced antibodies consisting of mouse variable regions joined to human constant regions. They constructed immunoglobulin genes in which the DNA segments encoding mouse variable regions specific for the hapten trinitrophenyl (TNP) were joined to genomic DNA segments encoding human mu and kappa constant regions. These chimeric genes were expressed as functional TNP binding chimeric IgM.

For a commentary on the work of Boulianne et al. and Morrison et al., see Munro, Nature, 312: 597 (December 13, 1984), Dickson, Genetic Engineering News, 5, No. 3 (March 1985), or Marx, Science, 229: 455 (August 1985).

Neuberger, M. S. et al., Nature, 314: 268 (March 25, 1985), also constructed a chimeric heavy chain immunoglobulin gene in which a DNA segment encoding a mouse variable region specific for the hapten 4-hydroxy-3-nitrophenacetyl (NP) was joined to a segment encoding the human epsilon region. When this chimeric gene was transfected into the J558L cell line, an antibody was produced which bound to the NP hapten and had human IgE properties.

Neuberger, M.S. et al., have also published work showing the preparation of cell lines that secrete hapten-specific antibodies in which the Fc portion has been replaced either with an active enzyme moiety (Williams, G. and Neuberger, M.S. Gene 43:319, 1986) or with a polypeptide displaying c-myc antigenic determinants (Nature, 312:604, 1984).

Neuberger, M. et al., PCT Publication WO 86/01533, (published March 13, 1986) also disclose production of chimeric antibodies (see p. 5) and suggests, among the technique's many uses the concept of "class switching" (see p. 6).

Taniguchi, M., in European Patent Publication No. 171 496 (published February 19, 1985) discloses the production of chimeric antibodies having variable regions with tumor specificity derived from experimental animals, and constant regions derived from human. The corresponding heavy and light chain genes are produced in the genomic form, and expressed in mammalian cells.

Takeda, S. et al., Nature, 314: 452 (April 4, 1985) have described a potential method for the construction of chimeric immunoglobulin genes which have intron sequences removed by the use of a retrovirus vector. However, an unexpected splice donor site caused the deletion of the V region leader sequence. Thus, this approach did not yield complete chimeric antibody molecules.

Cabilly, S. et al., Proc. Natl. Acad. Sci., USA, 81: 3273-3277 (June 1984), describe plasmids that direct the synthesis in E. coli of heavy chains and/or light chains of anti-carcinoembryonic antigen (CEA) antibody. Another plasmid was constructed for expression of a truncated form of heavy chain (Fd') fragment in E. coli. Functional CEA-binding activity was obtained by in vitro reconstitution, in E. coli extracts, of a portion of the heavy chain with light chain.

Boss, M. A., European Patent Application 120694 (published October 3, 1984) shows expression in E. coli of non-chimeric immunoglobulin chains with 4-nitrophenyl specificity. There is a broad description of chimeric antibodies but no details (see p. 9).

Wood, C. R. et al., Nature, 314: 446 (April, 1985) describe plasmids that direct the synthesis of mouse anti-NP antibody proteins in yeast. Heavy chain mu antibody proteins appeared to be glycosylated in the yeast cells. When both heavy and light chains were synthesized in the same cell, some of the protein was assembled into functional antibody molecules, as detected by anti-NP binding activity in soluble protein prepared from yeast cells.

Alexander, A. et al., Proc. Nat. Acad. Sci. USA, 79: 3260-3264 (1982), describe the preparation of a cDNA sequence coding for an abnormally short human Ig gamma heavy chain (OMM $gamma^3$ HCD serum protein) containing a 19- amino acid leader followed by the first 15 residues of the V region. An extensive internal deletion removes the remainder of the V and the entire $C_H1$ domain. This is cDNA coding for an internally deleted molecule.

Dolby, T. W. et al., Proc. Natl. Acad. Sci., USA, 77: 6027-6031 (1980), describe the preparation of a cDNA sequence and recombinant plasmids containing the same coding for mu and kappa human immunoglobulin polypeptides. One of the recombinant DNA molecules contained codons for part of the $CH_3$

constant region domain and the entire 3' noncoding sequence.

Seno, M. et al., Nucleic Acids Research, 11: 719-726 (1983), describe the preparation of a cDNA sequence and recombinant plasmids containing the same coding for part of the variable region and all of the constant region of the human IgE heavy chain (epsilon chain).

Kurokawa, T. et al., ibid, 11: 3077-3085 (1983), show the construction, using cDNA, of three expression plasmids coding for the constant portion of the human IgE heavy chain.

Liu, F. T. et al., Proc. Nat. Acad. Sci., USA, 81: 5369-5373 (September 1984), describe the preparation of a cDNA sequence and recombinant plasmids containing the same encoding about two-thirds of the $CH_2$, and all of the $C_H3$ and $C_H4$ domains of human IgE heavy chain.

Tsujimoto, Y. et al., Nucleic Acids Res., 12: 8407-8414 (November 1984), describe the preparation of a human V lambda cDNA sequence from an Ig lambda-producing human Burkitt lymphoma cell line, by taking advantage of a cloned constant region gene as a primer for cDNA synthesis.

Murphy, J., PCT Publication WO 83/03971 (published November 24, 1983) discloses hybrid proteins made of fragments comprising a toxin and a cell-specific ligand (which is suggested as possibly being an antibody).

Tan, et al., J. Immunol. 135:8564 (November, 1985), obtained expression of a chimeric human-mouse immunoglobulin genomic gene after transfection into mouse myeloma cells.

Jones, P. T., et al., Nature 321:552 (May 1986) constructed and expressed a genomic construct where CDR domains of variable regions from a mouse monoclonal antibody were used to substitute for the corresponding domains in a human antibody.

Sun, L.K., et al., Hybridoma 5 suppl. 1 S17 (1986), describes a chimeric human/mouse antibody with potential tumor specificty. The chimeric heavy and light chain genes are genomic constructs and expressed in mammalian cells.

Sahagan et al., J. Immun. 137:1066-1074 (August 1986) describe a chimeric antibody with specificity to a human tumor associated antigen, the genes for which are assembled from genomic sequences.

For a recent review of the field see also Morrison, S.L., Science 229: 1202-1207 (September 20, 1985) and Oi, V. T., et al., BioTechniques 4:214 (1986).

The Oi, et al., paper is relevant as it argues that the production of chimeric antibodies from cDNA constructs in yeast and/or bacteria is not necessarily advantageous.

See also Commentary on page 835 in Biotechnology 4 (1986).

## SUMMARY OF THE INVENTION

The invention provides a novel approach for producing genetically engineered antibodies of desired variable region specificity and constant region properties through gene cloning and expression of light and heavy chains. The cloned immunoglobulin gene products can be produced by expression in genetically engineered organisms.

The application of chemical gene synthesis, recombinant DNA cloning, and production of specific immunoglobulin chains in various organisms provides an effective solution for the efficient large scale production of human monoclonal antibodies with the antigen specificities of either human or non-human, especially rodent, monoclonal antibodies. The invention also provides a solution to the problem of class switching antibody molecules, so as to readily prepare immunoglobulins of a certain binding specificity of any given class.

The invention provides vectors comprising cDNA sequences coding for immunoglobulin chains comprising a constant human region and a variable, either human or non-human, region. The immunoglobulin chains can either be heavy or light.

The invention also provides gene sequences coding for immunoglobulin chains comprising a cDNA variable region of non-human origin and a genomic constant region of human origin.

The invention also provides sequences as above, present in recombinant DNA molecules, especially in vehicles such as plasmid vectors, capable of expression in desired prokaryotic or eukaryotic hosts.

The invention also provides consensus sequences and specific oligonucleotide sequences useful as probes for hybridization and priming cDNA synthesis of any hybridoma mRNA coding for variable regions of any desired specificity.

The invention provides hosts capable of producing, by culture, chimeric antibodies and methods of using these hosts.

The invention also provides chimeric immunoglobulin individual chains and whole assembled molecules having human constant regions and non-human variable regions, wherein both variable regions have the same binding specificity.

Among other immunoglobulin chains and/or molecules provided by the invention are:

(a) a complete functional, immunoglobulin molecule comprising:

(i) two identical chimeric heavy chains comprising a non-human variable region and human constant region and

(ii) two identical all (i.e. non-chimeric) human light chains.

(b) a complete, functional, immunoglobulin molecule comprising:

(i) two identical chimeric heavy chains comprising a non-human variable region and a human constant region, and

(ii) two identical all (i.e. non-chimeric) non-human light chains.

(c) a monovalent antibody, i.e., a complete, functional immunoglobulin molecule comprising:

(i) two identical chimeric heavy chains comprising a non-human variable region and a human constant region, and

(ii) two different light chains, only one of which has the same specificity as the variable region of the heavy chains. The resulting antibody molecule binds only to one end thereof and is therefore incapable of divalent binding;

(d) an antibody with two different specificities, i.e., a complete, functional immunoglobulin molecule comprising:

(i) two different chimeric heavy chains, the first one of which comprises a non-human variable region and a human constant region and the second comprises a different non-human variable region, and a human constant region, and

(ii) two different chimeric light chains, the first one of which comprises a non-human variable region having the same specificity as the first heavy chain variable region, and a human constant region, and the second comprises a non-human variable region having the same specificity as the second heavy chain variable region, and a human constant region.

The resulting antibody molecule binds to two different antigens.

Genetic sequences, especially cDNA sequences, coding for the aforementioned combinations of chimeric chains or of non-chimeric chains are also provided herein.

The invention also provides for a genetic sequence, especially a cDNA sequence, coding for the variable region of an antibody molecule heavy and/or light chain, operably linked to a sequence coding for a polypeptide different than an immunoglobulin chain (e.g., an enzyme). These sequences can be assembled by the methods of the invention, and expressed to yield mixed-function molecules.

The use of cDNA sequences is particularly advantageous over genomic sequences (which contain introns), in that cDNA sequences can be expressed in bacteria or other hosts which lack RNA splicing systems.

Among preferred specific antibodies are those having specificities to cancer-related antigens.

## BRIEF DESCRIPTION OF THE FIGURES

FIGURE 1 shows the DNA rearrangements and the expression of immunoglobulin mu and gamma heavy chain genes. This is a schematic representation of the human heavy chain gene complex, not shown to scale. Heavy chain variable V region formation occurs through the joining of $V_H$, D and $J_H$ gene segments. This generates an active mu gene. A different kind of DNA rearrangement called "class switching" relocates the joined $V_H$, D and $J_H$ region from the mu constant C region to another heavy chain C region (switching to gamma is diagrammed here). The scheme emphasizes that the J region is a common feature of all expressed heavy chain genes. The J region is also a common feature of expressed light chain genes.

FIGURE 2 shows the known nucleotide sequences of human and mouse J regions. Consensus sequences for the J regions are shown below the actual sequences. The oligonucleotide sequence below the mouse kappa J region consensus sequence is a Universal Immunoglobulin Gene (UIG) oligonucleotide which is used in the present invention.

FIGURE 3 shows a scheme noting the use of the UIG oligonucleotide primer for the synthesis of cDNA complementary to the variable region of immunoglobulin messenger RNA, or the use of oligo-dT as a primer for cDNA synthesis, followed by in vitro mutagenesis.

FIGURE 4 shows the synthesis and analysis of human IgGI genes, including three isolated clones (A.b), one of which (pGMH-6) is utilized as a cloning vector (B). A 1.5 kb deletion of pBR322 sequence between Bam HI and PvuII is marked. Not to scale.

FIGURE 5 shows the cloning vector pQ23, a modified pBR322, useful for cDNA cloning at the KpnI site. This vector also contains the useful restriction enzyme sites BglII plus SalI. Not to scale.

FIGURE 6 shows in A. the synthesis and analysis of human light chain kappa genes. The Figure also shows in B. (not to scale) construction of a human $C_K$ region cloning vector pING2001.

FIGURE 7 shows primers designed for immunoglobulin V region synthesis. (A) shows the heavy chain J-C regions and primers. A DNA version of each mouse J heavy region is shown directly above primers designed from that sequence. Mouse J regions are 5' to 3', left to right, while primers are 3' to 5', left to right. Primer names are included in brackets, and numbers of nucleotides (N) and number of mismatches with each $J_H$ region are listed to the right. Primers which introduce a BstEII site are underlined. (B) shows the light chain J regions and primers. The same as for (A) except for light chains. Primers designed to introduce a BglII site are underlined, as is the BclI site present in pING2016E. (C) shows mouse variable region consensus UIG primers. The actual primer sequence is shown below that consensus sequence. The human $C_K$ HindIII vector pGML60 is shown below. (D) shows a mouse gamma 2a J/C junction primer.

FIGURE 8 shows the synthesis of heavy chain V region module genes using oligonucleotide primed cDNA synthesis. Not to scale.

FIGURE 9 shows the construction of hybrid mouse-human immunoglobulin genes. Panel A shows construction of a heavy chain gene. Stippled regions show C region modules, while hatched or black regions show V region modules. Not to scale.

FIGURE 10 shows the construction of cDNA cloning-expression shuttle vectors for mammalian cells. The vectors pING2003 and pING2003E are derived from pL1, pUC12, pSV2-neo and M8-alphaRX12. Stippled regions indicate mouse heavy chain enhancer DNA, hatched regions indicate SV-40 DNA from pL1, and cross-hatched regions indicate SV-40 DNA from pSV2-neo. In the vectors pING2003 and pING2003E, thick lines represent pBR322 DNA from pSV2-neo, while thin lines represent pUC12 DNA. Arrows indicate the locations and directions of SV-40 early region promoters, and indicates a complete SV-40 intron sequence. Not to scale.

FIGURE 11 shows the construction of the heavy chain expression plasmid pING2006E. Arrows show SV-40 promoter locations and directions of transcription. Hatched and black areas show mouse V region modules, while stippled areas show human C region modules. Not to scale.

FIGURE 12 shows the structure of the chimeric anti-hepatitis heavy chain genes in the expression plasmids pING2006E and pING2012E. Panel A shows the structure of mouse-human chimeric anti-hepatitis heavy chain genes. The structure of human IgGl mRNA and cDNA is shown in A.a. The human heavy chain constant region cDNA clone pGMH-6 and the mouse heavy chain variable region cDNA clones pBS13-1 and pJ3-11 were used to make the hybrid gene used in pING2006E. Hatched gene blocks indicate mouse variable region sequences, while open gene blocks show human IgGl constant region sequences. Panel B shows the nucleotide sequence of the anti-hepatitis B heavy chain variable region in pING2006E and pING2012E. pING2012E was constructed by first inserting a BglII site at the SalI site of pING1202 (See Figure 16) to form pING1202BglII. The chimeric heavy chain gene from this plasmid was inserted into the expression vector pING2003E, resulting in pING2012E. pING2012E differs from pING 2006E in the region immediately upstream of the initiator ATG. Underlined nucleotides denote human J region sequences from the cDNA clone pGMH-6. Asterisked amino acid 117 indicates a single change at this site from mouse to human sequence (Ala to Ser) introduced in the chimeric gene J region. Sequencing was by the Sanger method on plasmid (open circle) and M13 (closed circle) templates.

FIGURE 13 shows in panel A the J-C junction region nucleotide sequence in light chain clones derived from pING2001 (pMACK-3, pING2013E, pING2007E, pING2010E-gpt and pING2014E-gpt). The J region sequence originating from pK2-3 is marked human JK4. The G nucleotide not predicted by genomic sequencing is marked with an asterisk. The oligonucleotide primer (K2-4BCLI) used to modify this sequence is shown below the human JK4 sequence. Panel B diagrams the method of site-directed mutagenesis used to make pING2016E-gpt. Not to scale.

FIGURE 14 Gene copy number of the transfected sequences in two transformants. nDNA from 2AE9, 2BH10 were digested with the enzymes indicated. The concentration of DNA is titrated down across the lanes with the amount indicated above them. The probe contains human C gamma 1 sequences (pmvHc24 ApaI-BamHI). The reference is germ-line or GM2146 nDNA digested with ApaI. The 3' ApaI site is 2 bp beyond the site of poly(A) addition (3).

FIGURE 15 shows the nucleotide sequence of the V region of the L6 $V_H$ cDNA clone pH3-6a. The sequence was determined by the dideoxytermination method using M13 subclones of gene fragments (shown below). Open circles denote amino acid residues confirmed by peptide sequence. A sequence homologous to $D_{SP.2}$ in the CDR3 region is underlined.

FIGURE 16 shows the nucleotide sequence of the V region of the L6 $V_K$ cDNA clone pL3-12a. The oligonucleotide primer used for site-directed mutagenesis is shown below the $J_K5$ segment. Open circles denote amino acid residues confirmed by peptide sequence.

FIGURE 17 shows the construction of chimeric L6-$V_H$ plus human C gamma 1 expression plasmids. Panel (a) shows the sequences of the BAL-31 deletion clones M13mp19-C1-delta 4 (C1-delta 4) and M13mp19-C1-delta 21(C1- delta 21). The 5' end of the cDNA clone, pH3-6a, is denoted with an arrow. M13 sequences are underlined. The oligonucleotide primer used for this experiment is H3-6a (5'-GACTGCACCAACTGG-3'), which primes in FR1 near the mature N terminus. Panel (b) shows the strategy for site-directed mutagenesis of 1 ug of clones C1-delta 4 and C1-delta 21, each annealed to 20 ng of the 31-mer oligonucleotide MJH2-ApaI. Complementary strand synthesis with the Klenow fragment of DNA polymerase was at room temperature for 30 min, then 15 °C for 72 hours. Transfected phage plaques were adsorbed to nitrocellulose, fixed with NaOH, and hybridized to $^{32}$P-labelled MJH2-ApaI oligonucleotide at 65 °C, 18 hours, in 4xTBS (0.6 M NaCl, 0.04 M Tris-HCl (pH 7.4), 0.004 M EDTA) plus 10% dextran sulfate. Final wash of the filters was at 65 °C, 4xSSPE, 0.1% SDS for 15 min. (Maniatis, T., et al., Molecular Cloning: A Laboratory Manual, 1982). Positive plaques were detected by overnight exposure to Kodak XAR film, and were directly picked for growth and restriction enzyme anaysis of RF DNA. Mismatches of the MJH2-ApaI oligonucleotide to the mouse $C_H1$ are denoted, resulting in the coding changes shown below the oligonucleotide. Panel (c) shows the strategy of the substitution of each of the mutagenized L6-$V_H$ modules for the resident $V_H$ of the chimeric expression plasmid pING2012 to generate pING2111 and pING2112.

FIGURE 18 shows the construction of the chimeric L6 expression plasmid pING2119. The SalI to BamHI fragment from pING2100 is identical to the SalI to BamHI A fragment from pING2012E.

FIGURE 19 shows the modification of the $V_K$ gene and its use in constructing light chain and heavy plus light chain expression plasmids.

(a) Deletion of the oligo d[GC] segment 5' of $V_K$ of L6. The oligonucleotide is a 22-mer and contains a SalI site. The 3 mismatches are shown. The $V_K$ gene, after mutagenesis, is joined as a SalI-HindIII fragment to the human C K module. The expression plasmid thus formed is pING2119.

(b) pING2114, a heavy plus light chain expression plasmid. The expression plasmid pING2114 contains the L6 heavy chain chimeric gene from pING2111 and the chimeric light chain from pING2119 (bold line).

FIGURE 20 shows a summary of the sequence alterations made in the construction of the L6 chimeric antibody expression plasmids. Residues underlined in the 5' untranslated region are derived from the cloned mouse kappa and heavy- chain genes. Residues circled in the V/C boundary result from mutagenesis operations to engineer restriction enzyme sites in this region. Residues denoted by small circles above them in the L6 heavy-chain chimera also result from mutagenesis. They are silent changes.

FIGURE 21 shows the 2H7 $V_H$ sequence. The $V_H$ gene contains $J_H1$ sequences and DSP.2 sequence elements. Small circles above the amino acid residues are those that matched to peptide sequences.

FIGURE 22 shows the 2H7 $V_L$ sequence. The $V_K$ gene contains $J_K5$ sequences. A 22-mer oligonucleotide was used to place a SalI site 5' of the ATG initiator codon. Small circles above the amino acid residues are those that matched to peptide sequences.

FIGURE 23 shows the chimeric immunoglobulin gene expression plasmids of the 2H7 specificity. One gene plasmids are pING2101 ($V_H$,neo), pING2106 ($V_K$,neo) and pING2107 ($V_K$,gpt). The others are two-gene plasmids. Their construction involved the ligation of the larger NdeI fragments of pING2101 and pING2107 to linearized pING2106 partially digested with NdeI. pHL2-11 and pHL2-26 were obtained from pING2101 and pING2106; pLL2-25 was obtained from pING2107 and pING2106.

FIGURE 24 shows a summary of the nucleotide changes introduced in the $V_H$ and $V_K$ in the construction of the chimeric plasmids. The cognate $V_H$ and $V_K$ nucleotide residues in the 5' end are underlined. Circles residues in the J-C junctions are derived from the human C modules.

## DESCRIPTION OF THE PREFERRED EMBODIMENTS INTRODUCTION

Generally, antibodies are composed of two light and two heavy chain molecules. Light and heavy chains are divided into domains of structural and functional homology. The variable regions of both light ($V_L$) and heavy ($V_H$) chains determine recognition and specificity. The constant region domains of light ($C_L$) and heavy ($C_H$) chains confer important biological properties such as antibody chain association, secretion, transplacental mobility, complement binding, and the like.

A complex series of events leads to immunoglobulin gene expression in B cells. The V region gene sequences conferring antigen specificity and binding are located in separate germ line gene segments called $V_H$, D and $J_H$; or $V_L$ and $J_L$. These gene segments are joined by DNA rearrangements to form the complete V regions expressed in heavy and light chains respectively (Figure 1). The rearranged, joined ($V_L$-$J_L$ and $V_H$-D-$J_H$) V segments then encode the complete variable regions or antigen binding domains of light and heavy chains, respectively.

DEFINITIONS

certain terms and phrases are used throughout the specification and claims. The following definitions are provided for purposes of clarity and consistency.

1. Expression vector - a plasmid DNA containing necessary regulatory signals for the synthesis of mRNA derived from gene sequences, which can be inserted into the vector.

2. Vector - a plasmid DNA containing a constant or variable region gene.

3. Expression plasmid - an expression vector that contains an inserted gene, such as a chimeric immunoglobulin gene.

4. Gene cloning - synthesis of a gene, insertion into DNA vectors, and identification by hybridization and the like.

5. Transfection - the transfer of DNA into mammalian cells.

GENETIC PROCESSES AND PRODUCTS

The invention provides a novel approach for the cloning and production of human antibodies with desired specificity. Generally, the method combines five elements:

(1) Isolation of messenger RNA (mRNA) from B cell hybridoma lines producing monoclonal antibodies against specific antigens, cloning and cDNA production therefrom;

(2) Preparation of Universal Immunoglobulin Gene (UIG) oligonucleotides, useful as primers and/or probes for cloning of the variable region gene segments in the light and heavy chain mRNA from specific human or non-human hybridoma cell lines, and cDNA production therefrom;

(3) Preparation of constant region gene segments by cDNA preparation and cloning, or genomic gene preparation and cloning;

(4) Construction of complete heavy or light chain coding sequences by linkage of the cloned specific immunoglobulin variable region gene segments of part (2) above to cloned human constant region gene segment modules;

(5) Expression and production of light and heavy chains in selected hosts, including prokaryotic and eukaryotic hosts, either in separate fermentations followed by assembly of antibody molecules in vitro, or through production of both chains in the same cell.

The invention employs cloned hybridoma B cell lines producing monoclonal antibodies of defined specificity for the isolation of mRNA for cDNA cloning. Because many lymphoid cell lines contain highly active nucleases which degrade mRNA during isolation, the invention uses mRNA preparation methods specifically developed for the isolation of intact mRNA from cells and tissues containing active nucleases. One such method yields total RNA preparations by cell or tissue disruption in an ethanol-perchlorate dry ice mixture which reduces nuclease action (Lizardi, P. M. et al., Anal. Biochem., 98: 116 (1979)). This method gives intact translatable mRNA.

Other methods that have been used for this invention include extraction of cells with lithium chloride plus urea (Auffray, C., and Rougeon, F., Eur. J. Biochem., 107: 303 (1980)) or guanidine thiocyanate (Chirgwin, J. M. et al., Biochemistry, 18: 5294 (1979)) to prepare total RNA.

One universal feature of all expressed immunoglobulin light and heavy chain genes and messenger RNAs is the so-called J region (i.e. joining region, see Figure 1). Heavy and light chain J regions have different sequences, but a high degree of sequence homology exists (greater than 80%) within the heavy $J_H$ regions or the kappa light chain J regions. The invention provides consensus sequences of light and heavy chain J regions useful in the design of oligonucleotides (designated herein as UIGs) for use as primers or probes for cloning immunoglobulin light or heavy chain mRNAs or genes (Figures 2 or 7). Depending on the nature of design of a particular UIG, it may be capable of hybridizing to all immunoglobulin mRNAs or genes containing a single specific J sequence, such as UIG-MJH3 which detects only mouse $J_H3$ sequences (Figure 7).

Another utility of a particular UIG probe may be hybridization to light chain or heavy chain mRNAs of a specific constant region, such as UIG-MJK which detects all mouse $J_K$ containing sequences (Figure 7). UIG design can also include a sequence to introduce a restriction enzyme site into the cDNA copy of an immunoglobulin gene (see Figure 7). The invention may, for example, utilize chemical gene synthesis to generate the UIG probes for the cloning of V regions in immunoglobulin mRNA from hybridoma cells making monoclonal antibodies of desired antigen specificities.

A multi-stage procedure is utilized for generating complete V+C region cDNA clones from hybridoma cell light and heavy chain mRNAs. In the first stage, the invention utilizes UIG probes as "primers" for reverse transcriptase copying of the complete V region and leader coding sequences of heavy and light chain mRNAs (Figure 3). The complementary strand of the primer extended cDNA is then synthesized, and this double-stranded cDNA is cloned in appropriate cDNA cloning vectors such as pBR322 (Gubler and Hoffman, Gene, 25: 263 (1983)) or pQ23 (Figure 5; Maniatis, T. et al., Molecular Cloning: A Laboratory Manual, Cold Spring Harbor Laboratory Publications, New York, page 224 (1982)). Clones are screened for specific hybridization with UIG oligonucleotide probes. Positive heavy and light chain clones identified by this screening procedure are mapped and sequenced to select those containing V region and leader coding sequences.

An alternative method is to make cDNA clones using oligo-dT as a primer, followed by selection of light and heavy chain clones by standard hybridization methods.

A second stage utilizes cloning of C region gene segments to form heavy and light chain module vectors. In one method cDNA clones of human heavy and light chain immunoglobulin mRNA are prepared. These cDNA clones are then converted into C region module vectors by site-directed mutagenesis to place a restriction site at a desired location near a boundary of the constant region. An alternative method utilizes genomic C region clones as the source for C region module vectors.

A third stage of cDNA cloning involves the generation of complete light and heavy chain coding sequences with linked V and C regions. The cloned V region segments generated as above are excised and ligated to light or heavy chain C region module vectors. For example, one can clone the complete human kappa light chain C region and the complete human $gamma_1$ C region. In addition, one can modify a human gamma 1 region and introduce a termination codon, thereby obtain a gene sequence which encodes the heavy chain portion of an Fab molecule.

The coding sequences having operationally linked V and C regions are then transferred into appropriate expression systems for expression in appropriate hosts, prokaryotic or eukaryotic. Operationally linked means in-frame joining of coding sequences to derive a continuously translatable gene sequence without alterations or interruptions of the triplet reading frame.

One particular advantage of using cDNA genetic sequences in the present invention is the fact that they code continuously for immunoglobulin chains, either heavy or light. By "continuously" is meant that the sequences do not contain introns (i.e. are not genomic sequences, but rather, since derived from mRNA by reverse transcription, are sequences of contiguous exons). This characteristic of the cDNA sequences provided by the invention allows them to be expressible in prokaryotic hosts, such as bacteria, or in lower eukaryotic hosts, such as yeast.

Another advantage of cDNA cloning methods is the ease and simplicity of obtaining V region gene modules.

The term "non-human" as used in the invention is meant to include any animal other than a human, wherein an immune response can be generated which then leads to usable B cells resulting in corresponding hybridomas or B cell clones obtained by viral transformation and the like. Such animals commonly include rodents such as the mouse or the rat. Because of ease of preparation and great availability, the mouse is at present the preferred, non-human animal. Mouse-mouse hybridomas are thus utilized as the preferred sources for heavy and light chain variable regions.

Preferably, the invention provides entire V and/or C region cDNA sequences. This means that the sequences code for substantially operable V and/or C regions, without lacking any major structural portions thereof.

The terms "constant" and "variable" are used functionally to denote those regions of the immunoglobulin chain, either heavy or light chain, which code for properties and features possessed by the variable and constant regions in natural non-chimeric antibodies. As noted, it is not necessary for the complete coding region for variable or constant regions to be present, as long as a functionally operating region is present and available.

A wide range of source hybridomas are available for the preparation of mRNA. For example, see the catalogue ATCC CELL LINES AND HYBRIDOMAS, December, 1984, American Type Culture Collection, 12301 Parklawn Drive, Rockville, Maryland 20852, U.S.A., pages 5-9 and the ECACC Catalogue, 2nd Edition; PHLS CAMR Porton Down, Salisbury, Wills; SP4OJG, U.K. pages 30-35 and 40-46. Hybridomas secreting monoclonal antibodies reactive to a wide variety of antigens are listed therein, are available from the collection, and usable in the invention. Of particular interest are hybridomas secreting antibodies which are reactive with viral antigens, including Dengue complex specific (ATCC HB 114), Dengue type 1 virus (ATCC HB 47), Dengue type 2 virus (ATCC HB 46), Dengue type 3 virus (ATCC HB 49), Dengue type 4 virus (ATCC HB 48), Epstein-Barr receptor (ATCC HB 135), Flavivirus group (ATCC HB 112), hepatitis B surface antigen (ATCC CRL 8017 and 8018), herpes simplex type I (ATCC HB 8068), herpes simplex type II (ATCC HB 8067), influenza virus (ATCC CL 189), influenza A virus, matrix protein (ATCC HB 64), influenza A virus, nucleoprotein (ATCC HB 65), influenza A Bangkok/1/79HA (ATCC HB 66), influenza AWSN NP (ATCC HB 67), SV40 large T antigen (ATCC TIB 115), SV40 large T antigen, C-terminal end (ATCC TIB 117), and SV40 nonviral T antigen (ATCC TIB 116). Examples of other hybridomas include those secreting antibodies to tumor associated antigens or to human lymphocyte antigens, such as those reactive to human tumor-associated CEA, high mw (ATCC CRL 8019); human tumor-associated alpha-fetoprotein, $IgG_1K$ - (ATCC HB 134); human B lymphocyte HLA-DR, monomorphic, $IgG_{2b}$ (ATCC HB 104); human T lymphocyte T cell precursors, $IgG_1$ (ATCC CRL 8022); human T lymphocyte T cell subset, helper, $IgG_{2b}$ (ATCC CRL 8002); T subset, suppressor/cytotoxic, human, $IgG_1$ (ATCC CRL 8013); T cell subset, suppressor/cytotoxic, human, $IgG_{2a}$ (ATCC CRL 8014); T cells, peripheral, human, $IgG_1$ (ATCC CRL 8000); T cells, peripheral, human, $IgG_{2a}$ (ATCC CRL 8001); thymocytes, "common," human, $IgG_1$ (ATCC CRL 8020).

These lines and others of similar nature can be utilized to copy the mRNA coding for variable region, using the UIG probes. Of particular interest are antibodies with specificity to human tumor antigens.

Expression vehicles include plasmids or other vectors. Preferred among these are vehicles carrying a functionally complete human constant heavy or light chain sequence having appropriate restriction sites engineered so that any variable heavy or light chain sequence with the appropriate cohesive ends can be easily inserted thereinto. Human constant heavy or light chain sequence-containing vehicles are thus an important embodiment of the invention. These vehicles can be used as intermediates for the expression of any desired complete heavy or light chain in any appropriate host.

One preferred host is yeast. Yeast provides substantial advantages for the production of immunoglobulin light and heavy chains. Yeasts carry out post-translational peptide modifications including glycosylation. A number of recombinant DNA strategies now exist which utilize strong promoter sequences and high copy number plasmids which can be used for overt production of the desired proteins in yeast. Yeast recognizes leader sequences on cloned mammalian gene products and secretes peptides bearing leader sequences (i.e. prepeptides) (Hitzman, et al., 11th International Conference on Yeast, Genetics and Molecular Biology, Montpelier, France, September 13-17, 1982).

1 Yeast gene expression systems can be routinely evaluated for the level of heavy and light chain production, protein stability, and secretion. Any of a series of yeast gene expression systems incorporating promoter and termination elements from the actively expressed genes coding for glycolytic enzymes produced in large quantities when yeasts are grown in mediums rich in glucose can be utilized. Known glycolytic genes can also provide very efficient transcription control signals. For example, the promoter and terminator signals of the iso-1-cytochrome C (CYC-1) gene can be utilized.

The following approach can be taken for evaluating optimal expression plasmids for the expression of cloned immunoglobulin cDNAs in yeast.

(1) The cloned immunoglobulin DNA linking V and C regions is attached to different transcription promoters and terminator DNA fragments;

(2) The chimeric genes are placed on yeast plasmids used for protein overproduction (see, for example, Beggs, J. D., Molecular Genetics and Yeast, Alfred Benzon Symposium, 16, Copenhagen (1981));

(3) Additional genetic units such as a yeast leader peptide may be included on immunoglobulin DNA constructs to obtain antibody secretion.

(4) A portion of the sequence, frequently the first 6 to 20 codons of the gene sequence may be modified to represent preferred yeast codon usage.

(5) The chimeric genes are placed on plasmids used for integration into yeast chromosomes.

The following approaches can be taken to simultaneously express both light and heavy chain genes in yeast.

(1) The light and heavy chain genes are each attached to a yeast promoter and a terminator sequence and placed on the same plasmid. This plasmid can be designed for either autonomous replication in yeast or integration at specific sites in the yeast chromosome.

(2) The light and heavy chain genes are each attached to a yeast promoter and terminator sequence on separate plasmids containing different selective markers. For example, the light chain gene can be placed on a plasmid containing the trp1 gene as a selective marker, while the heavy chain gene can be placed on a plasmid containing ura3 as a selective marker. The plasmids can be designed for either autonomous replication in yeast or integration at specific sites in yeast chromosomes. A yeast strain defective for both selective markers is either simultaneously or sequentially transformed with the plasmid containing light chain gene and with the plasmid containing heavy chain gene.

(3) The light and heavy chain genes are each attached to a yeast promoter and terminator sequence on separate plasmids each containing different selective markers as described in (2) above. A yeast mating type "a" strain defective in the selective markers found on the light and heavy chain expression plasmids (trp1 and ura3 in the above example) is transformed with the plasmid containing the light chain gene by selection for one of the two selective markers (trp1 in the above example). A yeast mating type "alpha" strain defective in the same selective markers as the "a" strain (i.e. trp1 and ura3 as examples) is transformed with a plasmid containing the heavy chain gene by selection for the alternate selective marker (i.e. ura3 in the above example). The "a" strain containing the light chain plasmid (phenotype: $Trp^+$ $Ura^-$ in the above example) and the strain containing the heavy chain plasmid (phenotype: $Trp^-$ $Ura^+$ in the above example) are mated and diploids are selected which are prototrophic for both of the above selective markers ($Trp^+$ $Ura^+$ in the above example).

Among bacterial hosts which may be utilized as transformation hosts, E. coli K12 strain 294 (ATCC 31446) is particularly useful. Other microbial strains which may be used include E. coli X1776 (ATCC 31537). The aforementioned strains, as well as E. coli W3110 (ATCC 27325) and other enterobacteria such as Salmonella typhimurium or Serratia marcescens, and various Pseudomonas species may be used.

In general, plasmid vectors containing replicon and control sequences which are derived from species compatible with a host cell are used in connection with these hosts. The vector ordinarily carries a replication site, as well as specific genes which are capable of providing phenotypic selection in transformed cells. For example, E. coli is readily transformed using pBR322, a plasmid derived from an E. coli species (Bolivar, et al., Gene, 2: 95 (1977)). pBR322 contains genes for ampicillin and tetracycline resistance, and thus provides easy means for identifying transformed cells. The pBR322 plasmid or other microbial plasmids must also contain, or be modified to contain, promoters which can be used by the microbial organism for expression of its own proteins. Those promoters most commonly used in recombinant DNA construction include the beta-lactamase (penicillinase) and lactose (beta-galactosidase) promoter systems (Chang et al., Nature, 275: 615 (1978); Itakura et al., Science, 198:1056 (1977)); and tryptophan promoter systems (Goeddel et al., Nucleic Acids Research, 8: 4057 (1980); EPO Publication No. 0036776). While these are the most commonly used, other microbial promoters have been discovered and utilized.

For example, a genetic construct for any heavy or light chimeric immunoglobulin chain can be placed under the control of the leftward promoter of bacteriophage lambda ($P_L$). This promoter is one of the strongest known promoters which can be controlled. Control is exerted by the lambda repressor, and adjacent restriction sites are known.

The expression of the immunoglobulin chain sequence can also be placed under control of other regulatory sequences which may be "homologous" to the organism in its untransformed state. For example, lactose dependent E. coli chromosomal DNA comprises a lactose or lac operon which mediates lactose digestion by elaborating the enzyme beta-galactosidase. The lac control elements may be obtained from bacteriophage lambda pLAC5, which is infective for E. coli. The lac promoter-operator system can be induced by IPTG.

Other promoter/operator system or portions thereof can be employed as well. For example, arabinose, colicine E1, galactose, alkaline phosphatase, tryptophan, xylose, tac, and the like can be used.

Other preferred hosts are mammalian cells, grown in vitro in tissue culture, or in vivo in animals. Mammalian cells provide post-translational modifications to immunoglobulin protein molecules including leader peptide removal, correct folding and assembly of heavy and light chains, glycosylation at correct sites, and secretion of functional antibody protein from the cell as $H_2L_2$ molecules.

Mammalian cells which may be useful as hosts for the production of antibody proteins include cells of fibroblast origin, such as Vero (ATCC CRL 81) or CHO-K1 (ATCC CRL 61), or cells of lymphoid origin, such as the hybridoma Sp2/0-Ag14 (ATCC CRL 1581) or the myeloma P3X63Ag8 (ATCC TIB 9), and its derivatives.

Several possible vector systems are available for the expression of cloned heavy chain and light chain genes in mammalian cells. One class of vectors utilizes DNA elements which provide an autonomously replicating extrachromosomal plasmid, derived from animal viruses, such as bovine papillomavirus (Sarver, N. et al., Proc. Natl. Acad. Sci., USA, 79: 7147 (1982)), polyoma virus (Deans, R. J. et al., Proc. Natl. Acad. Sci., USA, 81: 1292 (1984)), or SV40 virus (Lusky, M. and Botchan, M., Nature, 293: 79 (1981)). A second class of vectors relies upon the integration of the desired gene sequences into the host cell chromosome. Cells which have stably integrated the introduced DNA into their chromosomes can be selected by also introducing drug resistance genes such as E. coli gpt (Mulligan, R. C. and Berg, P., Proc. Natl. Acad. Sci., USA, 78: 2072 (1981)) or Tn5 neo (Southern, P. J. and Berg, P., J. Mol. Appl. Genet., 1: 327 (1982)). The selectable marker gene can be either directly linked to the DNA gene sequences to be expressed, or introduced into the same cell by co-transfection (Wigler, M. et al., Cell, 16: 77 (1979)).

Since an immunoglobulin cDNA is comprised only of sequences representing the mature mRNA encoding an antibody protein or its precursor, additional gene expression elements regulating transcription of the gene and processing of the RNA are required for optimal synthesis of immunoglobulin mRNA. These elements may include splice signals, as well as transcription promoters including inducible promoters, enhancers, and termination signals. cDNA expression vectors incorporating such elements include those described by Okayama, H. and Berg, P., Mol. Cell Biol., 3: 280 (1983); Cepko, C. L. et al., Cell, 37: 1053 (1984); and Kaufman, R. J., Proc. Natl. Acad. Sci., USA, 82: 689 (1985).

An approach to evaluate optimal vectors for the expression of immunoglobulin cDNA in mammalian cells involves first placing the immunoglobulin DNA sequences into vectors capable of stably integrating into the cell genome, or replicating autonomously as an extrachromosomal plasmid. The vectors can be used to evaluate different gene expression elements for optimal immunoglobulin synthesis.

An additional advantage of mammalian cells as hosts is their ability to express chimeric immunoglobulin genes which are derived from genomic sequences. Thus, mammalian cells may express chimeric immunoglobulin genes which are comprised of a variable region cDNA module plus a constant region which is composed in whole or in part of genomic sequences. Several human constant region genomic clones have been described (Ellison, J. W. et al., Nucl. Acids Res., 10: 4071 (1982), or Max, E. et al., Cell, 29: 691 (1982)). The use of such genomic sequences may be convenient for the simultaneous introduction of immunoglobulin enhancers, splice signals, and transcription termination signals along with the constant region gene segment.

Different approaches can be followed to obtain complete $H_2L_2$ antibodies.

First, one can separately express the light and heavy chains followed by in vitro assembly of purified light and heavy chains into complete $H_2L_2$ IgG antibodies. The assembly pathways used for generation of complete $H_2L_2$ IgG molecules in cells have been extensively studied (see, for example, Scharff, M., Harvey Lectures, 69: 125 (1974)). In vitro reaction parameters for the formation of IgG antibodies from reduced isolated light and heavy chains have been defined by Beychok, S., Cells of Immunoglobulin Synthesis, Academic Press, New York, page 69, 1979.

Second, it is possible to co-express light and heavy chains in the same cells to achieve intracellular association and linkage of heavy and light chains into complete $H_2L_2$ IgG antibodies. The co-expression can occur by using either the same or different plasmids in the same host.

The methods described herein can also be used to switch the class of any antibody of a given specificity and class to an antibody of the same specificity but of a different class, whether human or non-human. For example, human IgM antibodies can be transmuted to human IgG antibodies by preparing constructs containing human constant IgG cDNA or genomic sequences, linked to variable human cDNA sequences obtained from a cell producing the original IgM antibody. These constructs are then introduced into appropriate hosts and expressed.

## POLYPEPTIDE PRODUCTS

The invention provides "chimeric" immunoglobulin chains, either heavy or light. A chimeric chain contains a constant region substantially similar to that present in the heavy chain of a natural human immunoglobulin, and a variable region having any desired antigenic specificity. The variable region is either from human or non-human origin.

The invention also provides immunoglobulin molecules having heavy and light chains associated so that the overall molecule exhibits desired binding and recognition properties. Various types of immunoglobulin molecules are provided: monovalent, divalent, dispecific (i.e., with different variable regions), molecules with chimeric heavy chains and non-chimeric light chains, or molecules with variable binding domains attached to peptide moieties carrying desired functions.

Antibodies having chimeric heavy chains of the same or different variable region binding specificity and non-chimeric (i.e., all human or all non-human) light chains, can be prepared by appropriate association of the needed polypeptide chains. These chains are individually prepared by the modular assembly methods of the invention.

## USES

The antibodies of the invention having human constant region can be utilized for passive immunization, especially in humans, without negative immune reactions such as serum sickness or anaphylactic shock. The antibodies can, of course, also be utilized in prior art immunodiagnostic assays and kits, in labelled form for in vivo imaging, wherein the label can be a radioactive emitter, or an NMR contrasting agent such as a carbon-13 nucleus, or an X-ray contrasting agent, such as a heavy metal nucleus. The antibodies can also be used for in vitro localization of antigens by appropriate labelling.

The antibodies can be used for therapeutic purposes by themselves in complement mediated lysis or can be coupled to toxins or toher therapeutic moieties.

Class switching of antibodies is useful when it is desired to change the association, aggregation or other properties of antibodies obtained from cell fusion or hybridoma technology. For example, most human-human monoclonals are of the IgM class, which are known for their ease of reduction and aggregation. Changing such antibodies to other antibody types, such as IgG, IgA or IgE, is thus of great benefit.

Mixed antibody-enzyme molecules can be used for immunodiagnostic methods, such as ELISA. Mixed antibody-peptide effector conjugates can be used for targeted delivery of the effector moiety with a high degree of efficacy and specificity.

Having now generally described the invention, the same will be further understood by reference to certain specific examples which are included herein for purposes of illustration only and are not intended to be limiting unless otherwise specified.

## EXPERIMENTAL

### Materials and Methods

#### Tissue Culture Cell Lines

The human cell lines GM2146 and GM1500 were obtained from the Human Mutant Cell Repository (Camden, New Jersey) and cultured in RPMI1640 plus 10% fetal bovine serum (M. A. Bioproducts). The cell lines Sp2/0 and CRL 8017 were obtained from the American Type Culture Collection and grown in Dulbecco's Modified Eagle Medium (DMEM) plus 4.5 g/l glucose (M. A. Bioproducts) plus 10% fetal bovine serum (Hyclone, Sterile Systems, Logan, Utah). Media were supplemented with penicillin/streptomycin (Irvine Scientific, Irvine, California).

#### Recombinant Plasmid and Bacteriophage DNAs

The plasmids pBR322, pL1 and pUC12 were purchased from Pharmacia P-L Biochemicals (Milwaukee, Wisconsin). The plasmids pSV2-neo and pSV2-gpt were obtained from BRL (Gaithersburg, Maryland), and are available from the American Type Culture Collection (Rockville, Maryland). pHu-gamma-l is a subclone of the 8.3 Kb HindIII to BamHI fragment of the human IgGl chromosomal gene. A separate isolation of the human IgGl chromosomal gene is described by Ellison, J. W. et al., Nucl. Acids Res., 10: 4071 (1982). M8alphaRX12 contains the 0.7 Kb XbaI to EcoRI fragment containing the mouse heavy chain enhancer from the J-C intron region of the M603 chromosomal gene (Davis, M. et al., Nature, 283: 733) inserted into M13mp10. G-tailed pUC9 was purchased from Pharmacia P-L. DNA manipulations involving purification of plasmid DNA by buoyant density centrifugation, restriction endonuclease digestion, purification of DNA fragments by agarose gel electrophoresis, ligation and transformation of E. coli were as described by Maniatis, T. et al., Molecular Cloning: A Laboratory Manual, (1982). Restriction endonucleases and other DNA/RNA modifying enzymes were purchased from Boehringer-Mannheim (Indianapolis, Indiana), BRL, New England Biolabs (Beverly, Massachusetts) and Pharmacia P-L.

#### Oligonucleotide Preparation

Oligonucleotides were either synthesized by the triester method of Ito et al. (Nucl. Acids Res., 10: 1755 (1982)), or were purchased from ELESEN, Los Angeles, California. Tritylated, deblocked oligonucleotides were purified on Sephadex-G50, followed by reverse-phase HPLC with a 0-25% gradient of acetonitrile in 10mM triethylamine-acetic acid, pH 7.2, on a C18 uBondapak column (Waters Associates). Detritylation was in 80% acetic acid for 30 min., followed by evaporation thrice. Oligonucleotides were labeled with [gamma-$^{32}$P]ATP plus T4 polynucleotide kinase.

#### RNA Preparation and Analysis

Total cellular RNA was prepared from tissue culture cells by the method of Auffray, C. and Rougeon, F. (Eur. J. Biochem., 107: 303 (1980)) or Chirgwin, J. M. et al. (Biochemistry, 18: 5294 (1979)). Preparation of poly(A)$^+$ RNA, methyl-mercury agarose gel electrophoresis, and "Northern" transfer to nitrocellulose were as described by Maniatis, T. et al., supra. Total cellular RNA or poly(A)$^+$ RNA was directly bound to nitrocellulose by first treating the RNA with formaldehyde (White, B. A. and Bancroft, F. C., J. Biol. Chem., 257: 8569 (1982)). Hybridization to filterbound RNA was with nick-translated DNA fragments using conditions described by Margulies, D. H. et al. (Nature, 295: 168 (1982)) or with $^{32}$P-labelled oligonucleotide

using 4xSSC, 10X Denhardt's, 100 ug/ml salmon sperm DNA at 37°C overnight, followed by washing in 4xSSC at 37°C.

cDNA Preparation and Cloning

Oligo-dT primed cDNA libraries were prepared from poly(A)$^+$ RNA from GM1500 and GM2146 cells by the methods of Land, H. et al. (Nucl. Acids Res., 9: 2251 (1981)) and Gubler, V. and Hoffman, B. J., Gene, 25: 263 (1983), respectively. The cDNA libraries were screened by in situ hybridization (Maniatis, T., supra) with $^{32}$P-labelled oligonucleotides using the conditions shown above, or with nick-translated DNA fragments using the conditions of de Lange et al. (Cell, 34: 891 (1983)).

Oligonucleotide Primer Extension and Cloning

Poly(A)$^+$ RNA (20 ug) was mixed with 1.2 ug primer in 40 ul of 64mM KCl. After denaturation at 90°C for 5 min. and then chilling in ice, 3 units Human Placental Ribonuclease Inhibitor (BRL) was added in 3 ul of 1M Tris-HCl, pH 8.3. The oligonucleotide was annealed to the RNA at 42°C for 15 minutes, then 12 ul of .05M DTT, .05M $MgCl_2$, and 1 mM each of dATP, dTTP, dCTP, and dGTP was added. 2 ul of alpha-$^{32}$p-dATp (400 Ci/mmol, New England Nuclear) was added, followed by 3 ul of AMV reverse transcriptase (19 units/ul, Life Sciences).

After incubation at 42°C for 105 min., 2 ul 0.5 M EDTA and 50 ul 10mM Tris, 1mM EDTA, pH 7.6 were added. Unincorporated nucleotides were removed by Sephadex G-50 spun column chromatography, and the RNA-DNA hybrid was extracted with phenol, then with chloroform, and precipitated with ethanol. Second strand synthesis, homopolymer tailing with dGTP or dCTP, and insertion into homopolymer tailed vectors was as described by Gubler and Hoffman, supra.

Site-Directed Mutagenesis

Single stranded M13 subclone DNA (1 ug) was combined with 20 ng oligonucleotide primer in 12.5 ul of Hin buffer (7 mM Tris-HCl, pH 7.6, 7 mM $MgCl_2$, 50 mM NaCl). After heating to 95°C in a sealed tube, the primer was annealed to the template by slowly cooling from 70°C to 37°C for 90 minutes. 2 ul dNTPs (1 mM each), 1 ul $^{32}$P-dATP (10 uCi), 1 ul DTT (0.1 M) and 0.4 ul Klenow DNA PolI (2u, Boehringer Mannheim) were added and chains extended at 37°C for 30 minutes. To this was added 1 ul (10 ng) M13 reverse primer (New England Biolabs), and the heating/annealing and chain extension steps were repeated. The reaction was stopped with 2 ul of 0.5M EDTA, pH 8, plus 80 ul of 10 mM Tris-HCl, pH 7.6, 1 mM EDTA. The products were phenol extracted and purified by Sephadex® G-50 spun column chromatography and ethanol precipitated prior to restriction enzyme digestion and ligation to the appropriate vector.

Transfection of Myeloma Tissue Culture Cells

A variation of the method of Ochi, A. et al. (Nature, 302: 340 (1983)) was used for protoplast fusion. 50 ml of bacteria at $A_{600}$ of 0.7 were converted to protoplasts by the method of Sandri-Goldin, R. M. et al. - (Mol. Cell. Biol., 1: 743 (1981)), then diluted with 20 ml DMEM plus 10% FBS (final volume is 25 ml). Sp2/0 cells were harvested, pelleted at 2,200 x g, washed, repelleted and resuspended in DMEM at 2-5x10$^6$/ml. Bacterial protoplasts (10 ml) were mixed with 10x10$^6$ Sp2/0 cells and pelleted by centrifugation at 4,000 x g at 22°C for 20 min. After pipetting off the supernatant, the pellet was suspended in the remaining drop of medium by flicking the tube. 2ml of 10% DMSO, 37% (w/v) PEG6000 (Kodak) in DMEM was added dropwise with mixing over 45 sec. After 15 sec., 2 ml of 42% PEG6000 in DMEM was added over 45 sec. Complete DMEM (45 ml) was slowly added with mixing. Cells were pelleted at 2500 x g, then washed and pelleted thrice.

The electroporation method of Potter, H. et al. (Proc. Natl. Acad. Sci., USA, 81: 7161 (1984)) was used. After transfection, cells were allowed to recover in complete DMEM for 48-72 hours, then were seeded at 10,000 to 50,000 cells per well in 96-well culture plates in the presence of selective medium. G418 (GIBCO) selection was at 0.8 mg/ml, mycophenolic acid (Calbiochem) was at 6 ug/ml plus 0.25 mg/ml xanthine, and HAT (Sigma) was at the standard concentration.

## Assays for Immunoglobulin Synthesis and Secretion

Secreted immunoglobulin was measured directly from tissue culture cell supernatants. Cytoplasmic protein extract was prepared by vortexing $1x10^6$ cells in 160 ul of 1% NP40®, 0.15 M NaCl, 10 mM Tris, 1 mM EDTA, pH 7.6 at 0 °C, 15 minutes, followed by centrifugation at 10,000 x g to remove insoluble debris.

Double antibody sandwich ELISA (Voller, A. et al., in Manual of Clinical Immunology, 2nd Ed., Eds. Rose, N. and Friedman, H., pp. 359-371, 1980) using affinity purified antisera was used to detect specific immunoglobulins. For detection of human IgG, the plate-bound antiserum is goat anti-human IgG (KPL, Gaithersburg, Maryland) at 1/1000 dilution, while the peroxidase-bound antiserum is goat anti-human IgG (KPL or Tago, Burlingame) at 1/4000 dilution. For detection of human immunoglobulin kappa, the plate-bound antiserum is goat anti-human kappa (Tago) at 1/500 dilution, while the peroxidase-bound antiserum is goat anti-human kappa (Cappel) at 1/1000 dilution.

Antibodies binding hepatitis B surface antigen were detected using a commercial (Abbott, AUSAB) assay.

## EXAMPLES

The following examples show the preparation of chimeric antibodies each having a human constant region and a non-human variable region. These examples outline the step-by-step process of preparing the chimeric antibodies.

## EXAMPLE I: Human Antibody Constant Region Gene Modules and cDNA Expression Vectors

### (1) Preparation of cDNA Clones, and Vehicles Containing Same, for Heavy Chain Human Constant Region

The cell line GM2146 was used as the source in mRNA preparation and cDNA cloning. This cell line secretes IgGl (Simmons, J. G. et al., Scand. J. Immunol., 14: 1-13, 1981). Tests of this cell line indicated that it secretes IgA as well as IgG.

The cell line was cloned, and results indicated that five of six subclones secreted IgG only, while one of six subclones secreted IgA only. Poly(A)$^+$ RNA was prepared from the cell line and a cDNA library was prepared from the poly(A)$^+$ RNA by the method of Gubler, U. and Hoffman, B. J., Gene, 25: 263-269 (1983). An initial plating of the cDNA transformed into E. coli strains HB101 and RR1 yielded a total of 1500 colonies, which were screened by hybridization to a HindIII to BamHI fragment of a genomic clone of human IgGl (pHu-gamma-1). Four positive clones were found. A fragment containing the CH3 coding region of one of these clones, pGMH-3 (Figure 4), was used to rescreen the original library plus a new transformation of approximately 5000 colonies. Two of the largest clones, pGMH-6 and pGMH-15, were analyzed by restriction enzyme digestion (Figure 4). Both clones contained the entire constant region of human IgGl, although it was discovered that pGMH-6 had deleted approximately 1500 base pairs of pBR322 DNA, apparently without affecting the IgGl cDNA sequences.

Clone pGMH-6 provided the IgGl constant region module in the construction of cloning vectors for heavy chain variable region cloning.

### (2) Preparation of cDNA Clones, and Vehicles Containing Same, for Light Chain Human Constant Region

A human cell line (GM1500) producing $IgG_2K$ was selected for the initial cloning phase. Poly(A)$^+$ RNA prepared from GM1500 is active in in vitro translation using rabbit reticulocyte extracts. A cDNA library was prepared from this RNA by the method of Land et al., Nucl. Acids Res., 9: 2251-2266 (1981), utilizing KpnI digested and dG-tailed pQ23 as the cloning vector (Figure 5). This vector contains BglII, KpnI and SstI sites inserted between the BamHI and SalI sites of pBR322.

In order to identify the cDNA clones generated from GM1500 RNA which correspond to light chain mRNA, a DNA probe, UIG-HuK, was synthesized and purified. The UIG-HuK oligonucleotide has the sequence 5'-AGCCACAGTTCGTTT-3', and is designed to hybridize to all functional human kappa mRNA species at the J-C junction. This probe was used to prime cDNA synthesis on GM1500 RNA in the presence of dideoxynucleotides and reverse transcriptase. From 1.2 ug of total GM1500 poly(A)$^+$ RNA was used in this experiment, the entire J sequence and some of the V region was read, demonstrating that (1) GM1500 RNA is intact, (2) the kappa probe is of the correct sequence, and (3) GM1500 light chain mRNA contains $J_K4$ sequences.

cDNA clones positive for hybridization to the light chain probe were selected. Since the probe hybridizes to the J-C junction, the most important point was to determine if the clones had complete constant region sequence in addition to the J region.

Insert sizes for the two largest kappa cDNA clones were 0.6 and 0.9 kb; restriction enzyme mapping indicated that the entire constant region coding sequence was present in both clones (Figure 6). The human kappa cDNA clone pK2-3 was used to make the light chain constant region vector pING2001 by inserting the Sau3A fragment comprising the human kappa constant and J regions into the BclI site of pBR325 (Figure 6B).

A variant of the human kappa cDNA clone was made by placing a HindIII site in the J region. This was carried out by in vitro mutagenesis using a $J_K$HINDIII oligonucleotide primer (Figure 7c). The resultant plasmid is pGML60.

A vector, pING2003, was constructed for the transfer and expression of cDNA sequences in mammalian cells (Figure 10). This vector was constructed from pUC12 and two plasmids containing SV40 sequences. pL1 provides an SV40 early region promoter and an SV40 late region splice sequence. pSV2-neo sequences provide a selectable marker for mammalian cell transformation and SV40 polyadenylation signal sequences. pUC12 provides a multiple cloning site for cDNA insertion.

The pING2003 vector has several useful restriction sites for modifications. These include a HindIII site useful for the insertion of enhancer sequences, and a HindIII to XhoI fragment useful for the insertion of alternate promoter sequences. This vector is useful in the expression of cDNA genes in mammalian cells.

Addition of Enhancer Element to pING2003

Immunoglobulin enhancer elements have been shown to enhance transcription of genes in their vicinity in stably transformed mouse myeloma cells by several hundred fold (Gillies, S. D. et al., Cell, 33: 717, 1983; and Banerji, J. et al. Cell, 33: 729, 1983). To facilitate expression of the mouse- human immunoglobulin genes in mouse myeloma cells, the mouse immunoglobulin heavy chain enhancer element was added to the cDNA expression vector pING2003 (Figure 10). The mouse heavy chain enhancer region DNA was isolated from an M13 subclone of mouse heavy chain genomic DNA (M8-alpha-RX12, Deans, R. J., unpublished). DNA isolated from a SalI plus EcoRI digestion of this subclone was modified with HindIII linkers and inserted into the HindIII site of pING2003, resulting in the new cDNA expression vector pING2003E. This vector is useful in the efficient expression of cDNA genes in mammalian cells, particularly mouse myeloma or hybridoma cell lines.

EXAMPLE II: Human-Mouse Chimeric Anti-HBsAG Antibody Chain

(1) Preparation of cDNA Clones and Vehicles Containing Same, for Heavy Chain Mouse Anti-HBsAg Variable Region.

The cell line CRL8017 was obtained from the ATCC and subcloned. Subclones were grown and tested for mouse IgG anti-hepatitis B binding activity using a commercially available anti-HBsAg detection kit. Three positive subclones were found. Poly(A)$^+$ RNA was prepared from one of these subclones, and was fractionated on a methylmercury agarose gel. The RNA contained intact light chain and heavy chain mRNA's as inferred from specific hybridization to kappa UIG-MJK primer, and to the mouse heavy chain UIG-MJH3 probe (see Figure 7). In addition, the UIG-MJK primer was used for specific priming of anti-HBsAg poly(A)$^+$ RNA in a dideoxy sequencing reaction. Sufficient sequence was read to show that a major kappa RNA of the anti-HBsAg cell line contains the $J_K2$ sequence.

The conditions for variable region cDNA synthesis were optimized by using heavy and light chain UIG primers on anti-HBsAg poly(A)$^+$ RNA. Dideoxy chain extension experiments demonstrated that the mouse UIG-MJK primer and UIG-JH3 primer correctly primed kappa and heavy chain RNAs. When the reverse transcription was carried out in the absence of dideoxynucleotides, the main product using the kappa UIG-MJK primer was a 410±20 nucleotide fragment, while the main product using the heavy chain UIG-JH3 primer was a 430±30 nucleotide fragment. These correspond to the expected lengths of the variable and 5' untranslated regions of kappa and heavy chain immunoglobulin mRNAs. The conditions for the optimal priming of poly(A)$^+$ RNA from CRL8017 cells should work well for poly(A)$^+$ RNA isolated from any cell line producing a monoclonal antibody.

After determining optimal conditions for priming hybridoma mRNA with oligonucleotide primers, two oligonucleotides were designed and used for heavy chain V region cDNA synthesis. These two oligonucleotides are UIG-MJHBSTEII(13) and UIG-MJH3 (Figures 7 and 8). It should be noted that the primer sequence was designed to introduce a BstEII recognition site (GGTGACC) in the clone so that it could be joined at this site to the human IgGl constant module at the analogous position at the latter's J region. In this case, the primer had a single G to U mismatch with the mouse mRNA sequence that uses the $J_H3$ coding sequence. The UIG-MJHBSTEII(13) primer was 13 bases long and the mismatched residue was flanked by 7 matches 5' and 5 matches 3' of it. This was the 13-mer BstEII primer. To assess the priming efficiency of the 13-mer BstEII oligonucleotide, a 21-mer primer specific for mouse $J_H3$ (UIG-MJH3) was used. This primer had a perfect match for the 17 nucleotides on its 3' end.

These two primers and the $J_H3$ coding sequences are shown in Figure 8. The first strand cDNA products made via the 13-mer BstEII and the 21-mer $J_H3$ primers included bands of approximately 430 nucleotides, which represented the entire $V_H$ region. Under the standard priming conditions used, the priming efficiency of the 13-mer BstEII was much less than that of the 21-mer $J_H3$. Accordingly, a cDNA library was generated from the first strand synthesis from each of these primers, using the method of Gubler and Hoffman, supra.

First, the 21-mer $J_H3$ library was screened with the 21-mer $J_H3$ oligonucleotide. Filter hybridization was done at 30°, overnight, according to de Lange, T. et al., Cell, 34: 891-900 (1983). The filters were then washed at 51° in 6 x SSC, 0.1% SDS. Five colonies were selected. The largest had an insert of approximately 460 bp. More significantly, it contained three restriction sites predicted from the known $J_H3$ sequence, which are present upstream of the primer sequence. This clone, pJ3-11, was sequenced using the $J_H3$ primer by the chain-termination method (Wallace, R. B. et al., Gene, 16: 21-26 (1981)). The sequence obtained has the remaining $J_H3$ coding segment. Just upstream, a 13-nucleotide segment matched to a published D segment sequence (Dsp 2.2) (Kurosawa, Y. et al., J. Exp. Med., 155: 201 (1982), and Tonegawa, S., Nature, 302: 575 (1983)). A nonapeptide predicted from this area showed characteristic homology to the published mouse heavy chain V subgroups at amino acid residues 86 to 94, comprising the FR3 of heavy chain molecules. Plasmid pJ3-11 represented a rearranged VDJ sequence, and apparently contained the anti-hepatitis $V_H$ sequence produced by the cell line.

In order to isolate a $V_H$ region cDNA clone that had a BstEII site in the J region, an AluI to Sau96I, 265 nucleotide long, probe from pJ3-11 was next used to screen the cDNA library generated from the 13-mer BstEII primer. Six positive clones were isolated. The largest, pBs13-1, was further analyzed. The insert was 280 nucleotides long and its restriction map agreed with that of pJ3-11 except for the introduced BstEII site. Figure 9 illustrates how these two inserts were recombined to generate pMVHCa-13, a $V_H$ clone with the module-joining BstEII site. Three additional $V_H$ cDNA clones were isolated from a cDNA library generated from the 21-mer oligonucleotide UIG-MJH3BSTEII primer containing a BstEII site. These clones may provide alternate $V_H$ cDNA sequences to join to human $C_H$ sequences.

(2) Preparation of cDNA Clones, and Vehicles Containing Same, for Light Chain Mouse Anti-HBsAg Variable Region

Since the $J_K2$ sequence is present in mRNA prepared from the anti-hepatitis hybridoma cell line, the oligonucleotide UIG-JK2BGLII (Figure 7B), was designed to introduce a BglII site into the $J_K2$ region. Digestion with BglII would then allow direct insertion of a $V_K$ cDNA coding region into the BclI site of the previously noted human $C_K$ vector, pING2001. This insertion would result in the precise joining of a mouse variable region segment (including the J region) to a human kappa constant region segment, each in the proper coding frame and with no alteration in amino acid sequence for either mouse variable or human constant region.

The JK2BGLII oligonucleotide was used to prime anti-HBsAg mRNA to form a cDNA library as for heavy chain, supra, in pUC9. The cDNA was size-selected by polyacrylamide gel electrophoresis prior to cloning, and 80% of the cDNA clones were shown to have insert sizes between 300 and 750 nucleotides in length. Replica filters of this library were screened with two oligonucleotides, the original primer and a second probe complementary to $J_K2$ sequence 5' to the original primer.

It was discovered that the anti-hepatitis B monoclonal cell line CRL 8017 secretes immunoglobulins with at least two different light chains. One of them is derived from the myeloma NS-1, which was used as a fusion partner in generating the anti-hepatitis B cell line. Since NS-1 is derived from the myeloma MOPC21, the possibility was investigated that MOPC21 $V_K$ mRNA may be present in the $V_K$ cDNA library from the antihepatitis monoclonal cell line. Indeed, one cDNA clone (p6D4B) analyzed has an identical restriction enzyme map to that of MOPC21 $V_K$ cDNA, except for the inserted BglII site.

Two conclusions can be drawn from these results. The first is that it is possible to effectively use an oligonucleotide to introduce a restriction enzyme site while cloning a $V_K$ region from a hybridoma cell line. The second is that one must carefully monitor hybridoma cell lines for the presence of multiple V region sequences, only one of which is the desired sequence.

In order to further characterize the kappa light chain J regions present in the cell line mRNA, poly(A)+ RNA was bound to nitrocellulose by the formaldehyde "Dot blot" procedure of White and Bancroft, J. Biol. Chem., 257: 8569 (1982). The RNA was hybridized to $^{32}$P-labeled oligonucleotide probes specific for each functional kappa J region. These probes are shown in Figure 7B as the UIG probes 5JK1, MJK, 5JK4, and 5JK5. The results showed that the mRNA hybridized strongly to both MJK and 5JK4 oligonucleotide probes, indicating that both $J_K2$ and $J_K4$ sequences were present. Since $J_K2$ mRNA had been previously identified as the one derived from the parental hybridoma partner NS-1, it was concluded that the $J_K4$ mRNA encoded the anti-hepatitis binding specificity of the CRL 8017 cells.

Two different cDNA libraries were screened to isolate V region clones encoding $J_K4$ sequences. The first was primed by JK2BGLII, supra. The second was made by using thee oligonucleotide primer, JK4BGLII, which is specific fur $J_K4$ mRNA and introduces a BglII site into the J region of cloned V regions. The JK4BGLII primer was used to prime first strand cDNA synthesis to construct a cDNA library by the same method used to construct a JK2BGLII primed cDNA library, except that cDNA was not size selected prior to cloning.

Figure 7B tabulates the mismatches that each primer has with other functional mouse kappa J region sequences. Note that $J_K4$ has five mismatches in 21 nucleotides when compared with the JK2BGLII primer, and 3 in 23 with the JK4BGLII primer.

Both libraries were screened for V region clones containing $J_K4$ sequences by hybridizing to an oligonucleotide probe specific for $J_K4$ sequences (5JK4). The results of this screen are shown in Table 1.

Table 1*

| Library | Probe Specificity | |
|---|---|---|
| | $J_K2$ | $J_K4$ |
| JK2BGLII | 2% (30/1500) | 0.15% (2/1500) |
| JK4BGLII | N/D | 3.5% (31/875) |

* Percentage of clones containing $J_K2$ or $J_K4$ sequence plus a V region. The probes used were the oligonucleotide 5JK4 ($J_K4$ specificity, Figure 7) and p6D4B, which contains the NS-1 (MOPC21) V region sequence. N/D, not done.

Several $J_K4$ V region cDNA clones isolated from both libraries were characterized. These clones have identical restriction enzyme maps, including the engineered BglII site resulting from the oligonucleotide primed cDNA cloning procedure. The restriction map and sequence of one clone, pV17, show that pV17 contains V region gene sequences.

These results show that the JK2BGLII primer could correctly, although inefficiently, prime $J_K4$ mRNA sequences. Since the JK2BGLII primer had less mismatches with any other $J_K$ region mRNA than with $J_K4$ mRNA (Figure 7B), it is expected that the other $J_K$ mRNAs can be primed at the correct location with better efficiency using the JK2BGLII primer. Thus, efficient cDNA cloning of any functional mouse kappa V region may be obtained by using a mixture of the JK2BGLII and JK4BGLII primers.

The placement of a BglII site into the J region during cDNA cloning of the V regions allows joining of the cloned mouse V region gene module to the human kappa constant region gene module (Figure 9B).

After the aforementioned experiments were carried out it was found that the cDNA clone pV17 lacked a complete 5' coding region. Nucleotide sequencing showed that the A of the initiator codon ATG was not copied in pV17. This was not a random cDNA cloning artifact because two other cDNA clones had the same defect. Two approaches were devised to obtain a light chain gene with a complete 5' coding region.

First, a new cDNA library was constructed by first priming with an oligonucleotide (5'-ATATTTGCT-GATGCT CT-3') complementary to pV17 sequences 155 bases from the 5' end. From this library, clones hybridizing to a pV17 DNA fragment probe were selected, and some of these new cDNA clones have the initiator ATG plus about 20 nucleotides of 5' untranslated region. One of these clones, p2-12, supplies a 5' untranslated region of 23 nucleotides and a complete ATG initiator codon. When p2-12 was combined with pV17 derived sequences, a variable region with a complete 5' end was formed (pING2013E).

Second, site-directed mutagenesis on the existing light chain clone was used to simultaneously remove the poly-G tract and place a ribosome recognition sequence adjacent to the initiator ATG. The PstI fragment from pV17 was subcloned into M13mp18. An oligonucleotide (V17-IVM); 5'-GTGTCGACTCAGCATGAGGTTCC AGGTTC-3') was then used as a primer to mutate the pV17 sequence to include a SalI site and an initiator ATG into the pV17 sequence. The resultant plasmid pV17-IVM provided an alternate mouse variable region for joining to human constant region modules.

The complete nucleotide sequence of the variable region from pV17 was then determined. The sequence shows that pV17 contains a $V_K$-$J_K$ junction region, containing several conserved amino acids, and the hybrid $J_K2/J_K4$ region formed by priming the $J_K4$ RNA with the UIG-JK2BGLII oligonucleotide. However, the $V_K$ region in pV17 is non-functional, because the $V_K$ and $J_K$ regions are not in the same coding frame. Translation of the pV17 V region would thus result in an abnormal immunoglobulin light chain where the J region is translated in an incorrect frame. This defect may be caused by aberrant V-J joining, resulting in a non-functional kappa mRNA, as has been observed by Kelley, D.E. et al., Mol. Cell. Biol., 5:1660-1675 (1985).

Since the pV17 V region encodes an abnormal immunoglobulin, it is highly unlikely that this light chain is part of a functional anti-hepatitis antibody molecule. These results show the importance of monitoring hybridoma cells for the presence of multiple RNA species encoding V regions, only one of which is the desired sequence.

Further screening of CRL 8017 cDNA libraries was done to search for $V_K$ cDNA clones which are not from either of the two $V_K$ cDNA classes found so far (MOPC21-p6D4B, pV17). First an oligo-dT primed cDNA library made from CRL8017 RNA was screened with a DNA fragment probe specific for the kappa constant region, and separately with probes specific for MOPC21 and pV17 $V_K$ regions. A cDNA clone (p1E9L-81) that contains the kappa constant region, but has a different $V_K$ region than that of MOPC21 or pV17 was discovered. This method of screening oligo-dT primed cDNA libraries is a useful alternative to oligonucleotide screening of cDNA libraries, because nick-translated probes of high specific activity are used. Also, this method allows the simultaneous isolation of several classes of V region clones, such as all $V_K$ clones, by appropriate probe choice. Second, the UIG-JK2BGLII-primed cDNA library made from CRL 8017 RNA was screened with the UIG-5JK2 oligonucleotide probe (see Figure 7). A new class of $V_K$ cDNA clones was found whose members are homologous to p1E9L-81 and hybridize to the UIG-5JK2 probe, but not to a MOPC21 $V_K$ probe. The restriction endonuclease site maps and nucleotide sequences of these clones also differ from MOPC21-homologous $V_K$ cDNA clones from CRL8017 cells. These clones, however, have an aberrant V-J joint which results in a nonfunctional mRNA, and appear to be identical to one described by Cabilly and Riggs (Gene, 40:157 (1985)).

It was therefore concluded that the anti-hepatitis B cell line CRL8017 has at least three classes of $V_K$ mRNA corresponding to the above described cDNA clones p6D4B (MOPC21), p1E9L, and pV17. The pIE9L and pV17 clones are derived from mRNA from aberrantly rearranged Kappa genes, while the p6D4B clone is derived from the parent hybridoma fusion partner NS-1. None of these clones appear to encode the desired anti-hepatitis light chain.

(3) Preparation and Expression of Heavy Chain Containing Human Constant/Mouse Variable Regions

The V region sequences in pMVHCa-13 were joined to the human IgGl constant (C) region clone pGMH-6. Due to the presence of a second BstEII site within the IgGl CH1 region of pGMH-6, a multi-step ligation was required. First, the 220 nucleotide BstEII fragment from the J-CH1 region of pGMH-6 was ligated to the 1100 nucleotide IgG region BstEII to BamHI fragment of pGMH-6. In a separate ligation, the 420 nucleotide BstEII to BamHI fragment of pMVHCa-13, which comprises the mouse V region, was joined to a calf intestine phosphatase treated BamHI plasmid vector. The two ligations were then combined, ligase was added, and the products were transformed into HB101, resulting in the chimeric mouse V-human C clone pMVHCc-24 (Figure 9A).

The V region of the hybrid heavy chain gene in pMVHCc-24 was further analyzed by partial sequence analysis. This analysis showed that the cloned V region contained a D sequence which matches a known D sequence, DSP2.2 (Kurosawa and Tonegawa, supra). The sequence also predicted a 19 amino acid leader peptide similar to known mouse V heavy chain leader peptide sequences, and a 5' untranslated region of at least 3 nucleotides.

The BamHI fragment containing the mouse-human hybrid heavy chain gene of pMVHCc-24 was cloned into BamHI digested pING2003E vector, resulting in the expression plasmid pING2006E (Figure 11). The pING2006E plasmid should have an increased probability of efficient expression of the mouse-human chimeric immunoglobulin gene in B lymphoid cells because of the presence of the mouse heavy chain enhancer region.

A modification of the chimeric heavy chain gene present in pMVHCc-24 was done to provide an alternate heavy chain gene which lacks the oligo-dC region preceding the initiator ATG. The pING2012E

and pING2006E vectors are identical except for the nucleotides immediately preceding the ATG, as shown in Figure 12.

Bacteria harboring the pING2006E and pSV2-neo plasmids were converted into protoplasts by the method of Sandri-Goldin, R. M. et al., Mol. Cell. Biol., 1: 743 (1981). The protoplasts were then separately fused to SP2/0-Ag14 hybridoma cells (ATCC CRL 1581) by treatment with polyethyleneglycol (Ochi, A. et al., Nature, 302: 340, 1983). The fused cells were allowed to recover for 72 hours in complete medium before plating at 10,000 or 50,000 cells per well in a 96-well tissue culture plate. The cells were selected with G418 at 0.8 mg/ml for two weeks, when growth in some wells was clearly evident. Under these selection conditions, Sp2/0 cells were completely killed within 4-7 days by G418. Only cells which have integrated and expressed the neo gene present in the vectors will grow under G418 selection. The number of wells positive for growth by these integrative transfectants are shown in Table 2.

Table 2*

| Strain/Plasmid | 10,000 cells/well | 50,000 cells/well |
|---|---|---|
| MC1061/pING2006E | 3 (13%) | 12 (50%) |
| MC1061/pSV2-neo | 7 (29%) | 4 (17%) |
| MC1061/none | 0 | 0 |

* Percentage of wells showing positive growth out of 24 wells.

Cells transfected with pING2006E and pSV2-neo were tested for immunoglobulin gene expression at the RNA and protein level. Total cell RNA was prepared from transfected cells, bound to nitrocellulose and hybridized to nick-translated probes specific for the mouse-human hybrid heavy chain gene. Two clones were found which have a strong signal, representing expression of the gene at the RNA level. The amount of total cellular RNA hybridizing to the mouse-human probe appeared to be approximately 1/10 the level of heavy chain RNA in the original hybridoma cells. This probably represented about 1% of the total mRNA of the transfected cell.

The transfected mouse cells were also tested for production of cytoplasmic human heavy chain protein by an ELISA assay. It was found that 3 out of 7 pING2006E transfected cell lines produced detectable levels of human heavy chain protein. The mouse cell transformant producing the most mouse-human heavy chain protein gave a signal in the ELISA assay comparable to that of a 1/100 dilution of a human B cell line producing intact human immunoglobulin IgGl. This modest level of detected mouse-human heavy chain protein may be due to several factors, including instability of heavy chains in the absence of light chains in hybridoma cells, or incorrect processing of the chimeric gene transcript.

(4) Gene Amplification of the Integrated Chimeric Gene

Southern blot analysis showed that multiple copies of the pING2006E DNA sequences were integrated in tandem in the mouse genome. Restriction enzymes ApaI and BglII both cleave pING2006E singly. In the transformant, 2AE9, a band, from an ApaI or BglII digestion, of the expected size (8.2kb) was found to hybridize to the human C gamma 1 sequences (data not shown). BamHI band of the correct size (1.6kb) was found to hybridize to the human as well as the 1E9 $V_H$ sequences. Gene-copy titration experiment (Fig. 14) indicated that there are about 5 copies of pING2006E in the 2AE9 genome. That fact that only a single band was detected in the ApaI or BglII lane indicates that these individual copies are in a tandemly arranged array. A set of double digestions showed that pING2006E sequences suffered no rearrangement in their introduction into the mouse DNA (data not shown).

We next transfected the 2AE9 cells with a plasmid that contains a different selectable marker, the gpt gene, and selected clones growing out in DMEM-HAT. One clone, 2BH10, has about 38 ng soluble human gamma 1 protein per $10^6$ cells. Southern analysis showed that 2BH10 has about 30 copies of pING2006E (Fig. 14). They were amplified from the 5 copies in 2AE9 without rearrangement of the DNA sequences. (Compare the 2AE9 panel to the 2BH10). S1 data (data not shown) revealed that this increase in template led to a higher amount of IgG gene transcripts. We believe that these sequences were co-amplified with contiguous cellular sequences as a result of the second selection.

EXAMPLE III: A Human-Mouse Chimeric Antibody with Cancer Antigen Specificity

(1) Antibody L6

L6 monoclonal antibody (MAb) was obtained from a mouse which had been immunized with cells from a human lung carcinoma, after which spleen cells were hybridized with NS-1 mouse myeloma cells. The antibody binds to a previously not identified carbohydrate antigen which is expressed in large amounts at the surface of cells from most human carcinomas, including lung carcinomas (adeno, squamous), breast carcinomas, colon carcinomas and ovarian carcinomas, while the antigen is only present at trace levels in normal cells from the adult host. MAb L6 is an IgG2a and can mediate antibody dependent cellular cytotoxicity, ADCC, in the presence of human peripheral blood leukocytes as a source of effector cells, so as to lyse L6 positive tumor cells, and it can lyse L6 positive tumor cells in the presence of human serum as a source of complement; the lysis is detected as the release of $^{51}Cr$ from labelled cells over a 4 hour incubation period. MAb L6 can localize to L6 positive tumors xenotransplanted onto nude mice, and it can inhibit the outgrowth of such tumors. MAb L6 is described in Cancer Res. 46:3917-3923, 1986 (on MAb specificity) and in Proc. Natl. Acad. Sci. 83:7059-7063, 1986 (on MAb function).

(2) Identification of J Sequences in the Immunoglobulin mRNA of L6.

Frozen cells were thawed on ice for 10 minutes and then at room temperature. The suspension was diluted with 15 ml PBS and the cells were centrifuged down. They were resuspended, after washes in PBS, in 16 ml 3M LiCl, 6M urea and disrupted in a polytron shear. The preparation of mRNA and the selection of the poly(A+) fraction were carried out according to Auffray, C. and Rougeon, F., Eur. J. Biochem. 107:303, 1980.

The poly (A+) RNA from L6 was hybridized individually with labeled $J_H1'$ $J_H2$, $J_H3$ and $J_H4$ oligonucleotides under conditions described by Nobrega et al. Anal. Biochem 131:141, 1983). The products were then subjected to electrophoresis in a 1.7% agarose-TBE gel. The gel was fixed in 10% TCA, blotted dry and exposed for autoradiography. The result showed that the L6 $v_H$ contains $J_H2$ sequences.

For the analysis of the $V_K$ mRNA, the dot-blot method of White and Bancroft J. Biol. Chem. 257:8569, (1982) was used. Poly (A+) RNA was immobilized on nitrocellulose filters and was hybridized to labeled probe-oligonucleotides at 40° in 4xSSC. These experiments show that L6 contains $J_K5$ sequences. A faint hybridization to $J_K2$ was observed.

(3) V Region cDNA Clones.

A library primed by oligo (dT) on L6 poly (A+) RNA was screened for kappa clones with a mouse $C_K$ region probe. From the L6 library, several clones were isolated. A second screen with a 5' $J_K5$ specific probe identified the L6 ($J_K5$) light-chain clones. Heavy chain clones of L6 were isolated by screening with the $J_H2$ oligonucleotide.

The heavy and light chain genes or gene fragments from the cDNA clones, pH3-6a and pL3-12a were inserted into M13 bacteriophage vectors for nucleotide sequence analysis. The complete nucleotide sequences of the variable region of these clones were determined (FIGURES 15 and 16) by the dideoxy chain termination method. These sequences predict V region amino acid compositions that agree well with the observed compositions, and predict peptide sequences which have been verified by direct amino acid sequencing of portions of the V regions.

The nucleotide sequences of the cDNA clones show that they are immunoglobulin V region clones as they contain amino acid residues diagnostic of V domains (Kabat et al., Sequences of Proteins of Immunological Interest; U.S. Dept of HHS, 1983).

The L6 $V_H$ belongs to subgroup II. The cDNA predicts an N-terminal sequence of 24 amino acid residues identical to that of a known $V_H$ (45-165 CRI; Margolies et al. Mol. Immunol. 18:1065, 1981). The L6 $V_H$ has the $J_H2$ sequence. The L6 $V_L$ is from the $V_K$-KpnI family (Nishi et al. Proc. Nat. Acd. Sci. USA 82:6399, 1985), and uses $J_K5$. The cloned L6 $V_L$ predicts an amino acid sequence which was confirmed by amino acid sequencing of peptides from the L6 light chain corresponding to residues 18-40 and 80-96.

(4) In Vitro Mutagenesis to Engineer Restriction Enzyme Sites in the J Region for Joining to a Human C-Module, and to Remove Oligo (dC) Sequences 5' to the V Modules.

Both clones generated from priming with oligo (dT) L6 $V_K$ and L6 $V_H$ need to be modified. For the L6 $V_K$, the J-region mutagenesis primer $J_K$HindIII, as shown in FIGURE 17B, was utilized. A human $C_K$ module derived from a cDNA clone was mutagenized to contain the HindIII sequence (see Figure 17A). The mutagenesis reaction was performed on M13 subclones of these genes. The frequency of mutant clones ranged from 0.5 to 1% of the plaques obtained.

It had been previously observed that the oligo (dC) sequence upstream of the ATG codon in a $V_H$ chimericgene interferes with proper splicing in one particular gene construct. It was estimated that perhaps as much as 70% of the RNA transcripts had undergone the mis-splicing, wherein a cryptic 3' splice acceptor in the leader sequence was used. Therefore the oligo (dC) sequence upstream of the initiator ATG was removed in all of the clones.

In one approach, an oligonucleotide was used which contains a SalI restriction site to mutagenize the L6 $V_K$ clone. The primer used for this oligonucleotide-directed mutagenesis is a 22-mer which introduces a SalI site between the oligo (dC) and the initiator met codon (FIGURE 19).

In a different approach, the nuclease BAL-31 was used to chew away the oligo (dC) in the L6 $V_H$ clone pH3-6a. The size of the deletion in two of the mutants obtained was determined by nucleotide sequencing and is shown in FIGURE 17. In both of these mutuants (delta 4 and delta 21), all of the oligo (dC) 5' to the coding region were deleted.

These clones were then modified by oligonucleotide-directed mutagenesis with the MJH2-ApaI primer (FIGURE 17). This 31-base primer introduces an ApaI site in the mouse $C_H$ gene at a position analogous to an existing ApaI site in human Cgamma1 cDNA gene module. The primer introduces the appropriate codons for the human C gamma 1 gene. The chimeric heavy chain gene made by joining the mutagenized mouse $V_H$ gene module to a human $C_H$ module thus encodes a chimeric protein which contains no human amino acids for the entire $V_H$ region.

The human C gamma 1 gene module is a cDNA derived from GM2146 cells (Human Genetic Mutant Cell Repository, Newark, New Jersey). This C gamma 1 gene module was previously combined with a mouse $V_H$ gene module to form the chimeric expression plasmid pING2012E.

(5) L6 Chimeric Expression Plasmids.

L6 chimeric heavy chain expression plasmids were derived from the replacement of the $V_H$ module pING2012E with the $V_H$ modules of mutants delta 21 and delta 4 to give the expression plasmids pING2111 and pING2112 (FIGURE 17). These plasmids direct the synthesis of chimeric L6 heavy chain when transfected into mammalian cells.

For the L6 light chain chimeric gene, the SalI to HindIII fragment of the mouse $V_K$ module was joined to the human $C_K$ module by the procedure outlined in FIGURE 18, forming pING2119. Replacement of the neo sequence with the E. coli gpt gene derived from pSV2-gpt resulted in pING2120, which expressed L6 chimeric light chain and confers mycophenolic acid resistance when transfected into mammalian cells.

The inclusion of both heavy and light chain chimeric genes in the same plasmid allows for the introduction into transfected cells of a 1:1 gene ratio of heavy and light chain genes leading to a balanced gene dosage. This may improve expression and decrease manipulations of transfected cells for optimal chimeric antibody expression. For this purpose, the DNA fragments derived from the chimeric heavy and light chain genes of pING2111 and pING2119 were combined into the expression plasmid pING2114 (FIGURE 19). This expression plasmid contains a selectable $neo^R$ marker and separate transcription units for each chimeric gene, each including a mouse heavy chain enhancer.

The modifications and V-C joint regions of the L6 chimeric genes are summarized in FIGURE 20.

(6) Stable Transfection of Mouse Lymphoid Cells for the Production of Chimeric Antibody.

Electroporation was used (Potter et al. supra; Toneguzzo et al. Mol. Cell Biol. 6:703 1986) for the introduction of L6 chimeric expression plasmid DNA into mouse Sp2/0 cells. The electroporation technique gave a transfection frequency of 1-10 x $10^{-5}$ for the Sp2/0 cells.

The two gene expression plasmid pING2114 was linearized by digestion with AatII restriction endonuclease and transfected into Sp2/0 cells, giving approximately fifty G418 resistant clones which were screened for human heavy and light chain synthesis. The levels of chimeric antibody chain synthesis from the two producers, D7 and 3E3, are shown in Table 3. Chimeric L6 antibody was prepared by culturing the D7 transfectant cells for 24 hours at $2x10^6$ cells/ml in 5 1 DMEM supplemented with HEPES buffer and penicillin and streptomycin. The supernatant was concentrated over an Amicon YM30® membrane in 10mM sodium phosphate buffer, pH8.0. The preparation was loaded over a DEAE-Cellulose column, which separated the immunoglobulin into unbound and bound fractions. Samples from the DEAE-unbound, DEAE-bound and the pre-DEAE preparations (from 1.6 ul of medium) was separately purified by affinity chromatography on a Protein-A Sepharose® column, eluting with 0.1 M sodium citrate,

pH 3.5. The eluted antibody was neutralized and concentrated by Amicon centricon filtration, in phosphate-buffered saline. The yields for the three preparations were 12ug (DEAE unbound), 6ug (DEAE bound), and 9ug (pre-DEAE column). Western analysis of the antibody chains indicated that they were combined in an $H_2L_2$ tetramer like native immunoglobulins.

(7) A second purification for Chimeric L6 Antibody Secreted in Tissue Culture.

a. Sp2/0.pING2114.D7 cells were grown in culture medium [DMEM (Gibco #320-1965), supplemented with 10% Fetal Bovine Serum (Hyclone #A-1111-D), 10mM HEPES, 1x Glutamine-Pen-Strep (Irvine Scientific #9316) to 1 x $10^6$ cell/ml.

b. The cells were then centrifuged at 400xg and resuspended in serum-free culture medium at 2 x $10^6$ cell/ml for 18-24 hr.

c. The medium was centrifuged at 4000 RPM in a JS-4.2 rotor (3000xg) for 15 min.

d. 1.6 liter of supernatant was then filtered through a 0.45 micron filter and then concentrated over a YM30 (Amicon Corp.) filter to 25ml.

e. The conductance of the concentrated supernatant was adjusted to 5.7-5.6 mS/cm and the pH was adjusted to 8.0.

f. The supernatant was centrifuged at 2000xg, 5 min., and then loaded onto a 40 ml DEAE column, which was preequilibrated with 10mM sodium phosphate, pH8.0.

g. The flow through fraction was collected and loaded onto a 1ml protein A-Sepharose (Sigma) column preequilibrated with 10mM sodium phosphate, pH8.0.

h. The column was washed first with 6ml 10mM sodium phosphate buffer pH = 8.0, followed by 8ml 0.1M sodium citrate pH = 3.5, then by 6ml 0.1M citric acid (pH = 2.2). Fractions of 0.5ml were collected in tubes containing 50ul 2M Tris base (Sigma).

i. The bulk of the IgG was in the pH = 3.5 elution and was pooled and concentrated over Centricon 30 (Amicon Corp.) to approximately .06ml.

j. The buffer was changed to PBS (10mM sodium phosphate pH = 7.4, 0.15M NaCl) in Centricon 30 by repeated diluting with PBS and reconcentrating.

k. The IgG solution was then adjusted to 0.10ml and bovine serum albumin (Fraction V, U.S. Biochemicals) was added to 1.0% as a Stabilizing reagent.

(8) Production and Purification of Chimeric L6 Antibody Secreted in Ascites Fluid.

a. The ascites was first centrifuged a 2,000 xg for 10 min.

b. The conductance of the supernatant was adjusted to 5.7-5.6 mS/cm and its pH adjusted to 8.0.

c. Supernatant was then loaded onto a 40 ml DEAE-cellulose column pre-equilibrated with 10 mM $Na_2PO_4H$ pH 8.0.

d. The flow through from the DEAE column was collected and its pH was adjusted to 7.4, and then loaded onto a 1.0 ml goat anti-human IgG (H + L) - sepharose column.

e. The column was washed first with 6 ml of 10 mM sodium phosphate, 0.5 M sodium chloride, followed by 8 ml of 0.5 M $NH_4OH$, and 3 M sodium thiocyanate.

f. The sodium thiocyanate eluate was pooled and dialyzed against 2L PBS overnight.

The antibody can be further concentrated by steps j. and k. of the previous procedure.

TABLE 3

| Levels of Secreted Chimeric L6 Chains from Sp2/0 Transfectants[a] | | | | | |
|---|---|---|---|---|---|
| Culture Condition | FBS | Sp2/0.D7 | | Sp2/0.3E3 | |
| | | Kappa[b] | Gamma[c] | Kappa[b] | Gamma[c] |
| 1. 20 ml, 2d, seed @ $2x10^5$/ml | + | 17 | 77 | 100 | 700 |
| 2. 200 ml, 2d, seed @ $2.5x10^5$/ml | + | 0.9 | 6 | 80 | 215 |
| 3. 200 ml, 1d, seed @ $2x10^6$/ml | - | 1.9 | 3.8 | 97 | 221 |
| 4. Balb/c ascites | - | 5,160 | 19,170 | ND | ND |

a - Sp2/0 cells transfected by electroporation with pING2114(pL6HL)
b - ug/l measured by ELISA specific for human Kappa - human Bence-Jones protein standard.
c - ug/l measured by ELISA specific for human gamma - human IgG standard.
ND - Not determined.
FBS: Fetal Bovine Serum

(9) Studies Performed on the Chimeric L6 Antibody.

First, the samples were tested with a binding assay, in which cells of both an L6 antigen-positive and an L6 antigen-negative cell line were incubated with standard mouse monoclonal antibody L6, chimeric L6 antibody derived from the cell culture supernatants, and chimeric L6 antibody derived from ascites (as previously described) followed by a second reagent, fluorescein-isothiocyanate (FITC)-conjugated goat antibodies to human (or mouse, for the standard) immunoglobulin.

Since the binding assay showed strong reactivity of the chimeric L6 on the L6 antigen positive cell line and total lack of reactivity on the negative cell line, the next step was to test for the ability of the chimeric L6 to inhibit the binding of mouse L6 to antigen positive cells; such inhibition assays are used routinely to establish the identity of two antibodies' recognition of antigen. These data are discussed below ("Inhibition of binding"). As part of these studies, a rough estimate of antibody avidity was made.

Finally, two aspects of antibody function were studied, the ability to mediate ADCC in the presence of human peripheral blood leukocytes, and the ability to kill L6 positive tumor cells in the presence of human serum as a source of complement (see "Functional Assays" below).

Binding Assays. Cells from a human colon carcinoma line, 3347, which had been previously shown to express approximately 5 x $10^5$ molecules of the L6 antigen at the cell surface, were used as targets. Cells from the T cell line HSB2 was used as a negative control, since they, according to previous testing, do not express detectable amounts of the L6 antigen. The target cells were first incubated for 30 min at 4°C with either the chimeric L6 or with mouse L6 standard, which had been purified from mouse ascites. This was followed by incubation with a second, FITC-labelled, reagent, which for the chimeric antibody was goat-anti-human immunoglobulin, obtained from TAGO (Burlingame, CA), and used at a dilution of 1:50. For the mouse standard, it was goat-anti-mouse immunoglobulin, also obtained from TAGO and used at a dilution of 1:50. Antibody binding to the cell surface was determined using a Coulter Model EPIC-C cell sorter.

As shown in Table 4 and Table 4A, both the chimeric and the mouse standard L6 bound significantly, and to approximately the same extent, to the L6 positive 3347 line. They did not bind above background to the L6 negative HSB2 line.

In view of the fact that the three different chimeric L6 samples presented in Table 4 behaved similarly in the binding assays, they were pooled for the inhibition studies presented below. The same inhibition studies were performed for chimeric L6 derived from ascites fluid presented in Table 4A.

Inhibition of Binding. As the next step was studied the extent to which graded doses of the chimeric L6 antibody, or the standard mouse L6, could inhibit the binding of an FITC-labelled mouse L6 to the surface of antigen positive 3347 colon carcinoma cells.

Both the chimeric and mouse standard L6 inhibited the binding of the directly labelled L6 antibody, with the binding curves being parallel. The chimeric antibody was slightly less effective than the standard, as indicated by the results which showed that 3.4 ug/ml of the pooled chimeric L6 MAb, as compared to 2.0 ug/ml of the standard mouse L6 MAb was needed for 50% inhibition of the binding, and that 5.5 ug/ml of the chimeric L6 (derived from ascites) as compared to 2.7 ug/ml of the standard mouse L6 MAb was needed for 50% inhibition of binding.

As part of these studies, a rough estimate was made of antibody avidity. The avidity of the standard mouse L6 had been previously determined to be approximately 4 x $10^8$. The data indicated that there were no significant differences in avidity between the chimeric and the mouse L6.

Functional Assays. A comparison was made between the ability of the chimeric L6 and standard mouse L6 to lyse L6 antigen positive cells in the presence of human peripheral blood leukocytes as a source of effector cells (mediating Antibody Dependent Cellular Cytotoxcity, ADCC) or human serum as a source of complement (mediating Complement-Dependent Cytolysis, CDC).

As shown in Table 5 and Tables 5A-5D, the chimeric L6 was superior to the simultaneously tested sample of mouse L6 in causing ADCC, as measured by a 4 hr $^{51}Cr$ release test.

Tables 6 and 6A-6B present the data from studies on complement-mediated target cell lysis. In this case, a high cytolytic activity was observed with both the mouse and the chimeric L6 antibodies.

Conclusions.

The results presented above demonstrate a number of important, unexpected qualities of the chimeric L6 monoclonal antibody of the invention. Firstly, the chimeric L6 antibody binds to L6 antigen positive tumor cells to approximately the same extent as the mouse L6 standard and with approximately the same avidity. This is significant for the following reasons: the L6 antibody defines (a) a surface carbohydrate antigen, and (b) a protein antigen of about 20,000 daltons, each of which is characteristic of non-small cell lung carcinoma (NSCLC) and certain other human carcinomas. Significantly, the L6 antibody does not bind detectably to normal cells such as fibroblasts, endothelial cells, or epithelial cells in the major organs. Thus the chimeric L6 monoclonal antibody defines an antigen that is specific for carcinoma cells and not normal cells.

In addition to the ability of the chimeric L6 monoclonal antibodies of the present invention to bind specifically to malignant cells and localize tumors, the chimeric L6 exerts profound biological effects upon binding to its target, which make the chimeric antibody a prime candidate for tumor immunotherapy. The results presented herein demonstrate that chimeric L6 is capable of binding to tumor cells and upon binding kills the tumor cells, either by ADCC or CDC. Such tumor killing activity was demonstrated using concentrations of chimeric L6 antibody as low as 0.01 ug/ml (10ng/ml).

Although the prospect of attempting tumor therapy using monoclonal antibodies is attractive, with some partial tumor regressions being reported, to date such monoclonal antibody therapy has been met with limited success (Houghton, February 1985, Proc. Natl. Acad. Sci. 82:1242-1246). The therapeutic efficacy of mouse monoclonal antibodies (which are the ones that have been tried so far) appears to be too low for most practical purposes. The discovery of the profound biological activity of chimeric L6 coupled with its specificity for a carcinoma antigen makes the chimeric L6 antibody a choice therapeutic agent for the treatment of tumors in vivo. Moreover, because of the "human" properties which will make the chimeric L6 monoclonal antibodies more resistant to clearance in vivo, the chimeric L6 monoclonal antibodies will be advantageously used not only for therapy with unmodified chimeric antibodies, but also for development of various immunoconjugates with drugs, toxins, immunomodulators, isotopes, etc., as well as for diagnostic purposes such as in vivo imaging of tumors using appropriately labelled chimeric L6 antibodies. Such immunoconjugation techniques are known to those skilled in the art and can be used to modify the chimeric L6 antibody molecules of the present invention.

Two illustrative cell lines secreting chimeric L6 antibody were deposited prior to the filing date of this application at the ATCC, Rockville Maryland. These are transfected hybridoma C255 (corresponds to 3E3 cells, supra), ATCC HB 9240 and transfected hybridoma C256 (C7 cells, supra), ATCC HB 9241.

(10) Expression in Yeast of L6 Chains

Genetic sequence codings for Chimeric L6 antibody heavy and light chains were prepared and introduced into vectors. Yeast cells were transformed therewith and expression of separate heavy and light antibody chains for L6 antibody was detected.

TABLE 4

Binding Assays Of Chimeric L6 Antibody and Mouse L6 Monoclonal Antibody on an L6 Antigen Positive and L6 Antigen Negative Cell Line.

| | | Binding Ratio For* H3347 Cells (L6 +) | |
|---|---|---|---|
| Antibody | Batch | GAM | GAH |
| Standard L6 | | 56.6 | 4.2 |
| Chimeric L6 | a | 1.3 | 110.3 |
| | b | 1.3 | 110.3 |
| | c | 1.3 | 110.3 |
| | | Binding Ratio For* HSB-2 Cells (L6 -) | |
| | | GAM | GAH |
| Standard L6 | | 1.1 | 1.1 |
| Chimeric L6 | a | 1.0 | 1.0 |
| | b | 1.0 | 1.1 |
| | c | 1.0 | 1.1 |

* All assays were conducted using an antibody concentration of 10 ug/ml. The binding ratio is the number of times brighter a test sample is than a control sample treated with GAM (FITC conjugated goat-anti-mouse) or GAH (FITC conjugated goat anti-human) alone. A ratio of 1 means that the test sample is just as bright as the control; a ratio of 2 means the test sample is twice as bright as the control, etc.

TABLE 4A

Binding Assays Of Chimeric L6 Antibody and Mouse Monoclonal Antibody on an L6 Antigen Positive and L6 Antigen Negative Cell Line.

| Antibody | Antibody Concentration (ug/ml) | Binding Ratio For* H3347 Cells (L6 +) GAM | GAH |
|---|---|---|---|
| Standard L6 | 30 | 38 | 4 |
| | 10 | 49 | 4 |
| | 3 | 40 | 3 |
| Chimeric L6 (Ascites) | 30 | 2 | 108 |
| | 10 | 2 | 108 |
| | 3 | 1 | 42 |
| Chimeric L6 (Cell Culture) | 30 | 1 | 105 |
| | 10 | 1 | 86 |
| | 3 | 1 | 44 |
| | | Binding Ratio For** HSB-2 Cells (L6 -) GAM | GAH |
| Standard L6 | 10 | 1 | 1 |
| Chimeric L6 (Ascites) | 10 | 1 | 1 |
| Chimeric L6 (Cell Culture) | 10 | 1 | 1 |

* The binding ratio is the number of times brighter a test sample is than a control sample treated with GAM (FITC conjugated goat anti-human) alone. A ratio of 1 means that the test sample is just as bright as the control; a ratio of 2 means the test sample is twice as bright as the control, etc.

TABLE 5

| ADCC of Chimeric L6 (Mouse) L6 Antibodies On Colon Carcinoma Cell Line 3347. | | | |
|---|---|---|---|
| Antibody | Antibody Concentration (µg/ml) | PBL per Target Cell | % Cytolysis* |
| Chimeric L6 | 10 | 100 | 64 |
| | 5 | 100 | 70 |
| | 10 | 0 | 2 |
| Standard L6 | 10 | 100 | 24 |
| | 5 | 100 | 17 |
| | 10 | 0 | 2 |
| None | 0 | 100 | 1 |

* The target cells had been labelled with $^{51}Cr$ and were exposed for 4 hours to a combination of MAb and human peripheral blood leukocytes (PBL), and the release of $^{51}Cr$ was measured subsequently. The release of $^{51}Cr$ (after corrections of values for spontaneous release from untreated cells) is a measure of the percent cytolsis.

TABLE 5A

| ADCC of Chimeric L6 and Standard (Mouse) L6 Antibodies On Colon Carcinoma Cell Line 3347. | | | |
|---|---|---|---|
| Antibody | Antibody Concentration (µg/ml) | PBL per Target Cell | % Cytolysis* |
| Chimeric L6 (Ascites) | 20 | 100 | 80 |
| | 10 | 100 | 74 |
| | 5 | 100 | 71 |
| | 2.5 | 100 | 71 |
| | 20 | 0 | 0 |
| Chimeric L6 (Cell Culture) | 10 | 100 | 84 |
| | 5 | 100 | 74 |
| | 2.5 | 100 | 67 |
| | 10 | 0 | 3 |
| Standard L6 | 20 | 100 | 32 |
| | 10 | 100 | 26 |
| | 20 | 0 | 0 |

* The target cells had been labelled $^{51}Cr$ and were exposed for 4 hours to a combination of MAb and human peripheral blood leukocytes(PBL), and the release of $^{51}Cr$ was measured subsequently. The release of $^{51}Cr$ (after corrections of values for spontaneous release from untreated cells) is a measure of the percent cytolsis.

TABLE 5B

ADCC of Chimeric L6 and Standard (Mouse) L6 Antibodies On Colon Carcinoma Cell Line 3347.

| Antibody | Antibody Concentration (ug/ml) | PBL per Target Cell | % Cytolysis* |
|---|---|---|---|
| Chimeric L6 (Ascites) | 5 | 100 | 84 |
| | 2.5 | 100 | 78 |
| | 1.25 | 100 | 85 |
| | 0.63 | 100 | 81 |
| | 0.31 | 100 | 80 |
| | 0.16 | 100 | 71 |
| | 0.08 | 100 | 65 |
| | 5 | 0 | 0 |
| Standard L6 | 5 | 100 | 32 |
| | 5 | 0 | 0 |
| None | 0 | 100 | 19 |

* The target cells had been labelled with $^{51}Cr$ and were exposed for 4 hours to a combination of MAb and human peripheral blood leukocytes (PBL), and the release of $^{51}Cr$ was measured subsequently. The release of $^{51}Cr$ (after corrections of values for spontaneous release from untreated cells) is a measure of the percent cytolsis.

TABLE 5C

ADCC of Chimeric L6 and Standard (Mouse) L6 Antibodies On Lung Carcinoma Cell Line H2669.

| Antibody | Antibody Concentration (ug/ml) | PBL per Target Cell | % Cytolysis* |
|---|---|---|---|
| Chimeric L6 | 10 | 100 | 35 |
| (Ascites) | 1 | 100 | 31 |
| | 0.1 | 100 | 27 |
| | 0.01 | 100 | 15 |
| | 0.001 | 100 | 13 |
| | 0.0001 | 0 | 15 |
| Standard L6 | 10 | 100 | 9 |
| | 1 | 100 | 15 |
| None | 0 | 100 | 9 |
| Chimeric L6 | 10 | 10 | 19 |
| (Ascites) | 1 | 10 | 15 |
| | 0.1 | 10 | 11 |
| | 0.01 | 10 | 13 |
| | 0.001 | 10 | 22 |
| | 0.0001 | 10 | 11 |
| Standard L6 | 10 | 10 | 7 |
| | 1 | 10 | 6 |
| None | 0 | 10 | 8 |

TABLE 5C (cont'd)

| Antibody | Antibody Concentration (ug/ml) | PBL per Target Cell | % Cytolysis* |
|---|---|---|---|
| Chimeric L6 (Ascites) | 10 | 0 | 4 |
| Standard L6 | 10 | 0 | 9 |

* The target cells had been labelled with $^{51}Cr$ and were exposed for 4 hours to a combination of MAb and Human peripheral blood leukocytes (PBL), and the release of $^{51}Cr$ was measured subsequently. The release of $^{51}Cr$ (after corrections of values for spontaneous release from untreated cells) is a measure of the percent cytolysis.

TABLE 5D

| ADCC of Chimeric L6 and Standard (Mouse) L6 Antibodies On Colon Carcinoma Cell Line H3347. | | | |
|---|---|---|---|
| Antibody | Antibody Concentration (ug/ml) | PBL per Target Cell | % Cytolysis* |
| Chimeric L6 (Ascites) | 10 | 100 | 62 |
| | | | 66 |
| | 1 | 100 | 66 |
| | 0.1 | 100 | 69 |
| | 0.01 | 100 | 26 |
| | 0.001 | 100 | 8 |
| | 0.0001 | 0 | 3 |
| | 10 | 0 | 0 |
| Standard L6 | 10 | 100 | 19 |
| | 1 | 100 | 24 |
| | | 0 | 0 |
| None | 0 | 100 | 8 |

* The target cells had been labelled with $^{51}Cr$ and were exposed for 4 hours to a combination of MAb and Human peripheral blood leukocytes (PBL), and the release of $^{51}Cr$ (after corrections of values for spontaneous release from untreated cells) is a measure of the percent cytolysis.

TABLE 6

Complement-dependent cytotoxic effect of chimeric and standard (mouse) L6 on colon carcinoma cells from line 3347, as measured by a 4-hr $^{51}Cr$-release assay. Human serum from a healthy subject was used as the source of complement.

| Antibody | Human complement | % Cytolysis |
|---|---|---|
| L6 Standard 10 ug/ml | Yes | 90 |
| L6 chimeric 10 ug/ml | Yes | 89 |
| L6 Standard 10 ug/ml | No | 0 |
| L6 chimeric 10 ug/ml | No | 1 |

TABLE 6A

| Complement Dependent Cytotoxic Effect of Chimeric L6 and Standard (Mouse) L6 Antibodies on Colon Carcinoma Cell Line 3347 | | | |
|---|---|---|---|
| Antibody | Antibody Concentration (µg/ml) | PBL per Target Cell | % Cytolysis* |
| Chimeric L6 (Ascites) | 20 | + | 29 |
| | 10 | + | 23 |
| | 5 | + | 18 |
| | 2.5 | + | 8 |
| | 20 | Inactivated | 0 |
| | 10 | 0 | 0 |
| Chimeric L6 (Cell Culture)) | 20 | + | 29 |
| | 5 | + | 26 |
| | 2.5 | + | 18 |
| | 20 | + | 4 |
| | 10 | 0 | 4 |
| Standard L6 | 20 | + | 55 |
| | 10 | + | 37 |
| | 20 | Inactivated | 0 |
| | 20 | 0 | 1 |
| None | 0 | + | 0 |

* Complement mediated cytolysis was measured by a 4 hour $^{51}Cr$-release assay. Human serum from a healthy subject was used as the source of complement.

TABLE 6B

| Complement Dependent Cytotoxic Effect of Chimeric L6 and Standard (Mouse) L6 Antibodies on Colon Carcinoma Cell Line 3347 | | | |
|---|---|---|---|
| Antibody | Antibody Concentration (ug/ml) | PBL per Target Cell | % Cytolysis* |
| Chimeric L6 (Ascites) | 10 | + | 209 |
| | 5 | + | 155 |
| | 2.5 | + | 166 |
| | 1.25 | + | 114 |
| | 0.6 | + | 63 |
| | 0.3 | + | 17 |
| | 10 | 0 | 0 |
| Standard L6 | 10 | + | 96 |
| | 5 | + | 83 |
| | 2.5 | + | 48 |
| | 1.25 | + | 18 |
| | 0.6 | + | 7 |
| | 0.3 | + | 4 |
| | 10 | 0 | 2 |
| None | 0 | + | 0 |

* Complement mediated cytolysis was measured by a 4 hour $^{51}$Cr-release assay. Human serum from a healthy subject was used as the source of complement.

EXAMPLE IV : A Human-Mouse Chimeric Antibody with Specificity for Human B-Cell Antigen

The 2H7 mouse monoclonal antibody (gamma $2b^K$) recognizes a human B-cell surface antigen, Bp35 (Clark, E A. et al., Proc. Nat. Acad. Sci. USA, 82:1766 (1985)). The Bp35 molecule plays a role in B-cell activation. mRNA was prepared from the 2H7 cell line. Two cDNA libraries were generated - one using the heavy chain UIG-H primer and the other, oligo(dT). One $V_H$ clone, pH2-11, was isolated upon screening with the same UIG-H oligonucleotide. To isolate the light-chain clone, a mouse kappa-specific DNA fragment was used to screen the oligo(dT) library. Candidate clones were further screened with a mouse $J_K5$ sequences. One $V_K$ clone, pL2-12, was thus isolated. The light chain UIG-K was then used to engineer a restriction enzyme site in the J region.

The two cDNA clones were also modified at the 5' end to remove the artificial oligo d[C] sequence. In pH2-11 this was carried out by using the restriction enzyme NcoI which cuts one nucleotide residue 5' of the ATG initiator codon. In pL2-12 this was achieved by an oligonucleotide in vitro mutagenesis using a 22-mer containing a SalI site.

The DNA sequences of these two clones are shown in Figures 21, 22. To construct the chimeric heavy chain plasmid the $V_H$ module was joined to the human C gamma 1 module (pGMH6) at the $J_H$ BstEII site, and to construct the chimeric light chain the $V_K$ module was joined to the human $C_K$ module (pGML60) at the $J_K$ HindIII site. The expression vector sequences were derived from pING2012-neo as well as pING2016-gpt. The constructed plasmids are pING2101 ($V_H$C gamma 1-neo). pING2106 ($V_KC_K$-neo), pING2107 ($V_KC_K$-gpt). pING2101 and pING2106 were also used to generate plasmids containing both genes. They are pHL2-11 and pHL2-26. In addition, pING2106 and pING2014 were combined to a two light-chain plasmid, pLL2-25, to compensate for the poorer (compared to heavy chain) steady-state accumulation of light chain protein in transfected cells. (See Fig. 23) Fig. 24 shows the changes made to the variable region sequences during the construction.

The plasmid, pHL2-11, was linearized by AatII; and the DNA was used to transfect Sp2/0 cells by electroporation. Transformants were selected in G418-DMEM. One transformant, 1C9, produces 9.3 ng/ml chimeric kappa and 33-72 ng/ml chimeric gamma 1 protein as assayed by ELISA. Southern analysis of 1C9

DNA showed that there is one copy of the plasmid integrated in Sp2/0 genome.

**Claims**

**Claims for the following contracting states : BE, CH and LI, DE, FR, GB, IT, LU, NL, SE**

1. A vector comprising a cDNA sequence coding for the complete variable region of an immunoglobulin chain, said chain including a complete V-J junction in the case of a light chain, and a complete V-D-J junction in the case of a heavy chain; and said vector lacking any constant region, or any intron sequences.

2. A DNA fragment comprising a cDNA sequence coding for the complete constant IgG1 region for a heavy chain human immunoglobulin chain, said fragment lacking any variable region, and said constant region lacking any intron sequences.

3. A vector comprising a continuous coding sequence, uninterrupted by introns, comprising:
   i) a DNA sequence coding for the variable region of a non-human immunoglobulin chain including a V-J junction in the case of a light chain, and a V-D-J junction in the case of a heavy chain;
   ii) a DNA sequence coding for the constant region of a human immunoglobulin chain.

4. The vector of claims 1 or 3 which is a plasmid.

5. A bacterium transformed with the vector or fragment of claims 1, 2, 3 or 4.

6. A mammalian cell or yeast transfected with the vector or fragment of claims 1, 2, 3, or 4.

7. A polynucleotide molecule comprising a consensus sequence for the J region of a heavy chain immunoglobulin molecule, wherein said consensus sequence exhibits at least 80% sequence homology to known J region sequences without being identical.

8. The molecule of claim 7 wherein said sequence is for a human heavy chain J region.

9. The molecule of claim 7 wherein said sequence is for a mouse heavy chain J region.

10. A polynucleotide molecule comprising a consensus sequence for the J region of a light chain immunoglobulin molecule, wherein said consensus sequence exhibits at least 80% sequence homology to known J region sequences without being identical.

11. The molecule of claim 10 wherein said sequence is for a human Kappa J region.

12. The molecule of claim 10 wherein said sequence is for a mouse Kappa J region.

13. The molecule of claim 10 wherein said sequence is for a mouse Lambda J region.

14. A method of directly preparing a genetic sequence coding for a chimeric immunoglobulin chain having a constant human region and a variable non-human region of any desired specificity, which comprises:
   a) providing a cDNA sequence coding for a complete non-human variable region including a complete V-J junction in the case of a light chain and a complete V-D-J junction in the case of a heavy chain, and lacking any constant region sequences;
   b) providing a vector containing a genetic sequence coding for said constant region;
   c) operably linking said sequence a) to said vector b).

15. The method of claim 14 wherein step (c) comprises operably linking said cDNA sequence to said sequence of step (c) in a plasmid.

16. The method of claim 15 which further comprises transforming said plasmid into a host capable of expressing said plasmid.

17. The method of any of claims 14 - 16 wherein said chain is a heavy chain.

**18.** The method of any of claims 14 - 16 wherein said chain is a light chain.

**19.** The method claim 14 wherein said step a) comprises:
a') providing mRNA coding for said variable region from a cell secreting monoclonal antibodies of said desired specificity; and
a'') priming the formation, by reverse transcription using said mRNA as a template, of cDNA derived therefrom, with a polynucleotide molecule comprising a consensus genetic sequence for the J region of said immunoglobulin chain, wherein said consensus sequence exhibits at least 80% sequence homology to known J region sequences.

**20.** The method of claim 19 wherein said consensus genetic sequence is selected from the group consisting of:
(i) human heavy chain J region;
(ii) mouse heavy chain J region;
(iii) human Kappa J region;
(iv) mouse Kappa J region; and
(v) mouse Lambda J region

**21.** The method of claim 19 wherein said consensus genetic sequence is selected from the group consisting of those denoted as MJH1, MJH2, MJH3, MJH3-BSTEII, MJH-BSTEII(13), MJH4, 5JK1, 5JK2, JK2BGLII, 5JK4, JK4BGLII, 5JK5, and MJK:

MJH1- GCCAGTGGCAGAGGAGTCGGT

MJH2- GAGAGTGTCAGACGAGTCGGT

| | |
|---|---|
| MJH3- | ACCAGTGACAGAGACGTCGGT |
| MJH3-BSTEII- | TCCCTGAGACCAGTGGCAGAG |
| MJH-BSTEII(13)- | ACCAGTGGCAGAG |
| MJH4- | GTCAGTGGCAGAGGAGTCGGT |
| 5JK1- | GCAAGCCACCTCCGTGG |
| JK2BGLII- | CCCTGGTTCGACCTCTAGATT |
| 5JK2- | GTGCAAGCCTCCCCCCTGG |
| 5JK4- | GCAAGCCGAGCCCCTGT |
| JK4BGLII | GCCCCTGTTTCAACCTCTAGATT |
| 5JK5 | GCAAGCCACGACCCTGG |
| MJK | TGGTTCGACCTTTATTTTG |

**22.** The method of claim 19 wherein said consensus sequence further comprises the sequence coding for the recognition site of a restriction endonuclease enzyme.

**23.** cDNA expression vectors having restriction endonuclease site maps as shown in Fig. 10 containing an SV40 early region promoter, an SV40 late region splice sequence, the selectable marker neo, SV40 polyA signal sequences, a multiple cloning site (pING2003) and optionally in addition a mouse heavy chain enhancer element (pING2003 E).

**24.** The method of claim 19 wherein said consensus genetic sequence is selected from the group consisting of those denoted as UIGH, UIGK and $MJ_H2$-ApaI:

| | |
|---|---|
| UIG-H | AGGGACCACGGTCACCGTCTC |
| UIG-K | GGGACCAAGCTTGAG |
| $MJ_H2$-ApaI | TGTCAGAGGAGTCGGTCGTGTTTCCCGGGTA |

**Claims for the following Contracting State : AT**

**1.** A vector comprising a cDNA sequence coding for the complete variable region of an immunoglobulin chain, said chain including a complete V-J junction in the case of a light chain, and a complete V-D-J junction in the case of a heavy chain; and said vector lacking any constant region, or any intron sequences.

**2.** A DNA fragment comprising a cDNA sequence coding for the complete constant IgG1 region for a heavy chain human immunoglobulin chain, said fragment lacking any variable region, and said constant region lacking any intron sequences.

**3.** A vector comprising a continuous coding sequence, uninterrupted by introns, comprising:
i) a DNA sequence coding for the variable region of a non-human immunoglobulin chain including a V-J junction in the case of a light chain, and a V-D-J junction in the case of a heavy chain;
ii) a DNA sequence coding for the constant region of a human immunoglobulin chain.

**4.** The vector of claims 1 or 3 which is a plasmid.

**5.** A bacterium transformed with the vector or fragment of claims 1, 2, 3 or 4.

**6.** A mammalian cell or yeast transfected with the vector or fragment of claims 1, 2, 3, or 4.

**7.** A polynucleotide molecule comprising a consensus sequence for the J region of a heavy chain immunoglobulin molecule, wherein said consensus sequence exhibits at least 80% sequence homology to known J region sequences without being identical.

**8.** The molecule of claim 7 wherein said sequence is for a human heavy chain J region.

**9.** The molecule of claim 7 wherein said sequence is for a mouse heavy chain J region.

**10.** A polynucleotide molecule comprising a consensus sequence for the J region of a light chain immunoglobulin molecule, wherein said consensus sequence exhibits at least 80% sequence homology to known J region sequences without being identical.

**11.** The molecule of claim 10 wherein said sequence is for a human Kappa J region.

**12.** The molecule of claim 10 wherein said sequence is for a mouse Kappa J region.

**13.** The molecule of claim 10 wherein said sequence is for a mouse Lambda J region.

**14.** A method of directly preparing a genetic sequence coding for a chimeric immunoglobulin chain having a constant human region and a variable non-human region of any desired specificity, which comprises:
a) providing a cDNA sequence coding for a complete non-human variable region including a complete V-J junction in the case of a light chain and a complete V-D-J junction in the case of a heavy chain, and lacking any constant region sequences;
b) providing a vector containing a genetic sequence coding for said constant region;
c) operably linking said sequence a) to said vector b).

**15.** The method of claim 14 wherein step (c) comprises operably linking said cDNA sequence to said sequence of step (c) in a plasmid.

**16.** The method of claim 15 which further comprises transforming said plasmid into a host capable of expressing said plasmid.

**17.** The method of any of claims 14 - 16 wherein said chain is a heavy chain.

**18.** The method of any of claims 14 - 16 wherein said chain is a light chain.

**19.** The method claim 14 wherein said step a) comprises:
a') providing mRNA coding for said variable region from a cell secreting monoclonal antibodies of said desired specificity; and
a'') priming the formation, by reverse transcription using said mRNA as a template, of cDNA derived therefrom, with a polynucleotide molecule comprising a consensus genetic sequence for the J region of said immunoglobulin chain, wherein said consensus sequence exhibits at least 80% sequence homology to known J region sequences.

**20.** The method of claim 19 wherein said consensus genetic sequence is selected from the group consisting of:
(i) human heavy chain J region;
(ii) mouse heavy chain J region;
(iii) human Kappa J region;
(iv) mouse Kappa J region; and
(v) mouse Lambda J region

**21.** The method of claim 19 wherein said consensus genetic sequence is selected from the group consisting of those denoted as MJH1, MJH2, MJH3, MJH3-BSTEII, MJH-BSTEII(13), MJH4, 5JK1, 5JK2, JK2BGLII, 5JK4, JK4BGLII, 5JK5, and MJK:

MJH1- GCCAGTGGCAGAGGAGTCGGT

MJH2- GAGAGTGTCAGACGAGTCGGT

| | |
|---|---|
| MJH3- | ACCAGTGACAGAGACGTCGGT |
| MJH3-BSTEII- | TCCCTGAGACCAGTGGCAGAG |
| MJH-BSTEII(13)- | ACCAGTGGCAGAG |
| MJH4- | GTCAGTGGCAGAGGAGTCGGT |
| 5JK1- | GCAAGCCACCTCCGTGG |
| JK2BGLII- | CCCTGGTTCGACCTCTAGATT |
| 5JK2- | GTGCAAGCCTCCCCCCTGG |
| 5JK4- | GCAAGCCGAGCCCCTGT |
| JK4BGLII | GCCCCTGTTTCAACCTCTAGATT |
| 5JK5 | GCAAGCCACGACCCTGG |
| MJK | TGGTTCGACCTTTATTTTG |

22. The method of claim 19 wherein said consensus sequence further comprises the sequence coding for the recognition site of a restriction endonuclease enzyme.

23. cDNA expression vectors having restriction endonuclease site maps as shown in Fig. 10 containing an SV40 early region promoter, an SV40 late region splice sequence, the selectable marker neo, SV40 polyA signal sequences, a multiple cloning site (pING2003) and optionally in addtion a mouse heavy chain enhancer element (pING2003 E).

24. The method of claim 19 wherein said consensus genetic sequence is selected from the group consisting of those denoted as UIGH, UIGK and $MJ_H2$-APaI:

| | |
|---|---|
| UIG-H | AGGGACCACGGTCACCGTCTC |
| UIG-K | GGGACCAAGCTTGAG |
| $MJ_H2$-ApaI | TGTCAGAGGAGTCGGTCGTGTTTCCCGGGTA |

25. A method for producing a vector comprising a cDNA sequence coding for the complete variable region of a immunoglobulin chain, said chain including a complete V-J junction in the case of a light chain and a complete V-D-J junction in the case of a heavy chain by introducing said cDNA sequence into a vector lacking any constant region or any intron sequences.

26. A method for producing a DNA fragment coding for the complete constant IgG1 region for a heavy chain human immunoglobulin chain by obtaining a cDNA sequence coding for the complete constant IgG1 region for a heavy chain immunoglobulin chain, said DNA sequence lacking any variable region and said constant region lacking any intron sequences.

27. A method for producing a vector comprising a continuous coding sequence, uninterrupted by introns, comprising combining a DNA sequence coding for the variable region of a non-human immunoglobulin chain including a V-J junction in the case of a light chain, and a V-D-J junction in the case of a heavy chain and a DNA sequence coding for the constant region of a human immunoglobulin chain.

28. A method for producing a vector coding to claim 25 or 27, wherein the vector is a plasmid.

29. A method for producing a bacterium containing the vector or fragment according to any of claims 25 to 28 by transforming the bacterium with the said vector or fragment.

30. A method for producing a mammalian cell or a yeast containing the vector or fragment according to any of claims 25 to 28 by transfecting the mammalian cell or yeast with the said vector or fragment.

31. A method for synthesizing a polynucleotide molecule comprising a consensus sequence for the J region of a heavy chain immunoglobulin molecule, wherein said consensus sequence exhibits at least 80% sequence homology to known J region sequences without being identical, by synthesizing said polynucleotide molecule in a manner known per se.

32. The method according to claim 31, wherein said sequence is for a human heavy chain J region.

33. The method according to claim 31, wherein said sequence is for a mouse heavy chain J region.

34. A method for producing a polynucleotide molecule comprising a consensus sequence for the J region of a light chain immunoglobulin molecule, wherein said consensus sequence exhibits at least 80% sequence homology to known J region sequences without being identical by synthesizing said polynucleotide molecule in a manner known per se.

35. The method according to claim 34, wherein said sequence is for a human Kappa J region.

36. The method according to claim 34, wherein said sequence is for a mouse Kappa J region.

37. The method according to claim 34, wherein said sequence is for a mouse Lambda J region.

38. A method for producing a cDNA expression vector having restriction endonuclease site maps as shown in Fig. 10 by combining an SV40 early region promoter, an SV40 late region splice sequence, the selectable marker neo, SV40 polyA signal sequences, a multiple cloning site (pING2003) and optionally in addition a mouse heavy chain enhancer element (pING2003 E).

**Patentansprüche**

**Patentansprüche für folgende Vertragsstaaten : BE, CH and LI, DE, FR, GB, IT, LU, NL, SE**

1. Vektor, umfassend eine cDNA-Sequenz, die für die vollständige variable Region einer Immunglobulinkette kodiert, wobei diese Kette eine vollständige V-J-Verbindung im Fall einer leichten Kette und eine vollständige V-D-J-Verbindung im Fall einer schweren Kette enthält, und wobei dem Vektor eine konstante Region oder Intronsequenzen fehlen.

2. DNA-Fragment, umfassend eine cDNA-Sequenz, die für die vollständige konstante IgG1-Region für eine schwere Human-Immunglobulinkette kodiert, wobei dem Fragment eine variable Region fehlt und der konstanten Region Intronsequenzen fehlen.

**3.** Vektor, umfassend eine kontinuierliche kodierende Sequenz, die nicht von Introns unterbrochen wird, umfassend:
i) eine für die variable Region einer nicht-Human-Immunglobulinkette kodierende DNA-Sequenz einschließlich einer V-J-Verbindung im Fall einer leichten Kette und einer V-D-J-Verbindung im Fall einer schweren Kette;
ii) eine für die konstante Region einer Human-Immunglobulinkette kodierende DNA-Sequenz.

**4.** Vektor nach Anspruch 1 oder 3, der ein Plasmid ist.

**5.** Bakterium, transformiert mit dem Vektor oder dem Fragment nach einem der Ansprüche 1, 2, 3 oder 4.

**6.** Säugetierzelle oder Hefe, transfiziert mit dem Vektor oder Fragment nach einem der Ansprüche 1, 2, 3 oder 4.

**7.** Polynukleotidmolekül, umfassend eine Konsensussequenz für die J-Region eines Immunglobulinmoleküls für eine schwere Kette, wobei die Konsensussequenz mindestens 80% Sequenzhomologie mit bekannten J-Region-Sequenzen aufweist, ohne damit identisch zu sein.

**8.** Molekül nach Anspruch 7, wobei die Sequenz für die J-Region einer schweren Human-Kette ist.

**9.** Molekül nach Anspruch 7, wobei die Sequenz für die J-Region einer schweren Maus-Kette ist.

**10.** Polynukleotidmolekül, umfassend eine Konsensussequenz für die J-Region eines Immunglobulinmoleküls für eine leichte Kette, wobei die Konsensussequenz mindestens 80% Sequenzhomologie mit bekannten J-Region-Sequenzen aufweist, ohne damit identisch zu sein.

**11.** Molekül nach Anspruch 10, wobei die Sequenz für eine Human-Kappa-J-Region ist.

**12.** Molekül nach Anspruch 10, wobei die Sequenz für eine Maus-Kappa-J-Region ist.

**13.** Molekül nach Anspruch 10, wobei die Sequenz für eine Maus-Lambda-J-Region ist.

**14.** Verfahren zum direkten Herstellen einer genetischen Sequenz, die für eine chimäre Immunglobulinkette mit einer konstanten Human-Region und einer variablen nicht-Human-Region jeder gewünschten Spezifität kodiert, umfassend:
a) das Bereitstellen einer für eine vollständige variable nicht-Human-Region kodierenden cDNA-Sequenz einschließlich einer vollständigen V-J-Verbindung im Fall einer leichten Kette und einer vollständigen V-D-J-Verbindung im Fall einer schweren Kette, wobei Sequenzen eines konstanten Bereiches fehlen;
b) das Bereitstellen eines Vektors, der eine genetische Sequenz enthält, die für die konstante Region kodiert;
c) das funktionelle Verbinden der Sequenz a) mit dem Vektor b).

**15.** Verfahren nach Anspruch 14, wobei Schritt (c) das funktionelle Verbinden der cDNA-Sequenz mit der Sequenz aus Schritt (c) in einem Plasmid umfaßt.

**16.** Verfahren nach Anspruch 15, das weiter das Transformieren des Plasmides in einen Wirt umfaßt, der zur Expression des Plasmides fähig ist.

**17.** Verfahren nach einem der Ansprüche 14 bis 16, wobei die Kette eine schwere Kette ist.

**18.** Verfahren nach einem der Ansprüche 14 bis 16, wobei die Kette eine leichte Kette ist.

**19.** Verfahren nach Anspruch 14, wobei der Schritt (a) umfaßt:
a') das Bereitstellen einer mRNA, die für die variable Region kodiert, aus einer Zelle, die monoklonale Antikörper der gewünschten Spezifität ausscheidet; und

a") das Starten (Priming) der Bildung einer davon abgeleiteten cDNA durch reverse Transkription unter Verwendung der mRNA als Matrize mit einem Polynukleotidmolekül, das eine genetische Konsensussequenz für die J-Region der Immunglobulinkette umfaßt, wobei die Konsensussequenz mindestens 80% Sequenzhomologie mit bekannten J-Region-Sequenzen aufweist.

**20.** Verfahren nach Anspruch 19, wobei die genetische Konsensussequenz aus der Gruppe ausgewählt ist, die besteht aus:
(i) Human-J-Region für schwere Ketten;
(ii) Maus-J-Region für schwere Ketten;
(iii) Human-Kappa-J-Region;
(iv) Maus-Kappa-J-Region; und
(v) Maus-Lambda-J-Region.

**21.** Verfahren nach Anspruch 19, wobei die genetische Konsensussequenz aus der Gruppe ausgewählt ist, die aus den mit MJH1, MJH2, MJH3, MJH3-BSTEII, MJH-BSTEII(13), MJH4, 5JK1, 5JK2, JK2BGLII, 5JK4, JK4BGLII, 5JK5 und MJK bezeichneten besteht:

| | |
|---|---|
| MJH1- | GCCAGTGGCAGAGGAGTCGGT |
| MJH2- | GAGAGTGTCAGACGAGTCGGT |
| MJH3- | ACCAGTGACAGAGACGTCGGT |
| MJH3-BSTEII- | TCCCTGAGACCAGTGGCAGAG |
| MJH-BSTEII(13)- | ACCAGTGGCAGAG |
| MJH4- | GTCAGTGGCAGAGGAGTCGGT |
| 5JK1- | GCAAGCCACCTCCGTGG |
| JK2BGLII- | CCCTGGTTCGACCTCTAGATT |
| 5JK2- | GTGCAAGCCTCCCCCCTGG |
| 5JK4- | GCAAGCCGAGCCCCTGT |
| JK4BGLII | GCCCCTGTTTCAACCTCTAGATT |
| 5JK5 | GCAAGCCACGACCCTGG |
| MJK | TGGTTCGACCTTTATTTTG |

22. Verfahren nach Anspruch 19, wobei die Konsensussequenz weiter die für die Erkennungsstelle eines Restriktionsendonukleaseenzymes kodierende Sequenz umfaßt.

23. cDNA-Expressionsvektoren mit Restriktionsendonuklease-Schnittstellenkarten, wie in Figur 10 gezeigt, die einen Promotor aus der frühen Region von SV40, eine Spleißsequenz aus der späten Region von SV40, den selektierbaren Marker neo, SV40 polyA-Signalsequenzen, eine Vielfachklonierungstelle (pING2003) und gegebenenfalls zusätzlich ein Enhancer-Element aus einer schweren Mauskette (pING2003 E) enthalten.

24. Verfahren nach Anspruch 19, wobei die genetische Konsensussequenz aus der Gruppe ausgewählt ist, die aus den als UIGH, UIGK und $MJ_H$2-ApaI bezeichneten besteht:

| | |
|---|---|
| UIG-H | AGGGACCACGGTCACCGTCTC |
| UIG-K | GGGACCAAGCTTGAG |
| $MJ_H$2-ApaI | TGTCAGAGGAGTCGGTCGTGTTTCCCGGGTA |

**Patentansprüche für folgende Vertragsstaaten : AT**

1. Vektor, umfassend eine cDNA-Sequenz, die für die vollständige variable Region einer Immunglobulinkette kodiert, wobei diese Kette eine vollständige V-J-Verbindung im Fall einer leichten Kette und eine vollständige V-D-J-Verbindung im Fall einer schweren Kette enthält, und wobei dem Vektor eine konstante Region oder Intronsequenzen fehlen.

2. DNA-Fragment, umfassend eine cDNA-Sequenz, die für die vollständige konstante IgG1-Region für eine schwere Human-Immunglobulinkette kodiert, wobei dem Fragment eine variable Region fehlt und der konstanten Region Intronsequenzen fehlen.

3. Vektor, umfassend eine kontinuierliche kodierende Sequenz, die nicht von Introns unterbrochen wird, umfassend: i) eine für die variable Region einer nicht-Human-Immunglobulinkette kodierende DNA-Sequenz einschließlich einer V-J-Verbindung im Fall einer leichten Kette und einer V-D-J-Verbindung im Fall einer schweren Kette; ii) eine für die konstante Region einer Human-Immunglobulinkette kodierende DNA-Sequenz.

4. Vektor nach Anspruch 1 oder 3, der ein Plasmid ist.

5. Bakterium, transformiert mit dem Vektor oder dem Fragment nach einem der Ansprüche 1, 2, 3 oder 4.

6. Säugetierzelle oder Hefe, transfiziert mit dem Vektor oder Fragment nach einem der Ansprüche 1, 2, 3 oder 4.

7. Polynukleotidmolekül, umfassend eine Konsensussequenz für die J-Region eines Immunglobulinmoleküls für eine schwere Kette, wobei die Konsensussequenz mindestens 80% Sequenzhomologie mit bekannten J-Region-Sequenzen aufweist, ohne damit identisch zu sein.

8. Molekül nach Anspruch 7, wobei die Sequenz für die J-Region einer schweren Human-Kette ist.

9. Molekül nach Anspruch 7, wobei die Sequenz für die J-Region einer schweren Maus-Kette ist.

10. Polynukleotidmolekül, umfassend eine Konsensussequenz für die J-Region eines Immunglobulinmoleküls für eine leichte Kette, wobei die Konsensussequenz mindestens 80% Sequenzhomologie mit bekannten J-Region-Sequenzen aufweist, ohne damit identisch zu sein.

**11.** Molekül nach Anspruch 10, wobei die Sequenz für eine Human-Kappa-J-Region ist.

**12.** Molekül nach Anspruch 10, wobei die Sequenz für eine Maus-Kappa-J-Region ist.

**13.** Molekül nach Anspruch 10, wobei die Sequenz für eine Maus-Lambda-J-Region ist.

**14.** Verfahren zum direkten Herstellen einer genetischen Sequenz, die für eine chimäre Immunglobulinkette mit einer konstanten Human-Region und einer variablen nicht-Human-Region jeder gewünschten Spezifität kodiert, umfassend: a) das Bereitstellen einer für eine vollständige variable nicht-Human-Region kodierenden cDNA-Sequenz einschließlich einer vollständigen V-J-Verbindung im Fall einer leichten Kette und einer vollständigen V-D-J- Verbindung im Fall einer schweren Kette, wobei Sequenzen eines konstanten Bereiches fehlen; b) das Bereitstellen eines Vektors, der eine genetische Sequenz enthält, die für die konstante Region kodiert; c) das funktionelle Verbinden der Sequenz a) mit dem Vektor b).

**15.** Verfahren nach Anspruch 14, wobei Schritt (c) das funktionelle Verbinden der cDNA-Sequenz mit der Sequenz aus Schritt (c) in einem Plasmid umfaßt.

**16.** Verfahren nach Anspruch 15, das weiter das Transformieren des Plasmides in einen Wirt umfaßt, der zur Expression des Plasmides fähig ist.

**17.** Verfahren nach einem der Ansprüche 14 bis 16, wobei die Kette eine schwere Kette ist.

**18.** Verfahren nach einem der Ansprüche 14 bis 16, wobei die Kette eine leichte Kette ist.

**19.** Verfahren nach Anspruch 14, wobei der Schritt (a) umfaßt:
a') das Bereitstellen einer mRNA, die für die variable Region kodiert, aus einer Zelle, die monoklonale Antikörper der gewünschten Spezifität ausscheidet; und a'') das Starten (Priming) der Bildung einer davon abgeleiteten cDNA durch reverse Transkription unter Verwendung der mRNA als Matrize mit einem Polynukleotidmolekül, das eine genetische Konsensussequenz für die J-Region der Immunglobulinkette umfaßt, wobei die Konsensussequenz mindestens 80% Sequenzhomologie mit bekannten J-Region-Sequenzen aufweist.

**20.** Verfahren nach Anspruch 19, wobei die genetische Konsensussequenz aus der Gruppe ausgewählt ist, die besteht aus: (i) Human-J-Region für schwere Ketten; (ii) Maus-J-Region für schwere Ketten; (iii) Human-Kappa-J-Region; (iv) Maus-Kappa-J-Region; und (v) Maus-Lambda-J-Region.

**21.** Verfahren nach Anspruch 19, wobei die genetische Konsensussequenz aus der Gruppe ausgewählt ist, die aus den mit MJH1, MJH2, MJH3, MJH3-BSTEII, MJH-BSTEII(13), MJH4, 5JK1, 5JK2, JK2BGLII, 5JK4, JK4BGLII, 5JK5 und MJK bezeichneten besteht:

| | |
|---|---|
| MJH1- | GCCAGTGGCAGAGGAGTCGGT |
| MJH2- | GAGAGTGTCAGACGAGTCGGT |
| MJH3- | ACCAGTGACAGAGACGTCGGT |
| MJH3-BSTEII- | TCCCTGAGACCAGTGGCAGAG |
| MJH-BSTEII(13)- | ACCAGTGGCAGAG |
| MJH4- | GTCAGTGGCAGAGGAGTCGGT |
| 5JK1- | GCAAGCCACCTCCGTGG |
| JK2BGLII- | CCCTGGTTCGACCTCTAGATT |
| 5JK2- | GTGCAAGCCTCCCCCCTGG |
| 5JK4- | GCAAGCCGAGCCCCTGT |
| JK4BGLII | GCCCCTGTTTCAACCTCTAGATT |
| 5JK5 | GCAAGCCACGACCCTGG |
| MJK | TGGTTCGACCTTTATTTTG |

**22.** Verfahren nach Anspruch 19, wobei die Konsensussequenz weiter die für die Erkennungsstelle eines Restriktionsendonukleaseenzymes kodierende Sequenz umfaßt.

**23.** cDNA-Expressionsvektoren mit RestriktionsendonukleaseSchnittstellenkarten, wie in Figur 10 gezeigt, die einen Promotor aus der frühen Region von SV40, eine Spleißsequenz aus der späten Region von SV40, den selektierbaren Marker neo, SV40 polyA-Signalsequenzen, eine Vielfachklonierungsstelle (pING2003) und gegebenenfalls zusätzlich ein Enhancer-Element aus einer schweren Mauskette (pING2003 E) enthalten.

**24.** Verfahren nach Anspruch 19, wobei die genetische Konsensussequenz aus der Gruppe ausgewählt ist, die aus den als UIGH, UIGK und $MJ_H2$-ApaI bezeichneten besteht:

| | |
|---|---|
| UIG-H | AGGGACCACGGTCACCGTCTC |
| UIG-K | GGGACCAAGCTTGAG |
| $MJ_H2$-ApaI | TGTCAGAGGAGTCGGTCGTGTTTCCCGGGTA |

25. Verfahren zum Erzeugen eines Vektors, der eine für die vollständige variable Region einer Immunglobulinkette kodierende cDNA-Sequenz umfaßt, wobei die Kette eine vollständige V-J-Verbindung im Fall einer leichten Kette und eine vollständige V-D-J-Verbindung im Fall einer schweren Kette umfaßt, in dem die cDNA-Sequenz in einen Vektor, dem eine konstante Region oder Intronsequenzen fehlen, eingeführt wird.

26. Verfahren zum Erzeugen eines DNA-Fragmentes, das für eine vollständige konstante IgG1-Region für eine schwere Human-Immunglobulinkette kodiert, durch Erhalten einer cDNA-Sequenz, die für eine vollständige konstante IgG1-Region für eine schwere Immunglobulinkette kodiert, wobei der DNA-Sequenz ein variabler Bereich fehlt und dem konstanten Bereich Intronsequenzen fehlen.

27. Verfahren zum Erzeugen eines Vektors, der eine kontinuierliche kodierende Sequenz, die nicht durch Introns unterbrochen ist, umfaßt, umfassend das Verbinden einer für die variable Region einer nicht-Human-Immunglobulinkette einschließlich einer V-J-Verbindung im Fall einer leichten Kette und einer V-D-J-Verbindung im Fall einer schweren Kette kodierenden DNA-Sequenz mit einer für die konstante Region einer Human-Immunglobulinkette kodierenden DNA-Sequenz.

28. Verfahren zum Erzeugen eines Vektors nach Anspruch 25 oder 27, wobei der Vektor ein Plasmid ist.

29. Verfahren zum Erzeugen eines Bakteriums, das den Vektor oder ein Fragment nach einem der Ansprüche 25 bis 28 enthält, durch Transformieren des Bakteriums mit dem Vektor oder Fragment.

30. Verfahren zum Erzeugen einer Säugetierzelle oder einer Hefe, die den Vektor oder ein Fragment nach einem der Ansprüche 25 bis 28 enthält, durch Transfizieren der Säugetierzelle oder der Hefe mit dem Vektor oder Fragment.

31. Verfahren zum Synthetisieren eines Polynukleotidmoleküls, umfassend die Konsensussequenz für die J-Region eines Immunglobulinmoleküls für eine schwere Kette, wobei die Konsensussequenz mindestens 80% Sequenzhomologie mit bekannten J-Region-Sequenzen aufweist, ohne damit identisch zu sein, indem das Polynukleotidmolekül in an sich bekannter Weise synthetisiert wird.

32. Verfahren nach Anspruch 31, wobei die Sequenz für eine J-Region einer schweren Human-Kette ist.

33. Verfahren nach Anspruch 31, wobei die Sequenz für eine J-Region einer schweren Maus-Kette ist.

34. Verfahren zum Erzeugen eines Polynukleotidmoleküls, das eine Konsensussequenz für die J-Region eines Immunglobulinmoleküles für eine leichte Kette umfaßt, wobei diese Konsensussequenz mindestens 80% Sequenzhomologie mit bekannten J-Region-Sequenzen aufweist, ohne damit identisch zu sein, durch Synthetisieren des Polynukleotidmoleküles in an sich bekannter Weise.

35. Verfahren nach Anspruch 34, wobei die Sequenz für eine Human-Kappa-J-Region ist.

36. Verfahren nach Anspruch 34, wobei die Sequenz für eine Maus-Kappa-J-Region ist.

37. Verfahren nach Anspruch 34, wobei die Sequenz für eine Maus-Lambda-J-Region ist.

38. Verfahren zum Erzeugen von cDNA-Expressionsvektoren mit Restriktionsendonuklease-Schnittstellenkarten, wie gezeigt in Figur 10, indem ein Promotor aus der frühen Region von SV40, eine Spleiß-Sequenz aus der späten Region von SV40, der selektierbare Marker neo, polyA-Signalsequenzen aus SV40, eine Vielfachklonierungsstelle (pING2003) und gegebenenfalls zusätzlich ein Enhancer-Element aus einer schweren Maus-Kette (pING2003 E) verbunden werden.

**Revendications**

**Revendications pour les Etats contractants suivants : BE, CH et LI, DE, FR, GB, IT, LU, NL, SE**

1. Un vecteur comprenant une codification de séquence de cADN pour la zone variable complète d'une chaîne d'immunoglobuline, ladite chaîne comportant une jonction V-J complète dans le cas d'une chaîne légère et une jonction V-D-J complète dans le cas d'une chaîne lourde, et dans ledit vecteur étant absente toute zone constante, ou toute séquence d'introns.

**2.** Un fragment d'ADN comprenant une codification de séquence de cADN pour la zone IgG1 constante complète pour une chaîne d';immunoglobuline humaine à chaînes lourdes, dans ledit fragment étant absente toute zone variable et dans ladite zone constante étant absente toute séquence d'introns.

**3.** Un vecteur comprenant une séquence de codification continue, non-interrompue par des introns, comprenant:

i) une codification de séquence d'ADN pour la zone variable d'une chaîne d'immunoglobuline non-humaine comportant une jonction V-J dans le cas d'une chaîne légère et une jonction V-D-J dans le cas d'une chaîne lourde.

ii) une codification de séquence d'ADN pour la zone constante d'une chaîne d'immunoglobuline humaine.

**4.** Le vecteur suivant les revendications 1 ou 3, qui est un plasmide.

**5.** Une bactérie transformée par le vecteur ou fragment suivant les revendications 1, 2, 3 ou 4.

**6.** Une cellule mammalienne ou levure transfectée par le vecteur ou fragment suivant les revendications 1, 2, 3 ou 4.

**7.** Une molécule de polynucléotide comprenant une séquence de consensus pour la zone J d'une molécule d'immunoglobuline à chaînes lourdes, dans laquelle ladite séquence de consensus présente au moins 80% d'homologie de séquence avec les séquences de zone J connues, sans être identique.

**8.** La molécule suivant la revendication 7, dans laquelle ladite séquence est pour une zone J de chaîne lourde humaine.

**9.** La molécule suivant la revendication 7, dans laquelle ladite séquence est pour une zone J de chaîne lourde de souris.

**10.** Une molécule de polynucléotide comprenant une séquence de consensus pour la zone J d'une molécule d'immunoglobuline à chaînes légères, dans laquelle ladite séquence de consensus présente au moins 80% d'homologie de séquence avec les séquences de zones J connues, sans être identique.

**11.** La molécule suivant la revendication 10, dans laquelle ladite séquence est pour une zone J Kappa humaine.

**12.** La molécule suivant la revendication 10, dans laquelle ladite séquence est pour une zone J Kappa de souris.

**13.** La molécule suivant la revendication 10, dans laquelle ladite séquence est pour une zone J Lambda de souris.

**14.** Une méthode pour la préparation directe d'une codification de séquence génétique pour une chaîne d'immunoglobuline chimérique ayant une zone humaine constante et une zone non-humaine variable à toute spécificité souhaitée, qui comprend:

a) la prévision d'une codification de séquence de cADN pour une zone variable non-humaine complète comportant une jonction V-J dans le cas d'une chaîne légère et une jonction V-D-J dans le cas d'une chaîne lourde, et dans laquelle est absente toute séquence de zone constante.

b) la prévision d'un vecteur contenant une codification de séquence génétique pour ladite zone constante,

c) la liaison fonctionnelle de ladite séquence a) audit vecteur b).

**15.** La méthode suivant la revendication 14, dans laquelle l'étape (c) comprend la liaison fonctionnelle de ladite séquence de cADN à ladite séquence de l'étape (c) dans un plasmide.

**16.** La méthode suivant la revendication 15 qui comprend, en outre, la transformation dudit plasmide dans un hôte susceptible d'exprimer ledit plasmide.

**17.** La méthode suivant l'une ou l'autre des revendications 14 à 16. dans laquelle ladite chaîne est une chaîne lourde.

**18.** La méthode suivant l'une ou l'autre des revendications 14 à 16. dans laquelle ladite chaîne est une chaîne légère.

**19.** La méthode suivant la revendication 14, dans laquelle ladite étape a) comprend:
a') la prévision d'une codification de mARN pour ladite zone variable à partir d'une cellule sécrétant des anticorps monoclones à ladite spécificité souhaitée, et
a'') l'amorçage de la formation, par transcription inverse à l'aide dudit mARN comme échantillon. de cADN dérivé de celui-ci. avec une molécule de polynucléotide comprenant une séquence génétique de consensus pour la zone J de ladite chaîne d'immunoglobuline, dans laquelle ladite séquence de consensus présente au moins 80% d'homologie de séquence avec les séquences de zone J connues.

**20.** La méthode suivant la revendication 19, dans laquelle ladite séquence génétique de consensus est choisie parmi le groupe composé de:
(i) zone J de chaîne lourde humaine,
(ii) zone J de chaîne lourde de souris,
(iii) zone J de Kappa humaine,
(iv) zone J de Kappa de souris.
(v) zone J de Lambda de souris.

**21.** La méthode suivant la revendication 19, dans laquelle ladite séquence génétique de consensus est choisie parmi le groupe composé de celles désignées par MJH1, MJH2, MJH3, MJH3-BSTEII. MJH-BSTEII(13), MJH4, 5JK1, 5JK2, 5JK2, JK2BGLII, 5JK4, JK4BGLII, 5JK5 et MJK:

| | |
|---|---|
| MJH1- | GCCAGTGGCAGAGGAGTCGGT |
| MJH2- | GAGAGTGTCAGACGAGTCGGT |
| MJH3- | ACCAGTGACAGAGACGTCGGT |
| MJH3-BSTEII- | TCCCTGAGACCAGTGGCAGAG |
| MJH-BSTEII(13)- | ACCAGTGGCAGAG |
| MJH4- | GTCAGTGGCAGAGGAGTCGGT |
| 5JK1- | GCAAGCCACCTCCGTGG |
| JK2BGLII- | CCCTGGTTCGACCTCTAGATT |
| 5JK2- | GTGCAAGCCTCCCCCCTGG |
| 5JK4- | GCAAGCCGAGCCCCTGT |
| JK4BGLII | GCCCCTGTTTCAACCTCTAGATT |
| 5JK5 | GCAAGCCACGACCCTGG |

| | |
|---|---|
| MJK | TGGTTCGACCTTTATTTTG |

**22.** La méthode suivant la revendication 19, dans laquelle ladite séquence de consensus comprend, en outre, la codification de séquence pour le site de reconnaisance d'un enzyme endonucléase de restriction.

**23.** Vecteurs d'expression de cADN ayant des cartes de sites d'endonucléase de restriction telles qu'illustrées à la Figure 10 contentant un promoteur de zone précoce SV40, une séquence de greffe de zone tardive SV40 , le marqueur sélectionnable neo, des séquences de signaux polyA SV40, un site de clonage multiple (pING2003) et optionnellement, en outre, un élément rehausseur de chaîne lourde de souris (pING2003 E).

**24.** La méthode suivant la revendication 19, dans laquelle ladite sé quence génétique de consensus est choisie parmi le groupe composé de celles désignées par UIGH, UIGK et $MJ_H2$ -APaI:

| | |
|---|---|
| UIG-H | AGGGACCACGGTCACCGTCTC |
| UIG-K | GGGACCAAGCTTGAG |
| $MJ_H2$-ApaI | TGTCAGAGGAGTCGGTCGTGTTTCCCGGGTA |

**Revendications pour les Etats contractants suivant : AT**

**1.** Un vecteur comprenant une codification de séquence de cADN pour la zone variable complète d'une chaîne d'immunoglobuline, ladite chaîne comportant une jonction V-J complète dans le cas d'une chaîne légère et une jonction V-D-J complète dans le cas d'une chaîne lourde, et dans ledit vecteur étant absente toute zone constante, ou toute séquence d'introns.

**2.** Un fragment d'ADN comprenant une codification de séquence de cADN pour la zone IgG1 constante complète pour une chaîne d';immunoglobuline humaine à chaînes lourdes, dans ledit fragment étant absente toute zone variable et dans ladite zone constante étant absente toute séquence d'introns.

**3.** Un vecteur comprenant une séquence de codification continue, non-interrompue par des introns, comprenant:

i) une codification de séquence d'AN pour la zone variable d'une chaîne d'immunoglobuline non-humaine comportant une jonction V-J dans le cas d'une chaîne légère et une jonction V-D-J dans le cas d'une chaîne lourde,

ii) une codification de séquence d'ADN pour la zone constante d'une chaîne d'immunoglobuline humaine.

**4.** Le vecteur suivant les revendications 1 ou 3, qui est un plasmide.

**5.** Une bactérie transformée par le vecteur ou fragment suivant les revendications 1, 2, 3 ou 4.

**6.** Une cellule mammalienne ou levure transfectée par le vecteur ou fragment suivant les revendications 1, 2, 3 ou 4.

**7.** Une molécule de polynucléotide comprenant une séquence de consensus pour la zone J d'une molécule d'immunoglobuline à chaînes lourdes, dans laquelle ladite séquence de consensus présente au moins 80% d'homologie de séquence avec les séquences de zone J connues, sans être identique.

**8.** La molécule suivant la revendication 7, dans laquelle ladite séquence est pour une zone J de chaîne lourde humaine.

**9.** La molécule suivant la revendication 7, dans laquelle ladite séquence est pour une zone J de chaîne lourde de souris.

**10.** Une molécule de polynucléotide comprenant une séquence de consensus pour la zone J d'une molécule d'immunoglobuline à chaînes légères, dans laquelle ladite séquence de consensus présente au moins 80% d'homologie de séquence avec les séquences de zones J connues, sans être identique.

11. La molécule suivant la revendication 10, dans laquelle ladite séquence est pour une zone J Kappa humaine.

12. La molécule suivant la revendication 10, dans laquelle ladite séquence est pour une zone J Kappa de souris.

13. La molécule suivant la revendication 10, dans laquelle ladite séquence est pour une zone J Lambda de souris.

14. Une méthode pour la préparation directe d'une codification de séquence génétique pour une chaîne d'immunoglobuline chimérique ayant une zone humaine constante et une zone non-humaine variable à toute spécificité souhaitée, qui comprend:
a) la prévision d'une codification de séquence de cADN pour une zone variable non-humaine complète comportant une jonction V-J dans le cas d'une chaîne légère et une jonction V-D-J dans le cas d'une chaîne lourde, et dans laquelle est absente toute séquence de zone constante,
b) la prévision d'un vecteur contenant une codification de séquence génétique pour ladite zone constante,
c) la liaison fonctionnelle de ladite séquence a) audit vecteur b).

15. La méthode suivant la revendication 14, dans laquelle l'étape (c) comprend la liaison fonctionnelle de ladite séquence de cADN à ladite séquence de l'étape (c) dans un plasmide.

16. La méthode suivant la revendication 15 qui comprend, en outre, la transformation dudit plasmide dans un hôte susceptible d'exprimer ledit plasmide.

17. La méthode suivant l'une ou l'autre des revendications 14 à 16, dans laquelle ladite chaîne est une chaîne lourde.

18. La méthode suivant l'une ou l'autre des revendications 14 à 16, dans laquelle ladite chaîne est une chaîne légère.

19. La méthode suivant la revendication 14, dans laquelle ladite étape a) comprend:
a') la prévision d'une codification de mARN pour ladite zone variable à partir d'une cellule sécrétant des anticorps monoclones à ladite spécificité souhaitée, et
a'') l'amorçage de la formation, par transcription inverse à l'aide dudit mARN comme échantillon, de cADN dérivé de celui-ci, avec une molécule de polynucléotide comprenant une séquence génétique de consensus pour la zone J de ladite chaîne d'immunoglobuline, dans laquelle ladite séquence de consensus présente au moins 80% d'homologie de séquence avec les séquences de zone J connues.

20. La méthode suivant la revendication 19, dans laquelle ladite séquence génétique de consensus est choisie parmi le groupe composé de:
(i) zone J de chaîne lourde humaine,
(ii) zone J de chaîne lourde de souris,
(iii) zone J de Kappa humaine,
(iv) zone J de Kappa de souris,
(v) zone J de Lambda de souris.

21. La méthode suivant la revendication 19, dans laquelle ladite séquence génétique de consensus est choisie parmi le groupe composé de celles désignées par MJH1, MJH2, MJH3, MJH3-BSTEII, MJH-BSTEII(13), MJH4, 5JK1, 5JK2, JK2BGLII, 5JK4, JK4BGLII, 5JK5 et MJK:

| | |
|---|---|
| MJH1- | GCCAGTGGCAGAGGAGTCGGT |
| MJH2- | GAGAGTGTCAGACGAGTCGGT |
| MJH3- | ACCAGTGACAGAGACGTCGGT |
| MJH3-BSTEII- | TCCCTGAGACCAGTGGCAGAG |
| MJH-BSTEII(13)- | ACCAGTGGCAGAG |
| MJH4- | GTCAGTGGCAGAGGAGTCGGT |
| 5JK1- | GCAAGCCACCTCCGTGG |
| JK2BGLII- | CCCTGGTTCGACCTCTAGATT |
| 5JK2- | GTGCAAGCCTCCCCCCTGG |
| 5JK4- | GCAAGCCGAGCCCCTGT |
| JK4BGLII | GCCCCTGTTTCAACCTCTAGATT |
| 5JK5 | GCAAGCCACGACCCTGG |

| | |
|---|---|
| MJK | TGGTTCGACCTTTATTTTG |

**22.** La méthode suivant la revendication 19, dans laquelle ladite séquence de consensus comprend, en outre, la codification de séquence pour le site de reconnaissance d'un enzyme endonucléase de restriction.

**23.** Vecteurs d'expression de cADN ayant des cartes de sites d'endonucléase de restriction telles qu'illustrées à la Figure 10 contenant un promoteur de zone précoce SV40, une séquence de greffe de zone tardive SV40, le marqueur sélectionnable neo, des séquences de signaux polyA SV40 un site de clonage multiple (pING2003) et optionnellement, en outre, un élément rehausseur de chaîne lourde de souris (pING2003 E).

**24.** La méthode suivant la revendication 19, dans laquelle ladite séquence génétique de consensus est choisie parmi le groupe composé de celles désignées par UIGH, UIGK et $MJ_H2$-ApaI:

| | |
|---|---|
| UIG-H | AGGGACCACGGTCACCGTCTC |
| UIG-K | GGGACCAAGCTTGAG |
| $MJ_H2$-ApaI | TGTCAGAGGAGTCGGTCGTGTTTCCCGGGTA |

**25.** Une méthode pour la production d'un vecteur comprenant une codification de séquence de cADN pour la zone variable complète d'une chaîne d'immunoglobuline, ladite chaîne comportant une jonction V-J complète, dans le cas d'une chaîne légère, et une jonction V-D-J complète, dans le cas d'une chaîne lourde, par l'introduction de ladite séquence de cADN dans un vecteur dans lequel font défaut toute zone constante ou toutes séquences d'introns.

**26.** Une méthode pour la production d'une codification de fragment d'ADN pour la zone IgG1 constante complète pour une chaîne d'immunoglobuline humaine à chaines lourdes, par l'obtention d'une codification de séquence de cADN pour la zone IgG1 constante complète pour une chaîne d'immunoglobuline à chaines lourdes, dans ladite séquence d'ADN faisant défaut toute zone variable et dans ladite zone constante faisant défaut toutes séquences d'introns.

**27.** Une méthode pour la production d'un vecteur comprenant une séquence de codification continue, non-interrompue par des introns, comprenant la combinaison d'une codification de séquence d'ADN pour la zone variable d'une chaîne d'immunoglobuline non-humaine comportant une jonction V-J, dans le cas d'une chaîne légère, et une jonction V-D-J, dans le cas d'une chaîne lourde, avec une codification de séquence d'ADN pour la zone constante d'une chaîne d'immunoglobuline humaine.

**28.** Une méthode pour la production d'une codification de vecteur suivant la revendication 25 ou 27, dans laquelle le vecteur est un plasmide.

**29.** Une méthode pour la production d'une bactérie contenant le vecteur ou fragment suivant l'une ou l'autre des revendications 25 à 28, par transformation de la bactérie par ledit vecteur ou fragment.

**30.** Une méthode pour la production d'une cellule mammalienne ou d'une levure contenant le vecteur ou fragment suivant l'une ou l'autre des revendications 25 à 28, par transfection de la cellule mammalienne ou levure par ledit vecteur ou fragment.

**31.** Une méthode pour synthétiser une molécule de polynucléotide comprenant une séquence de consensus pour la zone J d'une molécule d'immunoglobuline à chaînes lourdes, dans laquelle ladite séquence de consensus présente au moins 80% d'homologie de séquence avec les séquences de zone J connues, sans être identique, par synthétisation de ladite molécule de polynucléotide de manière connue en soi.

**32.** La méthode suivant la revendication 31, dans laquelle ladite séquence est pour une zone J à chaînes lourdes humaines.

**33.** La méthode suivant la revendication 31, dans laquelle ladite séquence est pour une zone J à chaînes lourdes de souris.

**34.** Une méthode pour la production d'une molécule de polynucléotide comprenant une séquence de consensus pour la zone J d'une molécule d'immunoglobuline à chaînes légères, dans laquelle ladite séquence de consensus présente au moins 80% d'homologie de séquence avec les séquences de zone J connues, sans être identique, par synthétisation de ladite molécule de polynucléotide de manière connue en soi.

**35.** La méthode suivant la revendication 34, dans laquelle ladite séquence est pour une zone J Kappa humaine.

**36.** La méthode suivant la revendication 34, dans laquelle ladite séquence est pour une zone J Kappa de souris.

**37.** La méthode suivant lad revendication 34, dans laquelle ladite séquence est pour une zone J Lambda de souris.

**38.** Une méthode pour la production d'une vecteur d'expression de cADN ayant des cartes de sites d'endonucléase de restriction, tel qu'il lustré à la Figure 10, par combinaision d'un promoteur de zone précoce SV40, d'une séquence de greffe de zone tardrive SV40, du marqueur sélectionnable neo, de séquences de signaux polyA SV40, d'un site de clonage multiple (pING2003) et optionnellement, en outre, d'un élément rehausseur de chaîne lourde de souris (pING2003 E).

FIG. 1

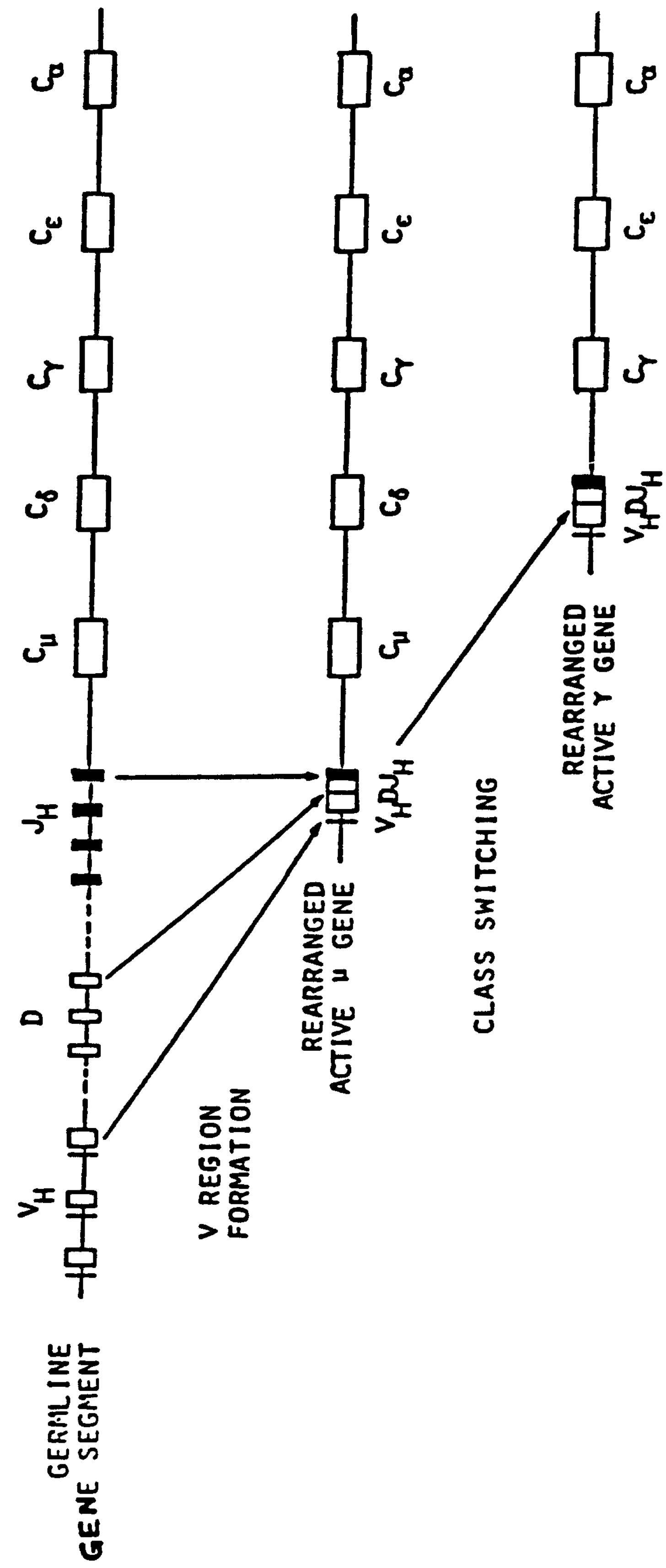
GERMLINE GENE SEGMENT
V REGION FORMATION
REARRANGED ACTIVE μ GENE
CLASS SWITCHING
REARRANGED ACTIVE γ GENE
$V_H$
D
$J_H$
$C_\mu$
$C_\delta$
$C_\gamma$
$C_\varepsilon$
$C_\alpha$
$V_HDJ_H$

# FIG. 2

## Ig heavy chain J-C region

**human heavy chain J regions** J | CH1

```
JH1    GCTGAATACTTCCAGCACTGGGGCCAGGGCACCCTGGTCACCGTCTCCTCAG
JH2   CTACTGGTACTTCGATCTCTGGGGCCGTGGCACCCTGGTCACTGTCTCCTCAG
JH3          ATGCTTTTGATGTCTGGGGCCAAGGGACAATGGTCACCGTCTCTTCAG
JH4          ACTACTTTGACTACTGGGGCCAAGGAACCCTGGTCACCGTCTCCTCAG
JH5        ACACTGGTTCGACTCCTGGGGCCAAGGAACCCTGGTCACCGTCTCCTCAG
JH6 AT(TAC)5GGTATGGACGTCTGGGGGCAAGGGACCACGGTCACCGTCTCCTCAG
Consensus          TCGACCTCTGGGGCCAAGGAACCCTGGTCACCGTCTCCTCAG
```

**mouse heavy chain J regions** J | CH1

```
JH1    TACTGGTACTTCGATGTCTGGGGCGCAGGGACCACGGTCACCGTCTCCTCAG
JH2           TACTTTGACTACTGGGGCCAAGGCACCACTCTCACAGTCTCCTCAG
JH3         CCTGGTTTGCTTACTGGGGCCAAGGGACTCTGGTCACTGTCTCTGCAG
JH4    TACTATGCTATGGACTACTGGGGTCAAGGAACCTCAGTCACCGTCTCCTCAG
Consensus        TTTGACTACTGGGGCCAAGGGACCACGGTCACCGTCTCCTCAG
```

## Ig light chain J-C region

**human Kappa J region** J | C

```
JK1  GGACGTTCGGCCAAGGGACCAAGGTGGAAATCAAAC
JK2  ACACTTTTGGCCAGGGGACCAAGCTGGAGATCAAAC
JK3  TCACTTTCGGCCCTGGGACCAAAGTGGATATCAAAC
JK4  TCACTTTCGGCGGAGGGACCAAGGTGGAGATCAAAC
JK5  TCACCTTCGGCCAAGGGACACGACTGGAGATTAAAC
Consensus TTCGGCCAAGGGACCAAGGTGGAGATCAAAC
```

**mouse Kappa J region** J | C

```
JK1 TGGACGTTCGGTGGAGGCACCAAGCTGGAAATCAAAC
JK2 TACACGTTCGGAGGGGGGACCAAGCTGGAAATAAAAC
JK3 TTCACATTCAGTGATGGGACCAGACTGGAAATAAAAC
JK4 TTCACGTTCGGCTCGGGGACAAAGTTGGAAATAAAAC
JK5 CTCACGTTCGGTGCTGGGACCAAGCTGGAGCTGAAAC
Consensus TTCGGTGGGGGGACCAAGCTGGAAATAAAAC
UIG[MJK]          3'TGGTTCGACCTTTATTTTG5'
```

**human Lambda pseudo J region** J | C

```
JPSL1 CACATGTTTGGCAGCAAGACCCAGCCCACTGTCTTAG
```

**mouse Lambda J region** J | C

```
JL1 TGGGTGTTCGGTGGAGGAACCAAACTGACTGTCCTAG
JL2 TATGTTTTCGGCGGTGGAACCAAGGTCACTGTCCTAG
JL3 TTTATTTTCGGCAGTGGAACCAAGGTCACTGTCCTAG
Consensus TTCGGCGGTGGAACCAAGGTCACTGTCCTAG
```

FIG. 3

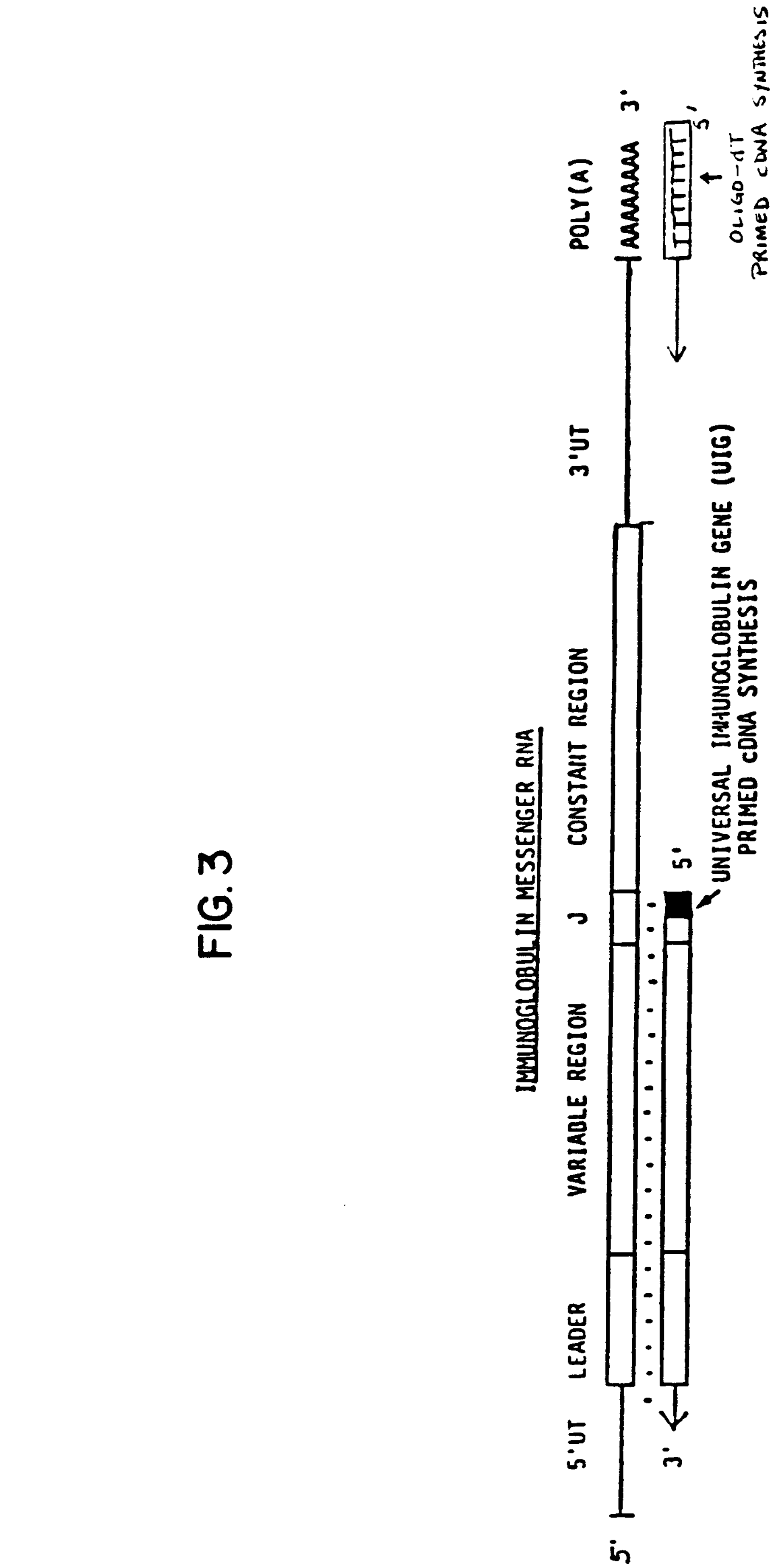
IMMUNOGLOBULIN MESSENGER RNA
5'
5'UT
LEADER
VARIABLE REGION
J
CONSTAIT REGION
3'UT
POLY(A)
AAAAAAA
3'
3'
5'
UNIVERSAL IMMUNOGLOBULIN GENE (UIG)
PRIMED cDNA SYNTHESIS
TTTTTTT
5'
OLIGO-dT
PRIMED cDNA SYNTHESIS

FIG. 4

A. Synthesis of Human IgG1 Genes

a. Human IgG1 Heavy Chain Structure

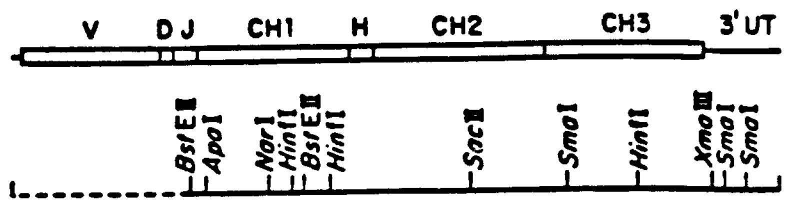


b. cDNA Clones

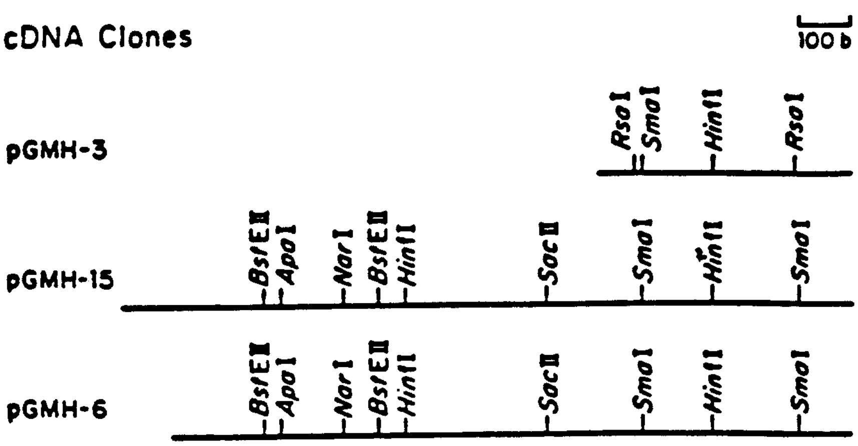


B. A Human IgG1 Constant Region Cloning Vector for V Region Module Insertion

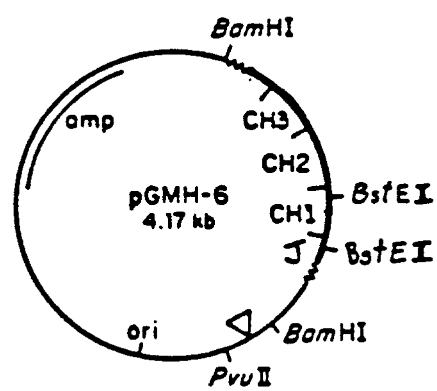

FIG. 5

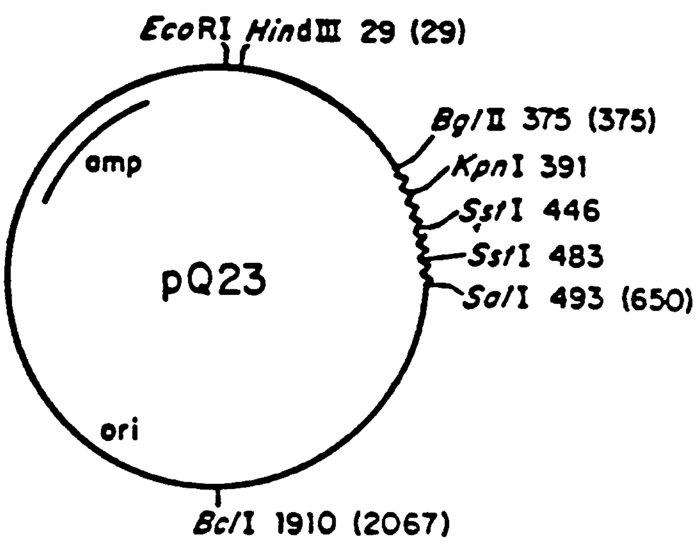
EcoRI
HindIII 29 (29)
BglII 375 (375)
KpnI 391
SstI 446
SstI 483
SalI 493 (650)
amp
pQ23
ori
BclI 1910 (2067)

FIG. 6

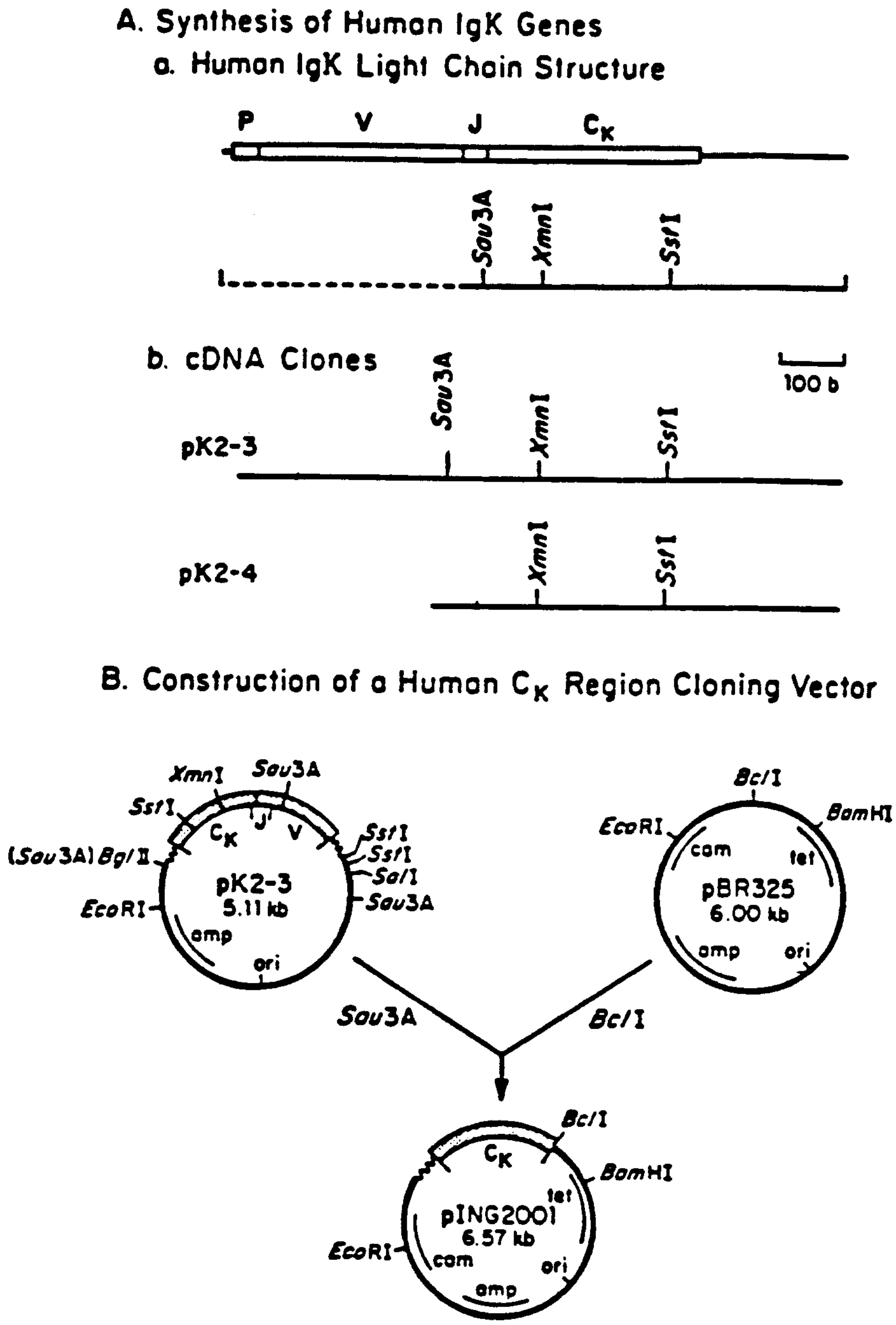
A. Synthesis of Human IgK Genes
a. Human IgK Light Chain Structure
P
V
J
CK
Sau3A
XmnI
SstI
b. cDNA Clones
100 b
pK2-3
pK2-4
B. Construction of a Human CK Region Cloning Vector
XmnI
Sau3A
SstI
(Sau3A) BglII
EcoRI
CK
J
V
SstI
SstI
SalI
Sau3A
pK2-3
5.11 kb
amp
ori
BclI
BamHI
EcoRI
cam
tet
pBR325
6.00 kb
amp
ori
Sau3A
BclI
pING2001
6.57 kb

# Fig. 7

**Primers Designed for Ig V Region Synthesis**

**A.** Ig Heavy Chain J-C Region

—J Region ———+— IgG1 CH1 Region —

| | |
|---|---|
| Human IgG1 pGMH-6 | GGTCACCGTCTCCTCAG CCTCCACCAAGGGCCCATC<br>Bst EII |

Mouse Heavy Chain J Regions and Primers

| | | Mismatches | | | | |
|---|---|---|---|---|---|---|
| | | N | JH1 | JH2 | JH3 | JH4 |
| JH1 | TACTGGTACTTCGATGTCTGGGGCGCAGGGACCACGGTCACCGTCTCCTCAG | | | | | |
| [MJH1] | GCCAGTGGCAGAGGAGTCGGT | 21 | 0 | 4 | 4 | 1 |
| JH2 | TACTTTGACTACTGGGGCCAAGGCACCACTCTCACAGTCTCCTCAG | | | | | |
| [MJH2] | GAGAGTGTCAGACGAGTCGGT | 21 | 4 | 1 | 7 | 4 |
| JH3 | CCTGGTTTGCTTACTGGGGCCAAGGGACTCTGGTCACTGTCTCTGCAG | | | | | |
| [MJH3] | ACCAGTGACAGAGACGTCGGT | 21 | 4 | 7 | 0 | 5 |
| [MJH3-BSTEII] | TCCCTGAGACCAGTGGCAGAG | 21 | 3 | 7 | 1 | 5 |
| [MJH-BSTEII (13)] | ACCAGTGGCAGAG | 13 | 1 | 4 | 1 | 2 |
| [MJH-BSTEII (13)] | Bst EII | | | | | |
| JH4 | TACTATGCTATGGACTACTGGGGTCAAGGAACCTCAGTCACCGTCTCCTCAG | | | | | |
| [MJH4] | GTCAGTGGCAGAGGAGTCGGT | 21 | 1 | 4 | 5 | 0 |

**B.** Ig Kappa Chain J-C Region

— J Region —+——— IgK Constant Region ———

| | |
|---|---|
| Human Kappa pK2-3 | CTGGAGATGAAAC GAACTGTGGCTGCACCATCTGTCTTCATCTTCCC |
| pING2016E | TGATCAAAC GAACTGTGGCTGCACCATCTGTCTTCATCTTCCC<br>BC/I |

Mouse Heavy Kappa J Regions and Primers

| | | Mismatches | | | | |
|---|---|---|---|---|---|---|
| | | N | JK1 | JK2 | JK4 | JK5 |
| JK1 | TGGACGTTCGGTGGAGGCACCAAGCTGGAAATCAAAC | | | | | |
| [5JK1] | GCAAGCCACCTCCGTGG | 17 | 0 | 3 | 6 | 3 |
| JK2 | TACACGTTCGGAGGGGGGACCAAGCTGGAAATAAAAC | | | | | |
| [JK2BGLII] | CCCTGGTTCGACCTCTAGATT | 21 | 3 | 3 | 5 | 3 |
| [5JK2] | GTGCAAGCCTCCCCCCTGG  BgII | | | | | |
| JK4 | TTCACGTTCGGCTCGGGGACAAAGTTGGAAATAAAAC | | | | | |
| [5JK4] | GCAAGCCGAGCCCCTGT | 17 | 6 | 4 | 0 | 4 |
| [JK4BGLII] | GCCCCTGTTTCAACCTCTAGATT<br>Bg II | 23 | 7 | 6 | 3 | 6 |
| JK5 | CTCACGTTCGGTGCTGGGACCAAGCTGGAGCTGAAAC | | | | | |
| [5JK5] | GCAAGCCACGACCCTGG | 17 | 3 | 3 | 4 | 0 |
| [MJK] | TGGTTCGACCTTTATTTTG | 19 | 1 | 0 | 2 | 3 |

# FIG. 7B

C. Mouse Variable Region Consensus Primers

mouse heavy chain J segments

```
JH1     TACTGGTACTTCGATGTCTGGGGCGCAGGGACCAC GGTCACC GTCTCCTCA
JH2            TACTTTGACTACTGGGGCCAAGGCACCAC TCTCACA GTCTCCTCA
JH3         CCTGGTTTGCTTACTGGGGCCAAGGGACTCTG GGTCACT GTCTCTGCA
JH4     TACTATGCTATGGACTACTGGGGTCAAGGAACCTC AGTCACC GTCTCCTCA

consensus primer:  UIG-H            AGGGACCAC GGTCACC GTCTC
                                              BstEII
                                    TCCCTGGTG CCAGTGG CAGAG
                                   3'                      5'
```

mouse light chain J segments

```
JK1                   TGGACGTTCGGTGGAGGCACC AAGCTG GAAATCAAA
JK2                   TACACGTTCGGAGGGGGGACC AAGCTG GAAATAAAA
JK4                   TTCACGTTCGGCTCGGGGACA AAGTTG GAAATAAAA
JK5                   CTCACGTTCGGTGCTGGGACC AAGCTG GAGCTGAAA

consensus primer:  UIG-K             GGGACC AAGCTT GAG
                                            HindIII
                                     CCCTGG TTCGAA CTC
                                    3'               5'

pGML60                           GGAGGGACC AAGGTG GAGATGAAA
                                 ---------C-T--------
                                          HindIII
```

D. Mouse γ2a J/C Junction Primer

```
MJH2-ApaI          TGTCAGAGGAGTCGGTCGTGTTTCCCGGGTA
                  3'                       ApaI   5'
```

FIG.9B

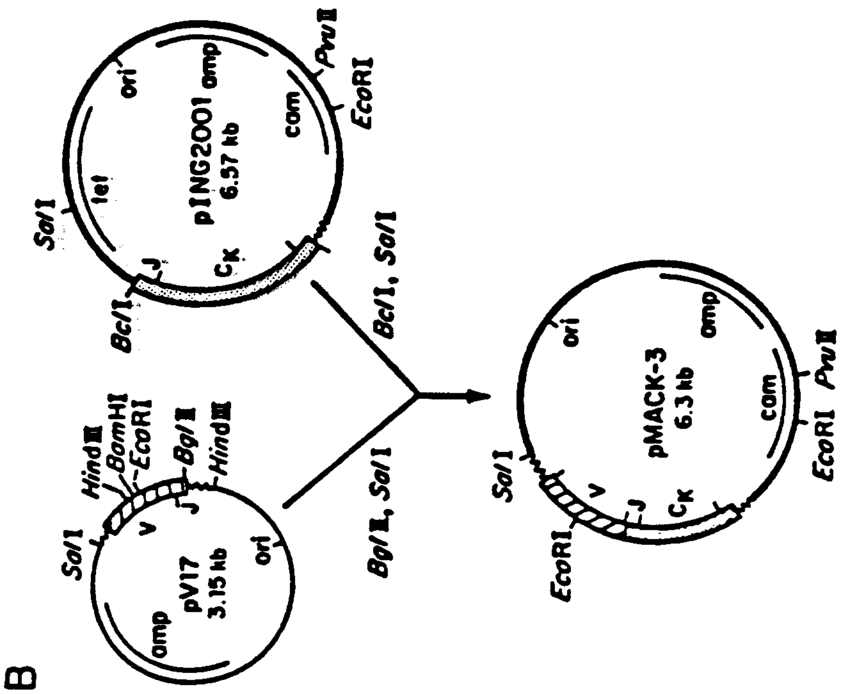
SalI
tet
ori
pING2001
6.57 kb
amp
cam
PvuII
EcoRI
BclI
J
Cκ
BclI, SalI
SalI
HindIII
BamHI
EcoRI
BglII
HindIII
V
J
pV17
3.15 kb
ori
amp
BglII, SalI
pMACK-3
6.3 kb
ori
amp
cam
EcoRI
PvuII
SalI
V
J
Cκ
EcoRI

FIG.9A

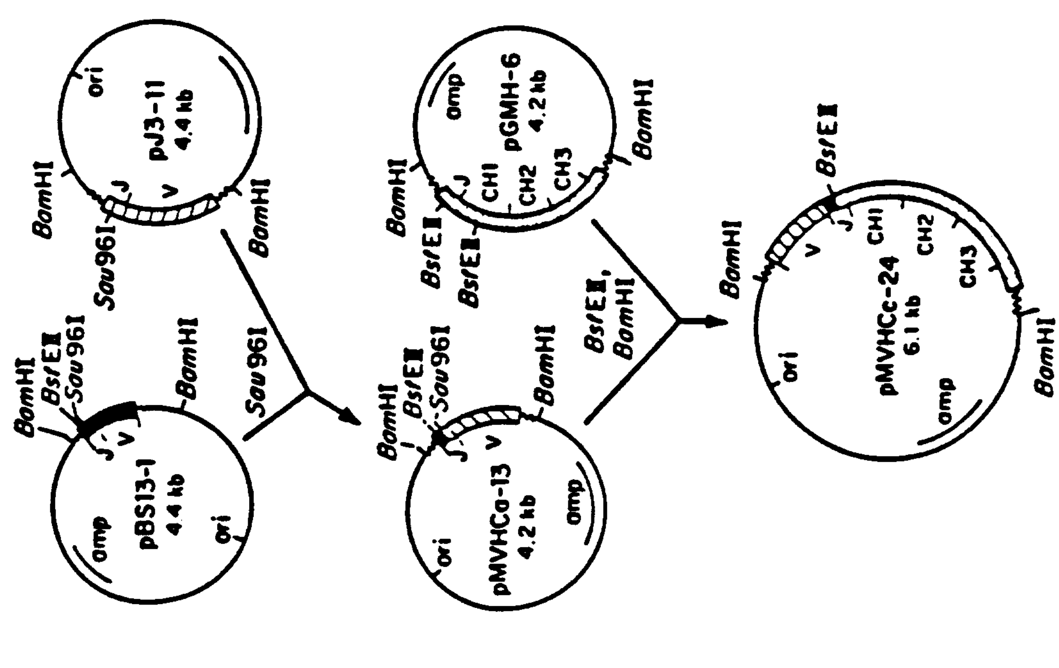
BamHI
BstEII
Sau96I
J
V
BamHI
pBS13-1
4.4 kb
amp
ori
Sau96I
BamHI
Sau96I
J
V
pJ3-11
4.4 kb
ori
BamHI
BamHI
BstEII
Sau96I
BamHI
pMVHCa-13
4.2 kb
ori
amp
BamHI
BstEII
BstEII
J
CH1
CH2
CH3
pGMH-6
4.2 kb
amp
BamHI
BstEII,
BamHI
BstEII
BamHI
V
J
CH1
CH2
CH3
pMVHCc-24
6.1 kb
ori
amp
BamHI

# FIG. 8

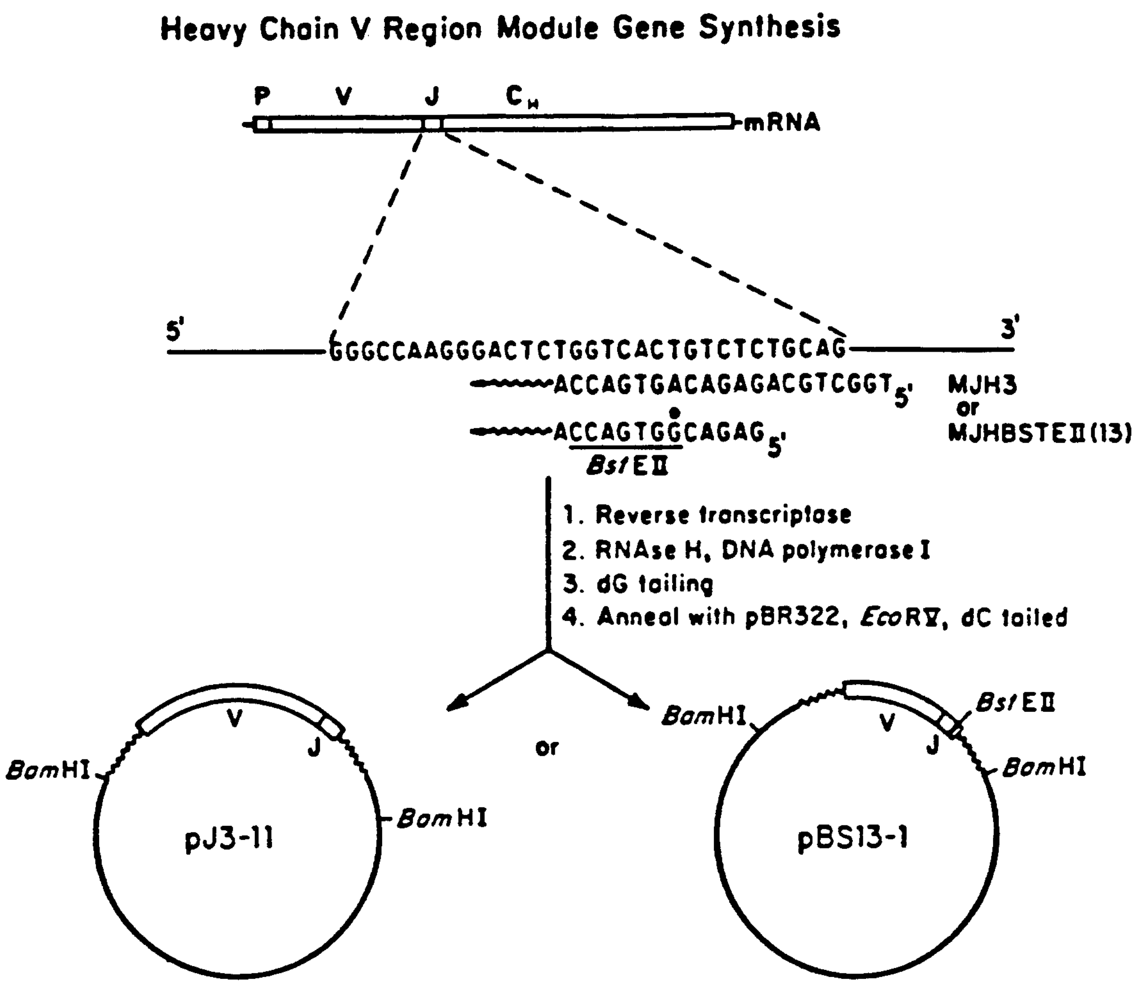
Heavy Chain V Region Module Gene Synthesis
P
V
J
$C_H$
mRNA
5'
3'
GGGCCAAGGGACTCTGGTCACTGTCTCTGCAG
ACCAGTGACAGAGACGTCGGT 5'
MJH3
or
MJHBSTEII(13)
ACCAGTGGCAGAG 5'
BstEII
1. Reverse transcriptase
2. RNAse H, DNA polymerase I
3. dG tailing
4. Anneal with pBR322, EcoRV, dC tailed
or
BamHI
V
J
BamHI
pJ3-11
BamHI
V
J
BstEII
BamHI
pBS13-1

FIG. 10

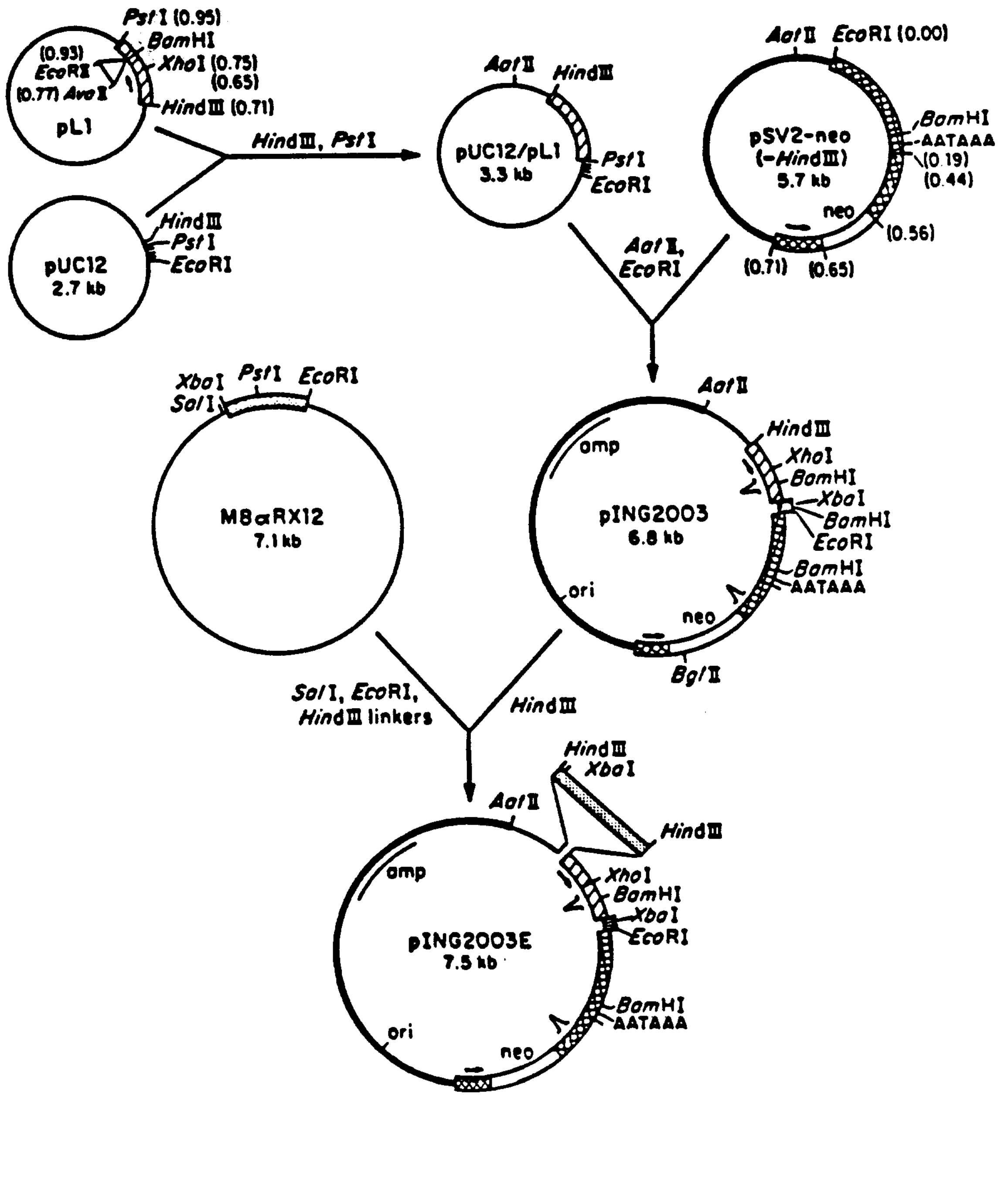
PstI (0.95)
BamHI
(0.93)
EcoRI
XhoI (0.75)
(0.65)
(0.77) AvaII
HindIII (0.71)
pL1
HindIII, PstI
pUC12
2.7 kb
HindIII
PstI
EcoRI
AatII
HindIII
pUC12/pL1
3.3 kb
PstI
EcoRI
AatII
EcoRI (0.00)
pSV2-neo
(-HindIII)
5.7 kb
BamHI
AATAAA
(0.19)
(0.44)
neo
(0.56)
(0.71)
(0.65)
AatII,
EcoRI
XbaI
PstI
EcoRI
SalI
M8αRX12
7.1 kb
AatII
HindIII
omp
XhoI
BamHI
XbaI
BamHI
EcoRI
pING2003
6.8 kb
BamHI
AATAAA
ori
neo
BglII
SalI, EcoRI,
HindIII linkers
HindIII
HindIII
XbaI
AatII
HindIII
omp
XhoI
BamHI
XbaI
EcoRI
pING2003E
7.5 kb
BamHI
AATAAA
ori
neo

FIG. 11

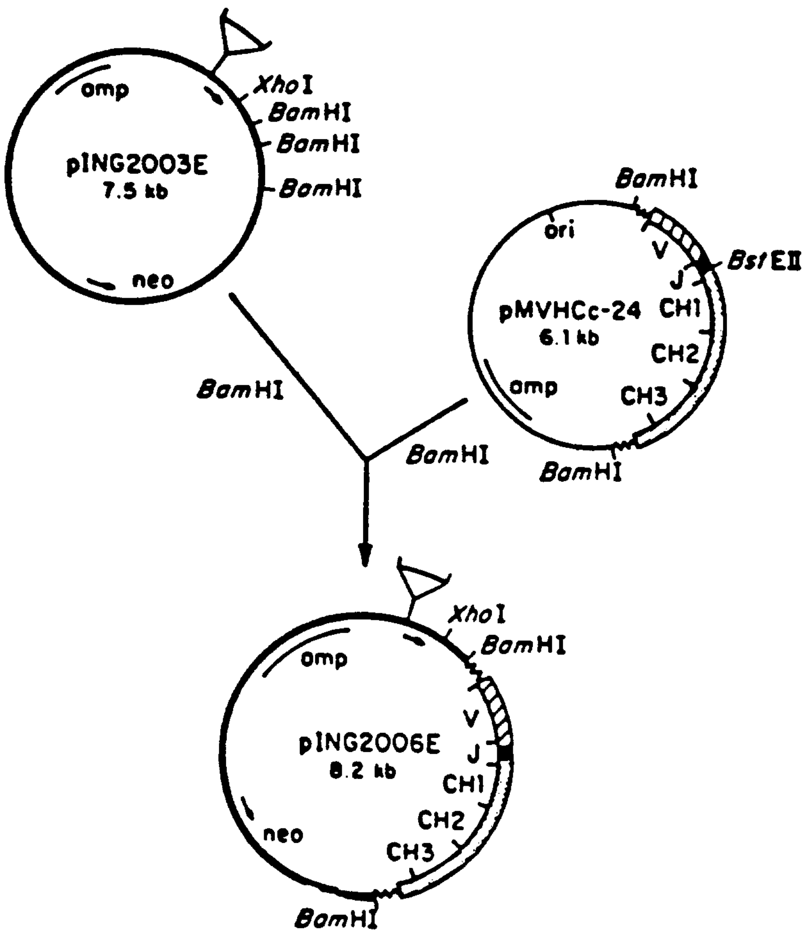
omp
XhoI
BamHI
BamHI
pING2003E
7.5 kb
BamHI
neo
BamHI
ori
V
BstEII
J
pMVHCc-24
6.1 kb
CH1
CH2
omp
CH3
BamHI
BamHI
BamHI
XhoI
BamHI
omp
V
pING2006E
8.2 kb
J
CH1
CH2
neo
CH3
BamHI

Fig. 12

A

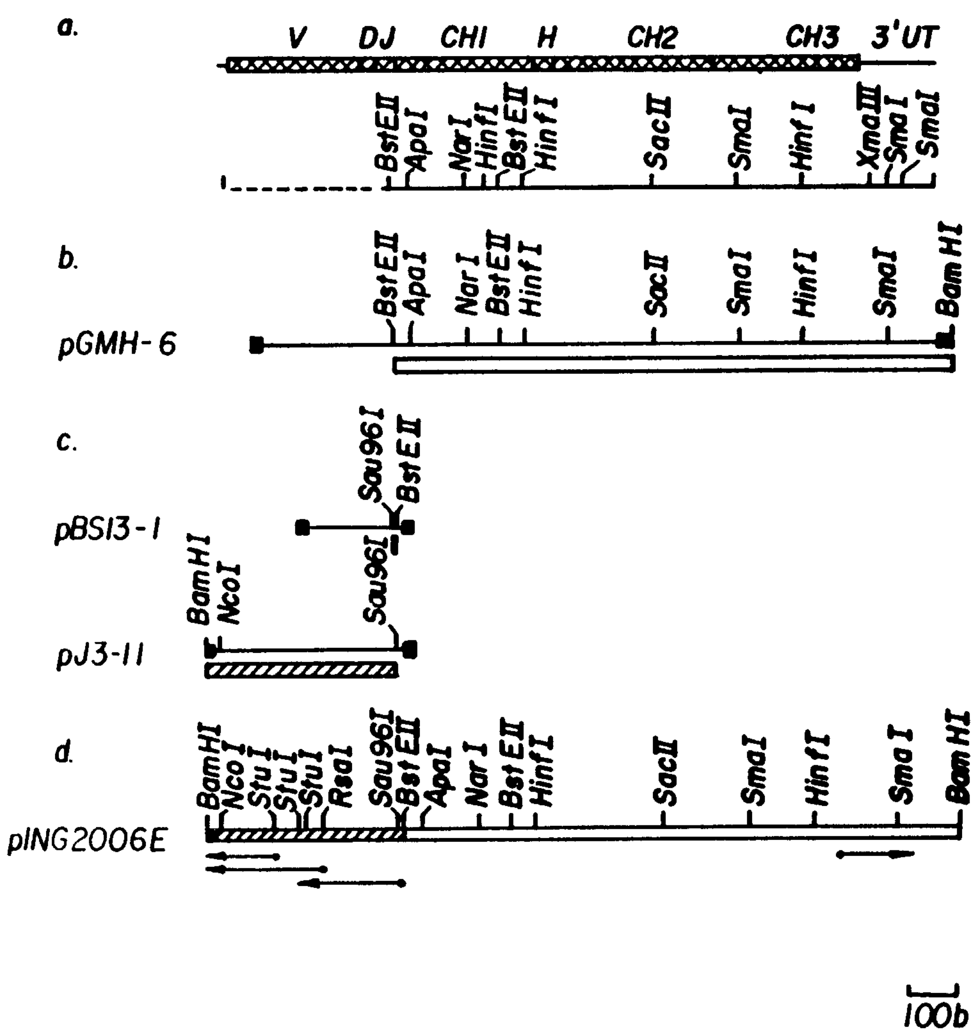


B

MET Gly Trp Ser Tyr Ile Ile Leu Phe Leu Val Ala Thr Ala Arg Asp
pING2006E GGA TCCμ CCC ACC ATG GGA TGG AGC TAT ATC ATC CTC TTT TTG GTA GCA ACA GCT AGA GAT
pING2012E GGA TCT GTC GAC ATG
30 45 60

2 * 12 17
Val His Ser Gln Val Gln Leu Gln Gln Pro Gly Ala Glu Leu Val Lys Pro Gly Ala Ser
GTG CAC TCC CAG GTC CAA TTG CAG CAG CCT GGG GCT GAA CTG GTG AAA CCT GGG GCT TCA
75 90 105 120

22 27 32 37
Val Lys Val Ser Cys Lys Ala Ser Gly Tyr Thr Phe Thr Ser Tyr Trp MET His Trp Val
GTG AAG GTG TCC TGC AAG GCC TCT GGC TAC ACC TTC ACC AGC TAC TGG ATG CAC TGG GTG
135 150 165 180

42 47 52 57
Lys Gln Arg Pro Gly Gln Gly Leu Asp Trp Ile Gly Glu Ile Asn Pro Ser Asn Gly Arg
AAG CAG AGG CCT GGA CAA GGC CTT GAC TGG ATT GGA GAG ATT AAT CCT AGC AAC GGT CGT
195 210 225 240

62 67 72 77
Thr Asn Tyr Asn Glu Lys Phe Lys Ser Lys Ala Thr Leu Thr Val Asp Lys Ser Ser Ser
ACT AAT TAC AAT GAG AAG TTC AAG AGC AAG GCC ACA CTG ACT GTA GAC AAA TCC TCC AGC
255 270 285 300

82 87 92 97
Thr Ala Tyr MET Gln Leu Ser Ser Leu Thr Ser Glu Asp Ser Ala Val Tyr Tyr Cys Ala
ACA GCC TAC ATG CAA CTC AGC AGC CTG ACA TCT GAG GAC TCT GCG GTC TAT TAC TGT GCC
315 330 345 360

BstEII
102 107 112 117
Ser Tyr Asp Tyr Asp Trp Phe Ala Tyr Trp Gly Gln Gly Thr Leu Val Thr Val Ser Ser*
TCC TAT GAT TAC GAC TGG TTT GCT TAC TGG GGC CAA GGG ACT CTG GTC ACC GTC TCC TCA
375 390 405 420

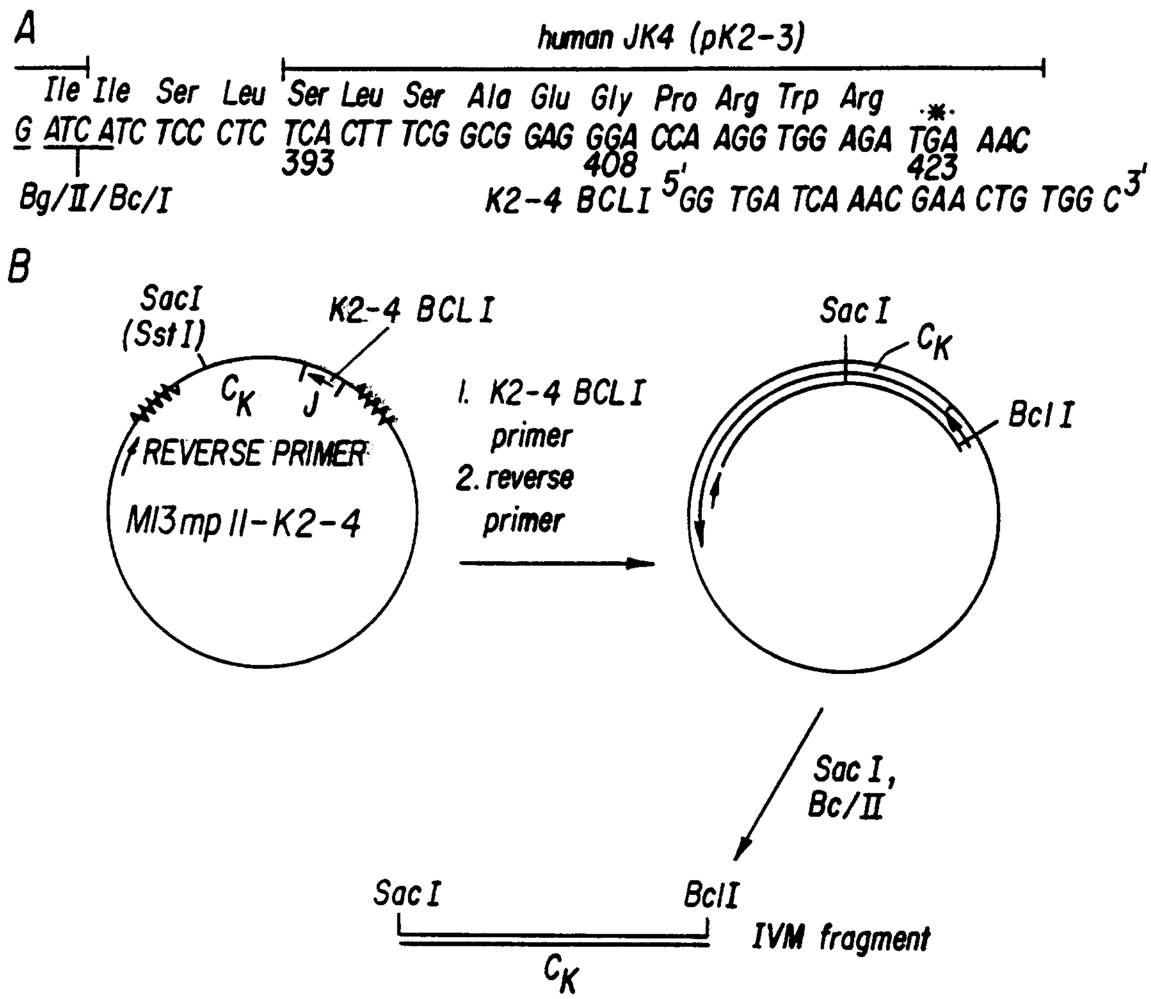
A
human JK4 (pK2-3)
Ile Ile Ser Leu Ser Leu Ser Ala Glu Gly Pro Arg Trp Arg
G ATC ATC TCC CTC TCA CTT TCG GCG GAG GGA CCA AGG TGG AGA TGA AAC
393
408
423
Bg/II/Bc/I
K2-4 BCLI 5' GG TGA TCA AAC GAA CTG TGG C 3'
B
SacI (SstI)
K2-4 BCLI
CK
J
REVERSE PRIMER
M13mp11-K2-4
1. K2-4 BCLI primer
2. reverse primer
Sac I
CK
BclI
Sac I, Bc/II
Sac I
BclI
IVM fragment
CK

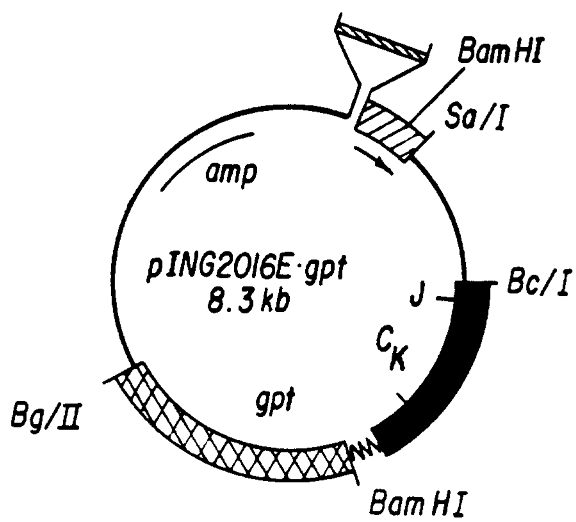
BamHI
Sa/I
amp
pING2016E·gpt
8.3 kb
J
Bc/I
CK
gpt
Bg/II
BamHI

FIG. 13

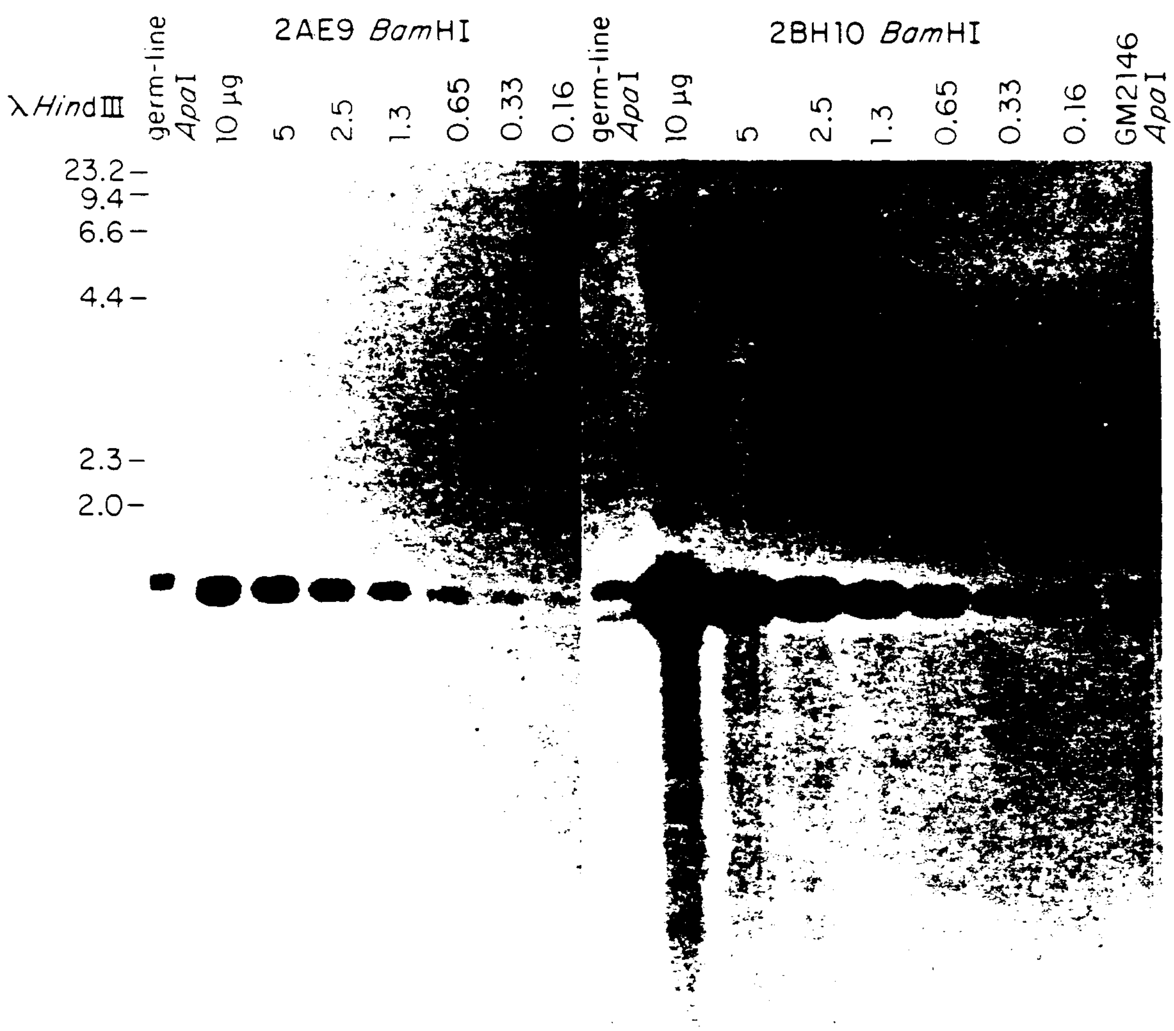


pMvHc24 ApaI-BamHI ($C_H$)

# FIG.15

MET Asp Trp Leu Trp Asn Leu
BG ATC CCC CCC CCC CCC CCC CCC CCC CAG TTT GTC TTA AGG CAC CAC TGA BCC CAA GTC TTA GAC ATC ATG GAT TGG CTG TGG AAC TTG
15 30 45 60 75 90

leader peptide →|← FR1

Leu Phe Leu MET Ala Ala Ala Gln Ser Ala Gln Ala Gln Ile Gln Leu Val Gln Ser Gly Pro Glu Leu Lys Lys Pro Gly Glu Thr Val
CTA TTC CTG ATG GCA GCT GCC CAA AGT GCC CAA GCA CAG ATC CAG TTG GTG CAG TCT GGA CCT GAG CTG AAG AAG CCT GGA GAG ACA GTC
105 120 135 150 165 180

FR1 →|← CDR1 →|← FR2

Lys Ile Ser Cys Lys Ala Ser Gly Tyr Thr Phe Thr Asn Tyr Gly MET Asn Trp Val Lys Gln Ala Pro Gly Lys Gly Leu Lys Trp MET
AAG ATC TCC TGC AAG GCT TCT GGG TAT ACC TTC ACA AAC TAT GGA ATG AAC TGG GTG AAG CAG GCT CCA GGA AAG GGT TTA AAG TGG ATG
Bg1 II 195 210 225 240 255 270

FR2 →|← CDR2
CDR2 →|← FR3

Gly Trp Ile Asn Thr Tyr Thr Gly Gln Pro Thr Tyr Ala Asp Asp Phe Lys Gly Arg Phe Ala Phe Ser Leu Glu Thr Ser Ala Tyr Thr
BGC TGG ATA AAC ACC TAC ACT GGA CAG CCA ACA TAT GCT GAT GAC TTC AAG GGA CGG TTT GCC TTC TCT TTG GAA ACC TCT GCC TAC ACT
285 300 315 330 345 360

FR3 →|← CDR3 |— $J_H$2—

Ala Tyr Leu Gln Ile Asn Asn Leu Lys Asn Glu Asp MET Ala Thr Tyr Phe Cys Ala Arg Phe Ser Tyr Gly Asn Ser Arg Tyr Ser Asp
GCC TAT TTG CAG ATC AAC AAC CTC AAA AAT GAG GAC ATG GCT ACA TAT TTC TGT GCA AGA TTT AGC TAT GGT AAC TCA CGT TAC TCT GAC
375 390 405 420 DSP. 2 435 450

———— $J_H$2 —|—— $C_{r2a}$
B →|← FR4

Tyr Trp Gly Gln Gly Thr Thr Leu Thr Val Ser Ser Ala Lys Thr Thr Ala Pro Ser Val Tyr Pro Leu Ala Pro Val Cys Gly Asp Thr
TAC TGG GGC CAA GGC ACC ACT CTC ACA GTC TCC TCA GCC AAA ACA ACA GCC CCA TCG GTC TAT CCA CTG GCC CCT GTG TGT GGA SAT ACA
465 480 495 510 525 540

Thr Gly Ser Ser Val Thr Leu Gly Cys Leu Val Lys Gly Tyr Phe Pro Glu Pro Val Thr Leu Thr Trp Asn Ser Gly
ACT GGC TCC TCG GTG ACT CTA BGA TGC CTG GTC AAG BGT TAT TTC CCT GAG CCA GTG ACC TTG ACC TGG AAC TCT GGA
555 570 585 600 615

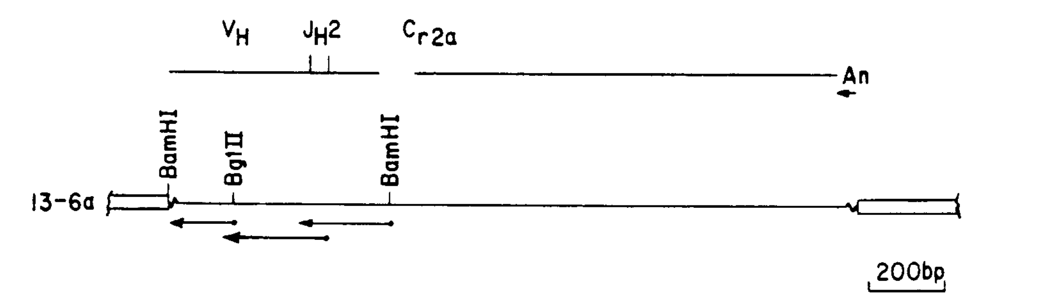

Fig. 16

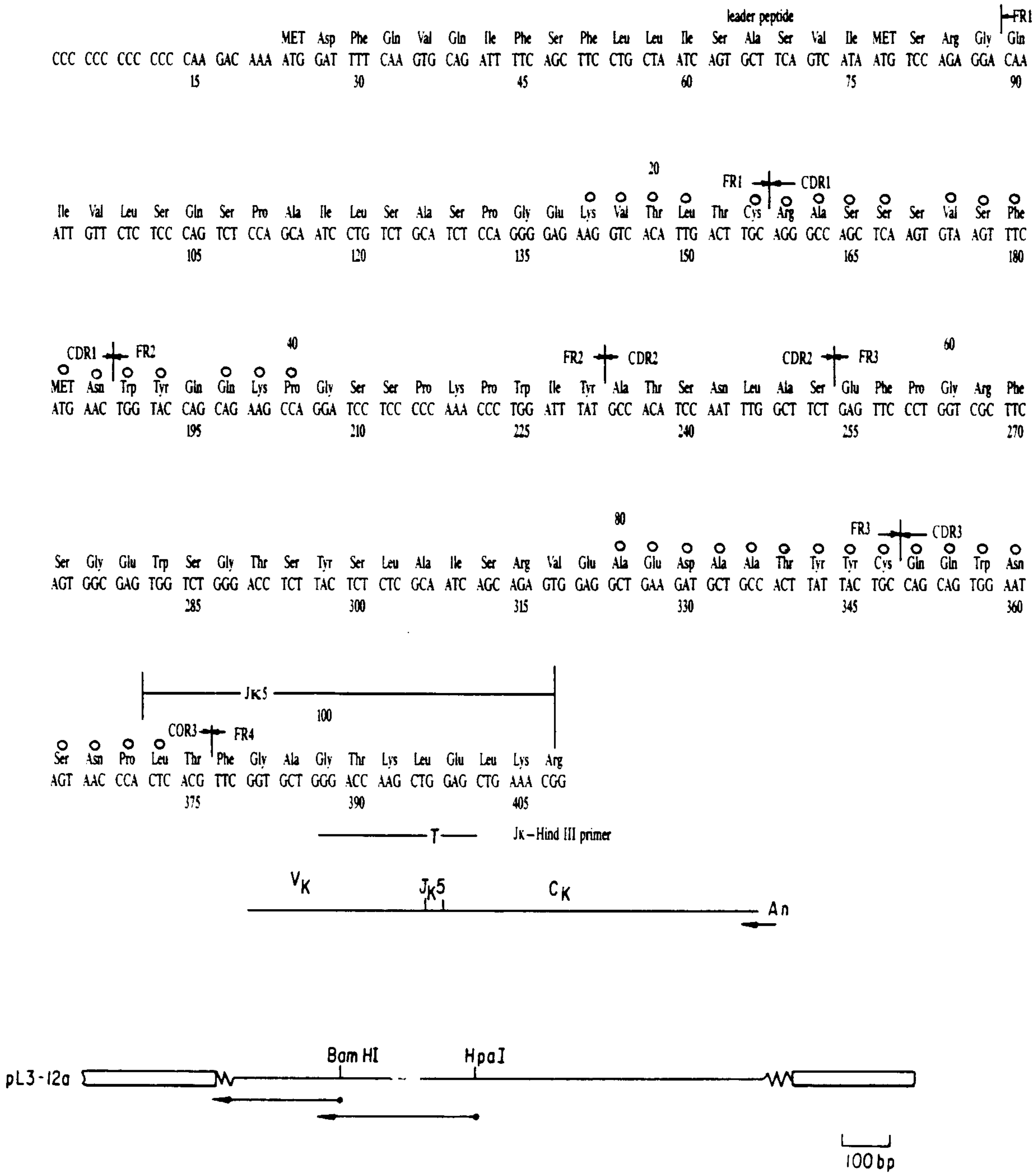
leader peptide
FR1
MET Asp Phe Gln Val Gln Ile Phe Ser Phe Leu Leu Ile Ser Ala Ser Val Ile MET Ser Arg Gly Gln
CCC CCC CCC CCC CAA GAC AAA ATG GAT TTT CAA GTG CAG ATT TTC AGC TTC CTG CTA ATC AGT GCT TCA GTC ATA ATG TCC AGA GGA CAA
15 30 45 60 75 90
20
FR1 CDR1
Ile Val Leu Ser Gln Ser Pro Ala Ile Leu Ser Ala Ser Pro Gly Glu Lys Val Thr Leu Thr Cys Arg Ala Ser Ser Ser Val Ser Phe
ATT GTT CTC TCC CAG TCT CCA GCA ATC CTG TCT GCA TCT CCA GGG GAG AAG GTC ACA TTG ACT TGC AGG GCC AGC TCA AGT GTA AGT TTC
105 120 135 150 165 180
CDR1 FR2
40
FR2 CDR2
CDR2 FR3
60
MET Asn Trp Tyr Gln Gln Lys Pro Gly Ser Ser Pro Lys Pro Trp Ile Tyr Ala Thr Ser Asn Leu Ala Ser Glu Phe Pro Gly Arg Phe
ATG AAC TGG TAC CAG CAG AAG CCA GGA TCC TCC CCC AAA CCC TGG ATT TAT GCC ACA TCC AAT TTG GCT TCT GAG TTC CCT GGT CGC TTC
195 210 225 240 255 270
80
FR3 CDR3
Ser Gly Glu Trp Ser Gly Thr Ser Tyr Ser Leu Ala Ile Ser Arg Val Glu Ala Glu Asp Ala Ala Thr Tyr Tyr Cys Gln Gln Trp Asn
AGT GGC GAG TGG TCT GGG ACC TCT TAC TCT CTC GCA ATC AGC AGA GTG GAG GCT GAA GAT GCT GCC ACT TAT TAC TGC CAG CAG TGG AAT
285 300 315 330 345 360
JK5
100
CDR3 FR4
Ser Asn Pro Leu Thr Phe Gly Ala Gly Thr Lys Leu Glu Leu Lys Arg
AGT AAC CCA CTC ACG TTC GGT GCT GGG ACC AAG CTG GAG CTG AAA CGG
375 390 405
T
JK-Hind III primer
VK
JK5
CK
An
Bam HI
HpaI
pL3-12a
100bp

Fig.17a

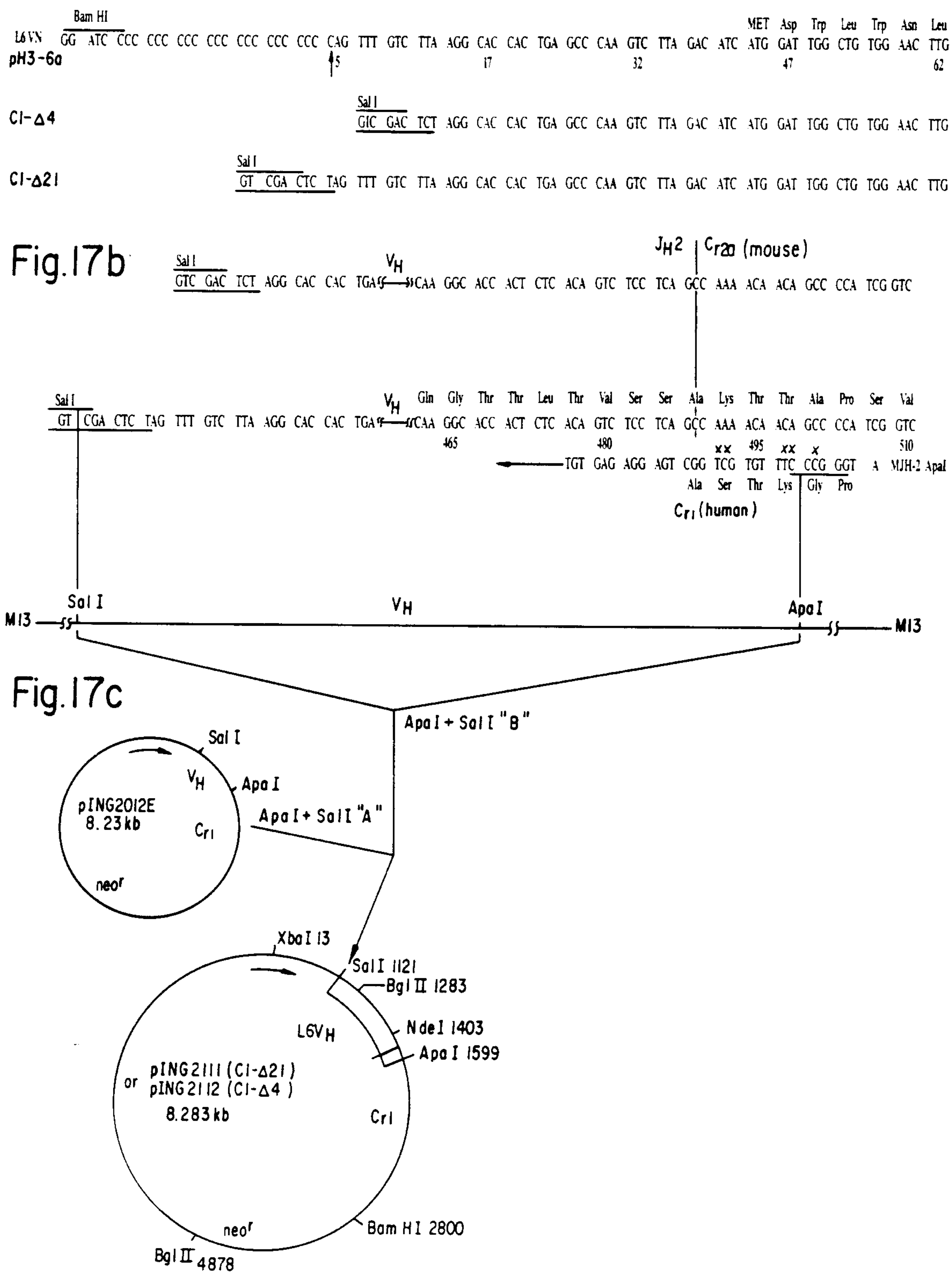
Bam HI
L6VN GG ATC CCC CCC CCC CCC CCC CCC CCC CAG TTT GTC TTA AGG CAC CAC TGA GCC CAA GTC TTA GAC ATC ATG GAT TGG CTG TGG AAC TTG
pH3-6a
MET Asp Trp Leu Trp Asn Leu
5 17 32 47 62
Cl-Δ4
Sal I
GTC GAC TCT AGG CAC CAC TGA GCC CAA GTC TTA GAC ATC ATG GAT TGG CTG TGG AAC TTG
Cl-Δ21
Sal I
GT CGA CTC TAG TTT GTC TTA AGG CAC CAC TGA GCC CAA GTC TTA GAC ATC ATG GAT TGG CTG TGG AAC TTG
Fig.17b
Sal I
GTC GAC TCT AGG CAC CAC TGA CAA GGC ACC ACT CTC ACA GTC TCC TCA GCC AAA ACA ACA GCC CCA TCG GTC
$V_H$
$J_H2$
$C_{γ2a}$ (mouse)
Sal I
GT CGA CTC TAG TTT GTC TTA AGG CAC CAC TGA CAA GGC ACC ACT CTC ACA GTC TCC TCA GCC AAA ACA ACA GCC CCA TCG GTC
Gln Gly Thr Thr Leu Thr Val Ser Ser Ala Lys Thr Thr Ala Pro Ser Val
465 480 495 510
TGT GAG AGG AGT CGG TCG TGT TTC CCG GGT A MJH-2 ApaI
Ala Ser Thr Lys Gly Pro
$C_{γ1}$ (human)
M13 Sal I $V_H$ Apa I M13
Fig.17c
Apa I + Sal I "B"
Sal I
$V_H$
Apa I
pING2012E
8.23 kb
$C_{γ1}$
neo^r
Apa I + Sal I "A"
Xba I 13
Sal I 1121
Bgl II 1283
Nde I 1403
Apa I 1599
L6$V_H$
or
pING2111 (Cl-Δ21)
pING2112 (Cl-Δ4)
8.283 kb
Cγ1
neo^r
Bam H I 2800
Bgl II 4878

FIG. 18

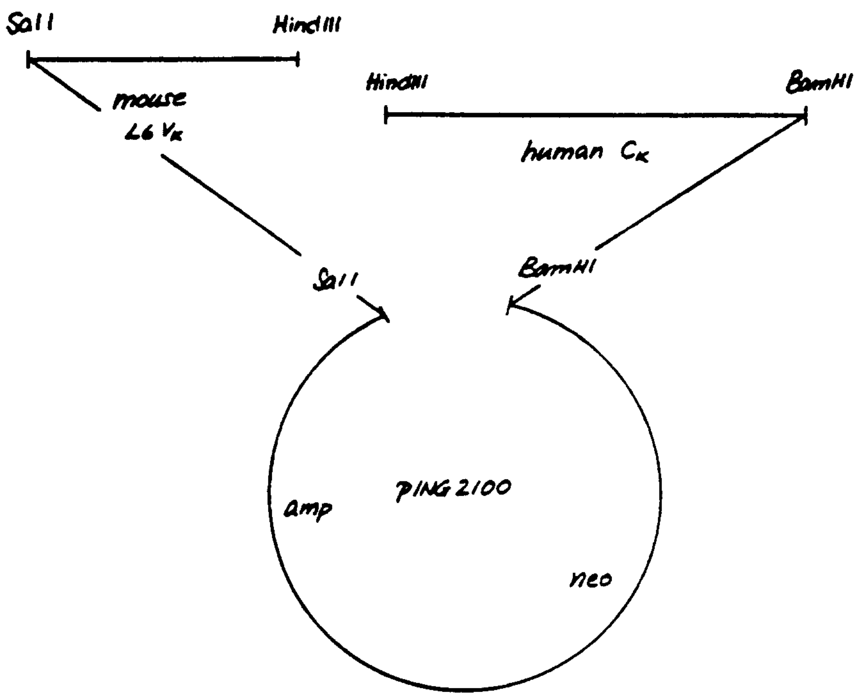
SalI
HindIII
mouse
L6 Vκ
HindIII
BamHI
human Cκ
SalI
BamHI
pING 2100
amp
neo

FIG. 19

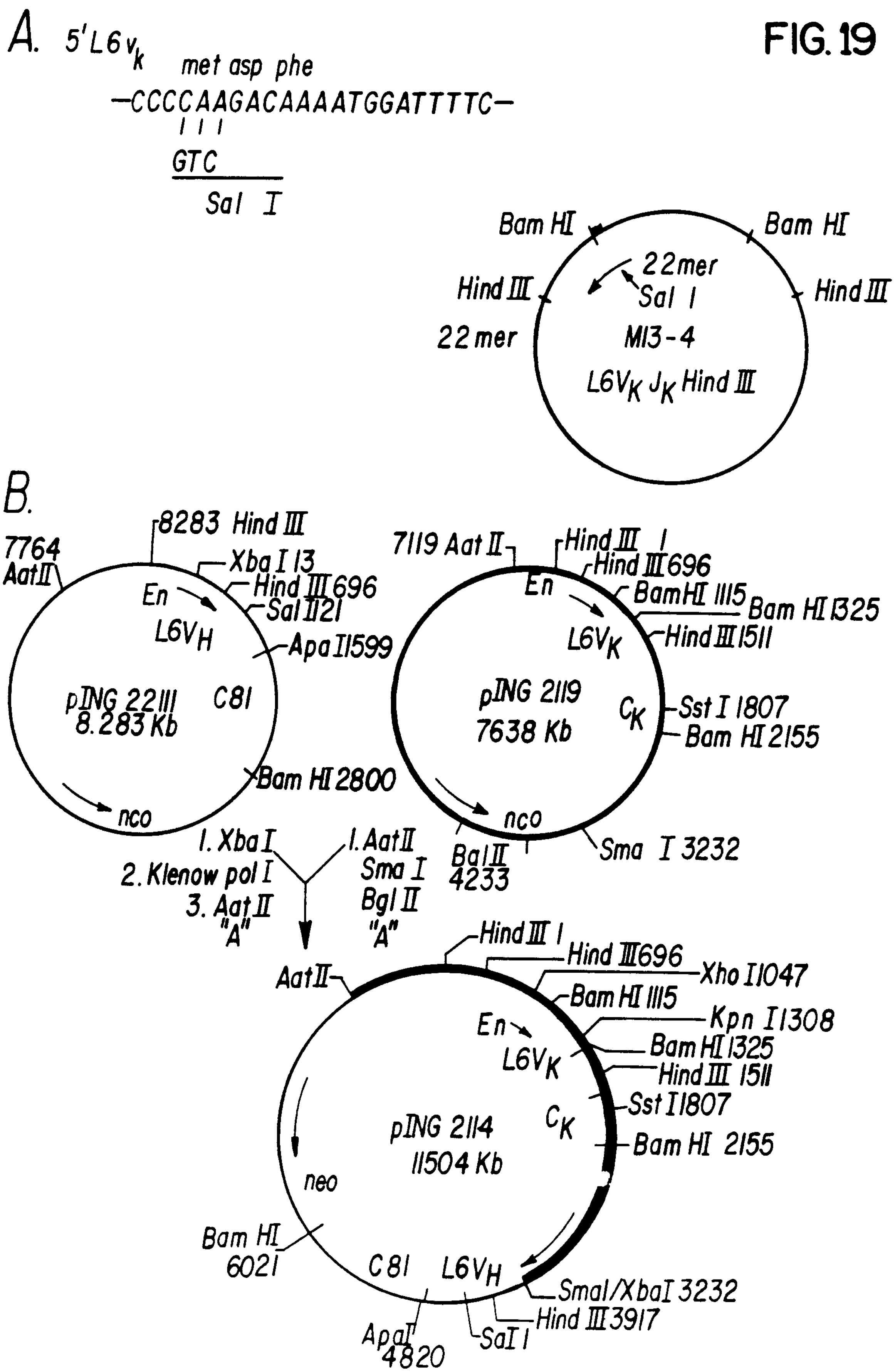
A.
5' L6 $v_k$
met asp phe
—CCCCAAGACAAAATGGATTTTC—
GTC
Sal I
Bam HI
Bam HI
Hind III
Hind III
22mer
Sal I
22 mer
M13-4
L6$V_K$ $J_K$ Hind III
B.
8283 Hind III
7764 AatII
Xba I 13
Hind III 696
Sal I 1121
En
L6$V_H$
Apa I 1599
pING 22111 8.283 Kb
C81
Bam HI 2800
nco
7119 Aat II
Hind III 1
Hind III 696
Bam HI 1115
Bam HI 1325
Hind III 1511
L6$V_K$
pING 2119 7638 Kb
$C_K$
Sst I 1807
Bam HI 2155
Sma I 3232
Bgl II 4233
1. Xba I
2. Klenow pol I
3. Aat II
"A"
1. Aat II
Sma I
Bgl II
"A"
Aat II
Hind III 1
Hind III 696
Xho I 1047
Bam HI 1115
Kpn I 1308
Bam HI 1325
Hind III 1511
Sst I 1807
Bam HI 2155
En
L6$V_K$
$C_K$
pING 2114 11504 Kb
neo
Bam HI 6021
C81
L6$V_H$
SmaI/XbaI 3232
Hind III 3917
Sal I
ApaI 4820

FIG. 20

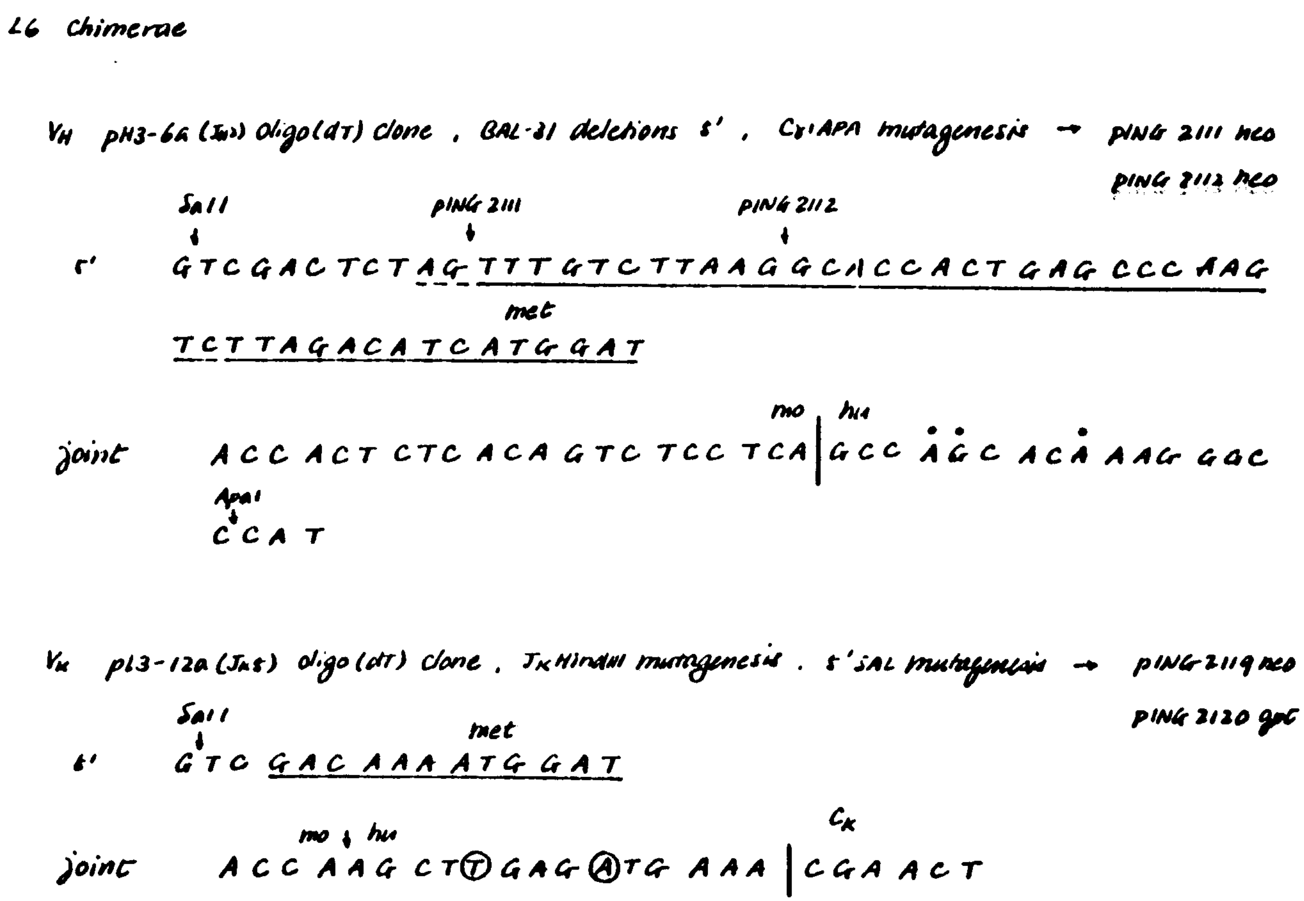
L6 Chimerae
VH pH3-6a (JH3) oligo(dT) clone, BAL-31 deletions 5', Cγ1 APA mutagenesis → pING 2111 neo
pING 2112 neo
SalI
pING 2111
pING 2112
5' GTCGACTCTAGTTTGTCTTAAGGCACCACTGAGCCCAAG
met
TCTTAGACATCATGGAT
mo
hu
joint ACC ACT CTC ACA GTC TCC TCA | GCC AGC ACA AAG GAC
ApaI
CCAT
VK pL3-12a (JK5) oligo(dT) clone, JK HindIII mutagenesis. 5' SAL mutagenesis → pING 2119 neo
pING 2120 gpt
SalI
met
5' GTC GAC AAA ATG GAT
CK
mo hu
joint ACC AAG CTT GAG ATG AAA | CGA ACT

# FIG. 21

2H7 heavy chain variable sequence

```
                                                                   leader
                                                 met gly phe ser arg ile phe
C33GTACCTCTCTACAGTCCCTGAAGACACTGACTCTAACCATG GGA TTC AGC AGG ATC TTT
                                                 ↑
                                                NcoI         | FR1  °   °   °   °
   peptide
leu phe leu leu ser val thr thr gly val his ser gln ala tyr leu gln
CTC TTC CTC CTG TCA GTA ACT ACA GGT GTC CAC TCC CAG GCT TAT CTA CAG

 °   °   °   °   °   °   °
gln ser gly ala glu leu val arg pro gly ala ser val lys met ser cys
CAG TCT GGG GCT GAG CTG GTG AGG CCT GGG GCC TCA GTG AAG ATG TCC TGC

                              FR1 | CDR1           CDR1 | FR2
lys ala ser gly tyr thr phe thr ser tyr asn met his trp val lys gln
AAG GCT TCT GGC TAC ACA TTT ACC AGT TAC AAT ATG CAC TGG GTA AAG CAG

                                     FR2 | CDR2
thr pro arg gln gly leu glu trp ile gly ala ile tyr pro gly asn gly
ACA CCT AGA CAG GGC CTG GAA TGG ATT GGA GCT ATT TAT CCA GGA AAT GGT

                                     CDR2 | FR3
asp thr ser tyr asn gln lys phe lys gly lys ala thr leu thr val asp
GAT ACT TCC TAC AAT CAG AAG TTC AAG GGC AAG GCC ACA CTG ACT GTA GAC

lys ser ser ser thr ala tyr met gln leu ser ser leu thr ser glu asp
AAA TCC TCC AGC ACA GCC TAC ATG CAG CTC AGC AGC CTG ACA TCT GAA GAC

                              FR3 | CDR3
ser ala val tyr phe cys ala arg val val tyr tyr ser asn ser tyr trp
TCT GCG GTC TAT TTC TGT GCA AGA GTG GTG TAC TAT AGT AAC TCT TAC TGG
                             JH1                               Dsp.2
             CDR3 | FR4                                   FR4
tyr phe asp val trp gly thr gly thr thr val thr val ser
TAC TTC GAT GTC TGG GGC ACA GGG ACC ACG GTC ACC GTC TCG30
                                ↑
                             BstEII                  JH BstEII primer
```

# FIG. 22

2H7 light chain variable sequence

```
                                                                  leader peptide
                         met asp phe gln val gln ile phe ser phe leu leu
C23CCCAAAATTCAAAGACAAAATG GAT TTT CAA GTG CAG ATT TTC AGC TTC CTG CTA
   ----------GTC-----------  SalI primer
                                             | FR1
 ile ser ala ser val ile ile ala arg gly gln ile val leu ser gln ser
 ATC AGT GCT TCA GTC ATA ATT GCC AGA GGA CAA ATT GTT CTC TCC CAG TCT

                                                                FR1 |
 pro ala ile leu ser ala ser pro gly glu lys val thr met thr cys arg
 CCA GCA ATC CTG TCT GCA TCT CCA GGG GAG AAG GTC ACA ATG ACT TGC AGG

 CDR1                              CDR1 | FR2
 ala ser ser ser val ser tyr met his trp tyr gln gln lys pro gly ser
 GCC AGC TCA AGT GTA AGT TAC ATG CAC TGG TAC CAG CAG AAG CCA GGA TCC
                                              KpnI ↑          ↑ BamHI
                         FR2 | CDR2                    CDR2 | FR3
 ser pro lys pro trp ile tyr ala pro ser asn leu ala ser gly val pro
 TCC CCC AAA CCC TGG ATT TAT GCC CCA TCC AAC CTG GCT TCT GGA GTC CCT

 ala arg phe ser gly ser gly ser gly thr ser tyr ser leu thr ile ser
 GCT CGC TTC AGT GGC AGT GGG TCT GGG ACC TCT TAC TCT CTC ACA ATC AGC

                         o   o   o   o   o   o  FR3 | CDR3 o   o   o   o
 arg val glu ala glu asp ala ala thr tyr tyr cys gln gln trp ser phe
 AGA GTG GAG GCT GAA GAT GCT GCC ACT TAT TAC TGC CAG CAG TGG AGT TTT

              ---------------------- JK5 ----------------------
 o   o   o  CDR3 | FR4  o   o   o                         FR4
 asn pro pro thr phe gly ala gly thr lys leu glu leu lys
 AAC CCA CCC ACG TTC GGT GCT GGG ACC AAG CTG GAG CTG AAA
                            ------------T------
                                         JKHindIII primer
```

FIG. 23

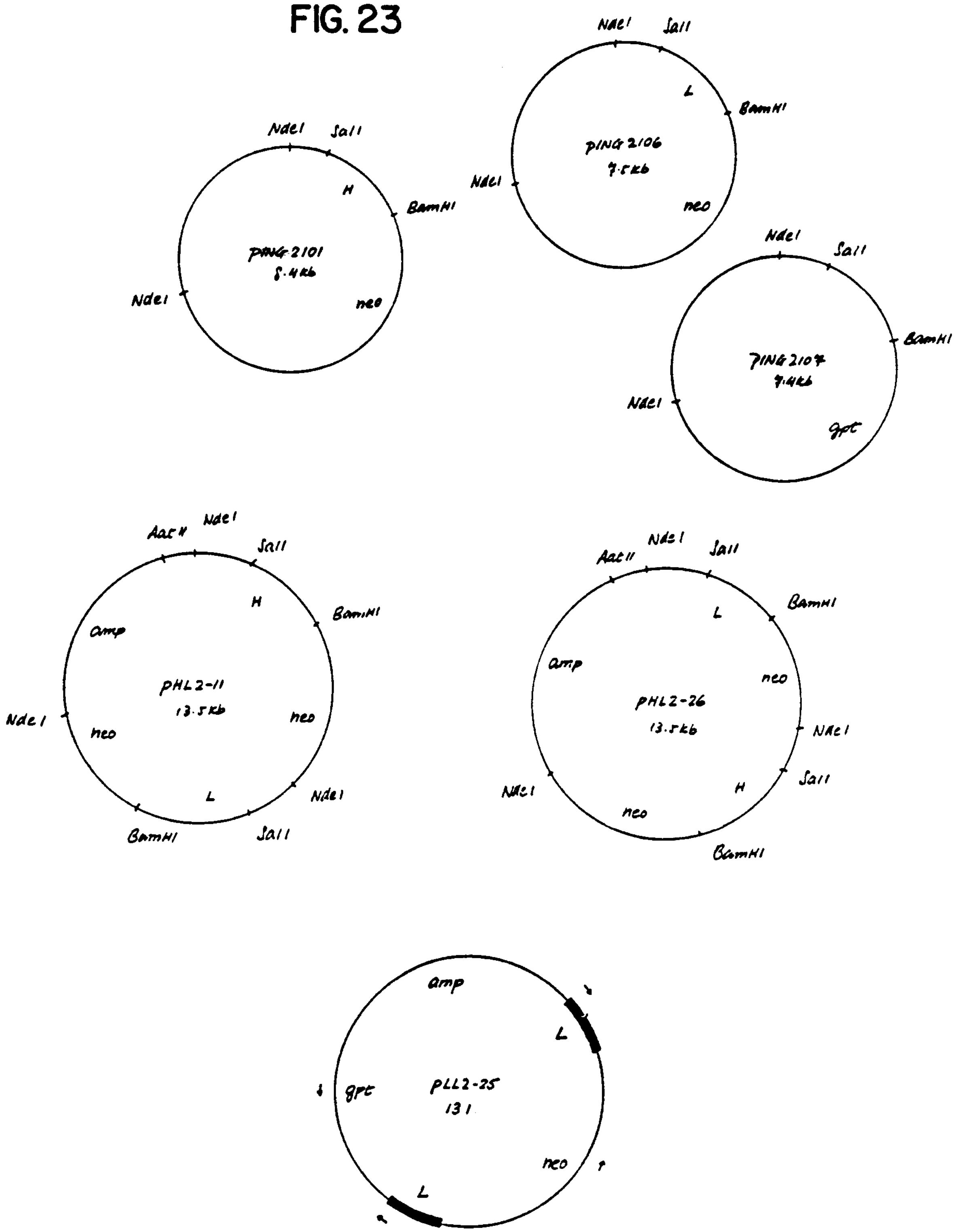
NdeI
SalI
BamHI
H
L
pING 2101
5.4Kb
pING 2106
7.5Kb
pING 2107
7.4Kb
neo
gpt
AatII
amp
pHL2-11
13.5Kb
pHL2-26
13.5Kb
pLL2-25
13 I

# FIG.24

2H7 Chimerae

$V_H$ pH2-7 ($J_H1$) $J_H$ BstEII clone, NcoI cut 5' ATG → pING 2101 neo

SalI ↓ ; met

5' GTCCA CATG GGA

mo ↓ hu ; $C_{\gamma}1$

joint ACG GTC ACC GTC TC(T) TCA | GCC TCC

$V_K$ pL2-12 ($J_K5$) oligo(dT) clone, $J_K$ HindIII mutagenesis. 5'SAL mutagenesis → pING 2106 neo

pING 2107 gpt

SalI ↓ ; met

5' GTC GAC AAA ATG GAT

mo ↓ hu ; $C_K$

joint ACC AAG CT(T) GAG (A)TG AAA | CGA ACT